# EUROPEAN PATENT APPLICATION

(11) **EP 4 596 605 A1**
(43) Date of publication of application: **06.08.2025**
(21) Application number: 23871041.2
(22) Date of filing: 28.09.2023
(51) Int. Cl.: C08G 65/00, C07C 229/26, A61K 9/127

(54) **PEGYLATED LIPID CONTAINING LYSINE CORE**

(30) Priority: 30.09.2022 CN 202211219118
(71) Applicant: Xiamen Sinopeg Biotech Co., Ltd., Xiamen, Fujian 361100 (CN)
(72) Inventor: WENG, Wengui, Xiamen, Fujian 361100 (CN); LIU, Chao, Xiamen, Fujian 361100 (CN); WEI, Guohua, Xiamen, Fujian 361100 (CN); WANG, Ailan, Xiamen, Fujian 361100 (CN); WANG, Linlin, Xiamen, Fujian 361100 (CN); LIN, Sheng, Xiamen, Fujian 361100 (CN); ZHENG, Zhongzhi, Xiamen, Fujian 361100 (CN); YUAN, Jinchun, Xiamen, Fujian 361100 (CN)
(74) Representative: Algemeen Octrooi- en Merkenbureau B.V.
(86) International application number: PCT/CN2023/122654
(87) International publication number: WO 2024/067816

(57) **Abstract**

The present application belongs to the field of drug delivery, and specifically relates to a PEGylated lipid containing a lysine core that can be used as a component of drug carrier, and a lipid composition containing the PEGylated lipid, a LNP pharmaceutical composition and formulations and applications thereof. In the present application, the PEGylated lipid containing a lysine core is more stable. The application of the PEGylated lipid in the preparation of lipid composition and lipid pharmaceutical composition has the advantages of prolonged circulation time, high biocompatibility, low toxicity, high encapsulation efficiency and high delivery efficiency.

## Description

### TECHNICAL FIELD

The present application belongs to the field of drug delivery, relating to a PEGylated lipid in general, in particular to a PEGylated lipid containing a lysine core that can be used as a drug carrier component, and a lipid composition containing the PEGylated lipid, a lipid pharmaceutical composition and formulations and applications thereof.

### BACKGROUND

Effective in vivo delivery is a critical element for drugs to achieve therapeutic efficacy. Exogenous drugs must penetrate the lipid membrane barrier to enter the cytoplasm and thus successfully translated into functional proteins. There are two barriers to drug delivery into the cell, enzymatic degradation on the way to delivery and a membrane barrier due to electrostatic repulsion. An ideal delivery system needs to fulfill several conditions: to effectively encapsulate and protect the drug before reaching the target to maintain its stability; to help the drug enter the cell efficiently; and to release the drug into the cytoplasm promptly before it reaches the lysosomes.

Currently, a variety of delivery vectors have been developed to facilitate the cellular uptake of drugs and protect them from degradation. The main non-viral vectors are protamine, lipid nanoparticles (LNPs), dendritic cells, inorganic nanoparticles, etc. Among them, LNP is a relatively mature technology platform for the delivery of RNA drugs, vaccines or gene editing tools. Compared with other types of nucleic acid drug delivery systems, LNP have many advantages, such as high nucleic acid encapsulation efficiency and effective transfection of cells, strong tissue penetration, low cytotoxicity and immunogenicity, and more conducive to drug delivery, which makes LNP an excellent nucleic acid delivery system. LNP is mainly composed of ionizable cationic lipid, cholesterol, neutral phospholipid, and PEGylated lipid (PEG-lipid).

Like other nanocarriers, PEGylated lipid provide an external polymerization layer for LNP, which prolongs the circulation time in vivo and prevents the aggregation of nanoparticles during storage and in the blood, and the number of PEGylated lipid may also determine the size of particles, which shows that PEGylated lipid is an important component in LNP for regulating half-life and cellular uptake.

Although there have been advances in the study of PEGylated lipid, such as the commercially well-established DMG-2k, fewer amino acid nuclear PEGylated lipids have been reported. The PEGylated lipid with glutamic acid and aspartic acid as backbone and ester bond as linking bond, such as compound PEG₂₀₀₀-Suc-TA₂, have been reported in reference CN114539083A, and the ester bonds are degradable linking groups that are unstable in vivo, thus affecting the delivery efficiency of PEGylated lipid. To overcome the shortcomings of the prior art, we design and synthesize a series of PEGylated lipid containing lysine core in the present application. The amino group at the lysine terminal leads to an amide or carbamate bond that is more stable in the body.

### SUMMARY

The application provides a novel PEGylated lipid and preparation methods thereof, comprising a lipid composition containing the PEGylated lipid and a lipid pharmaceutical composition containing the lipid composition and the formulation thereof, and a liposome or a lipid nanoparticle containing the lipid composition; especially the LNP-nucleic acid pharmaceutical composition containing the lipid composition and the formulation thereof has the advantages of high delivery efficiency, safety low toxicity, and high biocompatibility, which can improve the therapeutic and/or preventive effects of the drugs.

The above-described purposes of this application can be realized via the embodiments below.

In one embodiment, provided herein is a PEGylated lipid:
A PEGylated lipid, wherein the structure is represented by the general formula (1): 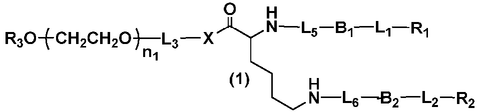 wherein,
X is -O- or -NH-;
L₁ and L₂ are each independently selected from the group consisting of a linking bond, -C(=O)-, -C(=O)O-, -OC(=O)-, -OC(=O)O-, -C(=O)NH-, -NHC(=O)-, -NHC(=O)O-, -OC(=O)NH- and -NHC(=O)NH-;
L₅ and L₆ are each independently selected from the group consisting of a linking bond, -C(=O)- and -C(=O)O-;
L₃ is a linking bond or a divalent linking group, and the divalent linking group is selected from the group consisting of -(CH₂)ₜ-, -O-, -C(=O)O-, -C(=O)O-, -OC(=O)O-, -OC(=O)O-, -C(=O)NH-, -NHC(=O)-, -NHC(=O)O-, -OC(=O)NH-, -NHC(=O)NH- and any combinations thereof, wherein, t is an integer from 1 to 12;
B₁ and B₂ are each independently a linking bond or a C₁₋₁₂ alkylene group;
R₁ and R₂ are each independently a C₅₋₃₀ aliphatic hydrocarbon group; wherein, the aliphatic hydrocarbon group is an alkyl group, an alkene group or an alkyne group;
R₃ is a hydrogen atom, a C₁₋₆ alkyl group, a carbocyclic group, a heterocyclic group or -L₄-R₀₁; wherein, L₄ is a divalent linking group and R₀₁ is a functional group that can react with bio-related substances;
n₁ is an integer from 20 to 1000.

The present application also provides a lipid composition, embodied as follows:
A lipid composition containing a PEGylated lipid with the structure represented by the formula (1).

The present application also provides a lipid pharmaceutical composition, embodied as follows:
Provided herein is a lipid pharmaceutical composition containing a lipid composition and a drug, and the lipid composition contains a PEGylated lipid with the structure represented by the formula (1), and the drug is selected from the group consisting of a nucleic acid drug, a gene vaccine, an anti-tumor drug, a small molecule drug, a peptide drug and a protein drug.

The present application also provides a formulation of lipid pharmaceutical composition, embodied as follows:
Provided herein is a formulation of lipid pharmaceutical composition containing the aforementioned lipid pharmaceutical composition and a pharmaceutically acceptable diluent or excipient.

The present application also provides a liposome or LNP, embodied as follows:
Provided herein is a liposome or LNP containing a lipid composition, and the lipid composition containing a PEGylated lipid with the structure represented by formula (1).

### Compared with the prior art, the present application brings the following beneficial effects:

In the present application, the branching center of PEGylated lipid is a lysine, which has the advantages of safety, low toxicity, high biocompatibility, simple and easy to obtain, easy to synthesize or commercially obtained, and cost-saving. The synthesis route of the PEGylated lipid is also relatively simple, providing more options for the delivery systems of nucleic acid drugs, gene vaccines, and small molecule drugs.

Compared with the glutamic acid core or aspartic acid core PEGylated lipid containing two ester bonds in the prior art, the novel PEGylated lipid of the present application contains fewer unstable linking bonds (ester bonds), and most of them only contain one degradable ester bond or do not contain ester bonds, therefore it is more stable in vivo, which can improve the cell uptake rate and transfection efficiency.

In the novel PEGylated lipid of the present application, the connecting part of the lysine core and the PEG chain can contain degradable ester bonds, and the PEG chain is easy to fall off in the process of systemic circulation, which is conducive to the encapsulated therapeutic agent or therapeutic agent escaping from the lipid nanoparticle into the cytoplasm to exert the corresponding curative effect.

The hydrophobic tail chain of the novel PEGylated lipid of the present application can also optionally be an unsaturated hydrocarbon group, which makes the molecular layer have a certain rigidity and is more stable, and also makes the arrangement of the molecular layer looser, with higher membrane fluidity, and then enhances the membrane fusion ability and cell uptake.

The PEG terminus of the novel PEGylated lipid of the present application can also modify various targeting groups, providing a relatively simple method for the preparation of targeted LNP and targeted therapy.

### BRIEF DESCRIPTION OF THE DRAWINGS

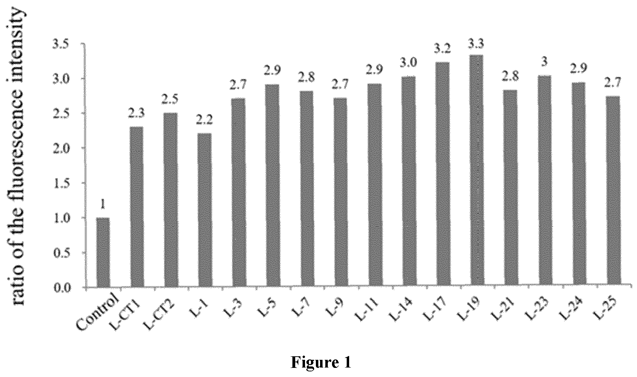

Fig. 1 shows the in vitro cell transfection result of the lipid pharmaceutical composition prepared in Example 13 of the present application.

### 1. DETAILED DESCRIPTION OF THE APPLICATION

The specific embodiments of the present application are described in detail. However, it should be understood that these embodiments are given only in an illustrative manner and not in a restrictive manner and that various variations and modifications within the scope of the present application will be obvious to those skilled in the art.

If the description in the references cited in the present application differs from the description of the present application, the present application shall prevail. This principle applies to all cited references throughout the entire specification.

### 1.1 Description of terms

In the present application, unless otherwise described, all technical and scientific terms used herein have the same meaning as generally understood by those of ordinary skill in the art. The disclosures of all patents and other publications cited herein are incorporated herein in their entirety by reference. In the event of a conflict between any description of the terms herein and any document incorporated herein by reference, the description and interpretation of the terms described below shall prevail. Unless otherwise indicated, terms have the following meanings.

In the present application, when two or more objects are "each independently preferably" selected from multiple levels of preferable options, the objects are not necessarily selected from preferable options of the same level. It is allowed that one is selected from a wider range of preferable options while another one is selected from a narrower range of preferable options. It is also allowed that one is selected from the maximum range of preferable options while another is selected from any allowable preferable options. It is also allowed that the objects are selected from preferable options of the same level.

In the present application, "each independently/selected from/preferably" not only refers to different groups that can be each independently/selected from/ preferably any option from the definition but also can be added with "at each occurrence" to indicate that the same group at different positions or times can be independently/selected from/preferably any option from the definition. For example, "L₃ is selected from the group consisting of a linking bond, -(CH₂)ₜₘ-, -C(=O)(CH₂)ₜₘ-, -C(=O)NH(CH₂)ₜₘ-, -C(=O)O(CH₂)ₜₘ-, -(CH₂)ₜₘC(=O)NH(CH₂)ₜₘ-, -(CH₂)ₜₘNHC(=O)(CH₂)ₜₘ-, -(CH₂)ₜₘC(=O)O(CH₂)ₜₘ- and -C(=O)(CH₂)ₜₘC(=O)NH(CH₂)ₜₘ-; wherein, the left end of L₃ is connected to the PEG chain and t is independently an integer from 1 to 6 at each occurrence"; the tm appearing in either of the two can be the same or different, and the tm at different positions in the same group can also be the same or different, such as "-(CH₂)ₜₘC(=O)O(CH₂)ₜₘ-".

In the present application, unless otherwise specified, "any" includes any one, any two, and any two or more.

In the present application, when at least two items are listed, "any combination" of the listed items refers to any two or more combinations of the aforementioned listed items; and there is no limit on the number of items. The number of any item can be zero, one, or greater than one. When the number of the same item is greater than one, it can be the same or different specific forms that satisfy that item. For example, "L₃ is a linking bond or a divalent linking bond, and the divalent linking bond is selected from the group consisting of -(CH₂)ₜₘ-, -O-, -C(=O)-, -C(=O)O-, -OC(=O)-, -OC(=O)O-, -C(=O)NH-, -NHC(=O)-, -NHC(=O)O-, -OC(=O)NH-, -NHC(=O)NH- and any combinations thereof", L₃ can be any one of the listed items or any two or more combinations, which can be -CH₂-O-CH₂- (i.e. a combination of one -O- and two -(CH₂)ₜₘ- in the listed terms, and the specific form of -(CH₂)ₜₘ- is -(CH₂)-), or -CH₂-NH-CH₂CH₂- (i.e. a combination of -NH- and two -(CH₂)ₜₘ- in the listed terms, wherein the specific forms of two -(CH₂)ₜₘ- are a methylene group and an ethylene group), or other structures that comply with the description.

The terms "include", "contain", and similar expressions in the present application shall be interpreted, unless otherwise specified, as "including but not limited to", "include but are not limited to", or "includes but is not limited to", etc., in the description and claims with openness and inclusiveness.

In the present application, "including but not limited to" a certain scope means that the items within the scope are optional, but not limited to the terms of the scope, and not all structures within the scope are applicable; in particular, the items expressly excluded by the present application are not among the candidates. The basic principle is to take the smooth implementation of the application as the screening criterion.

In the present application, the numerical interval includes both the numerical interval marked by the dash line (e.g., 1-6) and the numerical interval marked by the wavy line such as (1~6), and the "to" marked numerical interval (e.g., 1 to 6). Unless otherwise specified, an integer interval represents the group of all integers within the range of the interval, and the range includes two endpoints. For example, the number of EO units is preferably 20 to 70, and the selection range includes but is not limited to the numerical intervals represented by integers and non-integers. For another example, "integers from 1 to 6" represent groups of 1, 2, 3, 4, 5, and 6. For another example, -(CH₂)₁₋₄- represents the group consisting of -CH₂-, -(CH₂)₂-, -(CH₂)₃- and -(CH₂)₄-. For another example, 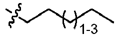 represents the group consisting of

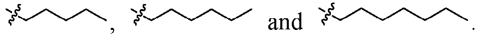

The numerical range in the present application includes but is not limited to the numerical intervals represented by integers, non-integers, percentages, and fractions, and the numerical intervals include two endpoints unless otherwise specified.

In the present application, for the molecular weight of polymers, "about" or "approximately" followed by a numerical value generally suggests a ±10% numerical range; in some cases, the numerical range can be magnified to ±15% which is no more than ±20%. Based on the preset values. For example, the deviations between 11 kDa, 12 kDa, and 10 kDa are 10% and 20%, respectively; and "about 10 kDa" includes but is not limited to 11 kDa and 12 kDa. For another example, when the molecular weight of a PEG component in the specified formula is about 5 kDa, the corresponding molecular weight or number average molecular weight is allowed to vary in the range of 5 kDa±10%, that is, 4500 to 5500 Da.

In the present application, for percentages, when the numerical value given (excluding the percent sign) is accurate to N (including 1, 0.1, 0.01, 0.001, etc.), "about", and "approximately" generally refers to the numerical range of ±0.5*N. For example, about 1% refers to the range of 0.5%-1.5% and about 2.2% refers to the range of 2.15%-2.25%.

In the present application and unless otherwise specified, divalent linking groups e.g., a hydrocarbylene group, an alkylene group, an arylene group, an amide bond, and the like, either one of the two linking ends could be chosen to be linked to another group. For example, when an amide bond serves as a divalent linking group between GroupA and GroupB, both GroupA-C(=O)NH-GroupB and GroupB-NHC(=O)-GroupA are allowable.

In the structural formula of the present application, when the atomic attribution problem of the linking group is involved, " 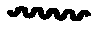 " is used to mark the linking bond. For example, 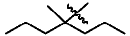 is used to denote the group structure; specifically, 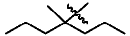 represents -CCH₃(CH₂CH₂CH₃)₂; and 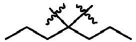 represents -C(CH₂CH₂CH₃)₂-. The non-group form 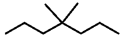 represents (CH₃)₂C(CH₂CH₂CH₃)₂.

In the present application, a number or a numerical interval written in the subscript of "C" can be used to indicate the number of carbon atoms of a group, and the number of carbon atoms does not include the contribution of a substituent group, unless otherwise indicated. For example, C₁₋₁₂ means "having 1 to 12 carbon atoms". For another example, C₁₋₁₀ alkylene group means any alkylene group with the number of carbon atoms in the range indicated by the subscript. That is, the group can be selected from the group consisting of the C₁, C₂, C₃, C₄, C₅, C₆, C₇, C₈, C₉ and C₁₀ alkylene groups, including but not limited to the linear C₁₋₁₀ alkylene group (e.g., -(CH₂)₆-) and the branched C₁₋₁₀ alkylene group (e.g., -(CH₂)₃-CH(CH₃)-(CH₂)₃-). For another example, "substituted C₁₋₁₂ alkyl group" refers to an alkyl group selected from the group consisting of C₁, C₂, C₃, C₄, C₅, C₆, C₇, C₈, C₉, C₁₀, C₁₁ and C₁₂ alkyl group with one or more hydrogen atoms being substituted, and there is no special restriction on the number of carbon atoms and heteroatoms in the substituent.

In the present application and unless otherwise specified, a structure with isomers may refer to any form of the isomers. For example, when *cis-* and *trans-* isomers are present, it can refer to either a cis-structure or a trans-structure; when *E*/*Z* isomers are present, it can refer to either an (*E*)-structure or a (*Z*)-structure; and when optical activity is present, it can refer to either a laevoisomer or a dextroisomer.

In the present application, if there is a discrepancy between the structure described herein and the name of the structure, the described structure shall have greater weight.

In the present application, "molecular weight" represents the mass of a compound. The "average molecular weight" of a compound represented by a general formula refers to the molecular mass of the compound in macroscopic matter, and unless otherwise specified, also refers to the "number average molecular weight" (Mₙ). The number average molecular weight can be used to describe the molecular weight of polydisperse blocks or substances. The measuring unit of "molecular weight" and "average molecular weight" is Dalton (Da), unless otherwise specified. The molecular weight of the polyethylene glycol chain can also be measured in "degree of polymerization" which is, specifically, the number of repeat units (oxyethylene units, i.e., EO units) in the compound molecule. Accordingly, "average degree of polymerization", and "number-average degree of polymerization" preferably represent the average value or the number average value of the number of repeating units.

In the present application, "any suitable" in "any suitable linking group", "any suitable reactive group", etc., refers to a structure that conforms to the basic principle of the chemical structure and can enable the preparation method of the present application to be successfully implemented. The chemical structure described in this way can be considered to have a clear, definite range.

In the present application, "micro-modification" refers to a process of chemical modification that can be completed through simple chemical reactions. Simple chemical reactions mainly include deprotection, salt complexation and decomplexation, ionization, protonation, deprotonation, leaving group transformation, etc.

Accordingly, "variant form with micro-modification" corresponds to the "micro-modification", referring to a structure that can be transformed into the target reactive group after simple chemical reactions selected from deprotection, salt complexation and decomplexation, ionization, protonation, deprotonation, leaving group transformation, etc. The leaving group transformation includes but is not limited to the transformation from the form of ester to the form of acid chloride.

In the present application, the terms "stable" (or "can remain stable") and "degradable" (or "can be degraded') are a pair of opposing concepts. The detailed examples of stable groups and degradable groups are given in paragraphs [0134]-[0145] in CN113402405A.

In the present application, for the PEGylated drug circulating in the blood, the ether bond (-CH₂CH₂-O-CH₂CH₂-) between the repeating units of the PEGylated component is generally considered to be stable, which can provide a reference for distinguishing "stable existence" and "degradable", but this is not a critical judgment standard. A linking group is considered stable when it is more stable in the environment than the ether bond in the PEG fragment.

In the present application, the compound of general formula (1) shall be understood to include the salt of general formula (1). The term "salt" used is derived from either, any two, or more combinations of acid adduct salts formed with inorganic and/or organic acids and alkali adduct salts formed with inorganic and/or organic bases. When a compound in general formula (1) contains an alkaline moiety (such as, but not limited to, pyridine or imidazole) and an acidic moiety (e.g., but not limited to carboxylic acids), zwitterionic ions ("inner salts") can be formed and included in the term "salt". "Salt" can be pharmaceutically acceptable (i.e., non-toxic, physiologically acceptable) salt, or other salts. The salts of the general formula (1) compound can be formed by reacting the compound of formula (1) with a certain amount (such as equivalent) of an acid or base in a medium such as salt precipitation or an aqueous medium and then lyophilized. Examples of acid addition salts include acetate, adipate, alginate, ascorbate, aspartate, benzoate, benzesulfonate, bisulfate, borate, butyrate, citrate, camphorate, camphor sulfonate, cyclopentane propionate, digluconate, lauryl sulfate, ethanesulfonate, fumarate, glucaprotane, glyceryl phosphate, hemisulfate, henanthate, caproate, hydrochloride, hydrobromide, hydroiodate, 2-hydroxyethionate, lactate, maleate, Methanesulfonate, 2-naphthalene sulfonate, niacinate, nitrate, oxalate, pectinate, persulfate, 3-phenylpropionate, phosphate, picrate, neovalerate, propionate, salicylate, succinate, sulfate, sulfonate, tartrate, thiocyanate, tosylate, undecanoate, etc.; salts with organic bases, such as organic amines, and salts with amino acids, such as arginine or lysine. Basic nitrogenous groups can be quaternized with reagents such as lower alkyl halides (e.g., methyl, ethyl, propyl, and butyl chloride, bromides, and iodides), dialkyl sulfates (e.g., dimethyl, diethyl, dibutyl, and dipentyl sulfates), long-chain halides (e.g., decyl, lauryl, tetradecyl and stearyl chlorides, bromides, and iodides), aryl alkyl halides (e.g., benzyl and phenylethyl bromide), and others. The acid and base addition salts are preferably pharmaceutically acceptable salts and, for this disclosure, are considered to be equivalent to the free forms of the corresponding compound of general formula (1).

In the present application, unless otherwise specified, "group" refers to a free radical formed by a compound losing one or more atoms, and the free radical contains at least one atom. The remaining group formed removal of other atom or group is also known as a residue with respect to the compound. The group in the present application is not a hydrogen atom. The valence of groups is not particularly limited, and examples include a monovalent group, a divalent group, a trivalent group, a tetravalent group, ..., a hectovalent group, etc. Wherein, the groups with valence being equal to or greater than two are collectively defined as "linking groups". A linking group can also contain only one atom, such as an ether group (-O-), and a thioether group (-S-). In particular, when a group can be defined as a linking group, it means that the group can be absent and only act as a link.

In the present application, unless otherwise specified, a "linking bond" refers to a bond that only acts as a connection without any atom.

In the present application, the "group" in the divalent linking group can be replaced with "bond" without changing the meaning. For example, but not limited to, divalent ether groups can also be referred to as ether bonds, divalent ester groups can also be referred to as ester bonds, and divalent carbamate groups can also be referred to as urethane bonds.

In the present application, the "substituted" means that at least one hydrogen atom is replaced by a substituent, or that one group is replaced by another. In the present application, the group used for the substitution is called a "substituted" group. The number of atoms contained in the substituted group can be one or greater than one. When the number of atoms contained in the substituted group is one, it can also be called a "substituted atom".

In the present application, when a group is replaced by another group, one or more hydrogen atoms in a group may be replaced by another group, wherein the other group is a substituted group, or a group as a whole can be replaced by another group. The specific situation is determined according to the conventional understanding of those skilled in the art. For example, "the phenyl group is replaced by a chlorine atom", in combination with different additional conditions, it can be that the phenyl group is replaced by a chlorine atom, or the phenyl group contains a chlorine atomic substituent. Another example, "the hydroxyl group is replaced by -OTs", it is generally believed that the hydroxyl group as a whole is replaced by the -OTs group.

In the present application, the "substituent" is a non-hydrogen atom or a group containing a non-hydrogen atom, the non-hydrogen atom is for example, but not limited to halogen atoms such as F, Cl, Br and I, and the group containing non-hydrogen atom is for example, but not limited to an oxygen group (=O), a hydroxyl group (-OH), a hydrocarbon oxygen group (-ORt; wherein, Rt is a hydrocarbon group), a carboxyl group (-COOH), an amine group (-NRR, two R are each independently H or a hydrocarbon group), a cyano group (-CN), an azide group (-N₃), a C₁₋₂₀ alkyl group and a cycloalkyl group.

In the present application, when the compound or group is "substituted", it means that the compound or group (for example, but not limited to an alkyl group, an alkylene group, an aliphatic hydrocarbon group, an aliphatic hydrocarbon derivative residue, an amino acid residue or a monosaccharide residue) contains one or more substituents.

In the present application, a compound, a group, or an atom can be substituted and hybridized at the same time; for example, -CH₂-CH₂-CH₂- is replaced with -CH₂-S-CH(CH₃)-.

In the present application, unless otherwise specified, "amino group" has the same meaning as "amine group", including monovalent, divalent, trivalent, tetravalent neutral group or cationic group, which is substituted or unsubstituted. For example, the -NH₂ in CH₃-NH₂ is defined as an "amino group" or "primary amine group ". For another example, the -NH- in CH₃-NH-CH₃ is defined as a "secondary amine group" or a "secondary amino group", wherein -NH-CH₃ can be understood as a methyl substituted amine group.

In the present application, "amine group" includes, but is not limited to, a primary amine group, a secondary amine group, a tertiary amine group and a quaternary ammonium ion. For example, but not limited to -NRₜRₜ and -N⁺RₜRₜRₜ, wherein each Rₜ is independently a hydrogen atom or an arbitrary hydrocarbon structure; the structure includes but is not limited to an alkyl group, an alkylene group, a cycloalkyl group, an alkenyl group, an alkynyl group, a phenyl group, etc.

In the present application, unless otherwise specified, there is no difference between the meaning of "end-capping group" and "end group", and both can be reactive groups or non-reactive groups, and can be targeted groups or non-targeted groups. In the present application, there is no special restriction on the definition of the capping group/end group of the specific polyethylene glycol chain; for example, the capping group/end group of 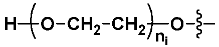 can be a hydrogen atom, can also be a hydroxyl group, and can be a hydroxyethyl group; for example, the capping group/end group of 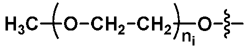 can be a methyl group, can also be a methoxy group, and can be a methoxyethyl group.

In the present application, "functional group" is preferably a reactive group, a protected reactive group, a precursor of a reactive group, and the like.

The preparation method of the present application, unless otherwise specified, the reactive group also comprises its protected form, and the protected form may be deprotected in any suitable step of the actual preparation process to obtain the corresponding active form.

In the present application, "hydrocarbon" refers to a hydrocarbon compound composed of carbon atoms and hydrogen atoms.

In the present application, hydrocarbons are classified into saturated hydrocarbons and unsaturated hydrocarbons in terms of the degree of saturation. Wherein, unsaturated hydrocarbons include hydrocarbons containing carbon-carbon double bonds, carbon-carbon triple bonds, or aromatic rings. There is no specific limit to the degree of unsaturation of unsaturated aliphatic hydrocarbons. For example, the unsaturated aliphatic hydrocarbon includes, but is not limited to alkenes (containing carbon-carbon double bonds), alkynes (containing carbon-carbon triple bonds), dienes (containing two conjugated carbon-carbon double bonds), multiolefins (containing two or more carbon-carbon double bonds), and the like.

In the present application, a "hydrocarbylene group" is a divalent hydrocarbon group.

In the present application, "aliphatic hydrocarbon group" refers to the residue of an aliphatic hydrocarbon molecule with at least one hydrogen atom being removed. Unless otherwise specified, aliphatic hydrocarbon groups refer to monovalent hydrocarbon groups in the present application. Aliphatic hydrocarbon group includes saturated aliphatic hydrocarbon groups and unsaturated aliphatic hydrocarbon groups. Unless otherwise expressly stated in this specification, aliphatic hydrocarbon groups are optionally substituted.

In the present application, the structures of hydrocarbons are not particularly limited, including linear structures, branched structures, ring-containing structures, dendritic structures, comb-like structures, hyperbranched structures, etc. Unless otherwise specified, linear structures, branched structures, and ring-containing structures correspond to linear hydrocarbons, branched hydrocarbons and cyclic hydrocarbons, respectively. Wherein, hydrocarbons that contain no rings are termed open-chain hydrocarbons.

In the present application, the source of hydrocarbon group is not particularly limited; for example, hydrocarbon group can be derived from an aliphatic hydrocarbon or an aromatic hydrocarbon, from a saturated hydrocarbon or an unsaturated hydrocarbon, from a linear hydrocarbon, a branched hydrocarbon or a cyclic hydrocarbon, or from a hydrocarbon or a heterohydrocarbon, etc. According to the degree of saturation, hydrocarbon groups can be derived from alkanes, alkenes, alkynes, dienes, etc. With respect to cyclic hydrocarbons, hydrocarbon groups can be derived from alicyclic hydrocarbons or aromatic hydrocarbons, or monocyclic hydrocarbons or polycyclic hydrocarbons. With respect to heterocyclic hydrocarbons, hydrocarbon groups can be derived from aliphatic heterocyclic hydrocarbons or aromatic heterocyclic hydrocarbons.

In the present application, an "alkane" refers to a saturated aliphatic hydrocarbon, and "alkyl group" refers to a hydrocarbon group obtained from an alkane losing a hydrogen atom at any location.

In the present application, "alkylene group" is also a divalent alkyl group, including an open-chain alkylene group and a divalent cycloalkyl group. An open-chain alkylene group refers to a divalent alkyl group without a cyclic structure, and a divalent cycloalkyl group refers to a divalent alkyl group containing a cyclic structure.

The source of the amino acid in the present application, unless otherwise specified, is not particularly limited; that is, the amino acid can be either natural or unnatural, or a mixture thereof. The configuration of the amino acid in the present application is not particularly limited, which could be L-type or D-type, or a mixture thereof.

The source of amino acids in the present application is not particularly restricted in the absence of special indication, and can be both natural source and unnatural source, and can also be a mixture of the two. The amino acid structure type in the present application is not particularly restricted in the absence of special indication, and can refer to both L-type and D-type, and can also be a mixture of the two.

In the present application, "bio-related substances" include, but are not limited to substances described, enumerated and cited in the references CN104877127A, WO/2016/206540A, CN201610252378.X (CN106967213A), CN201710125672.9, CN201710126727.8 and each cited literature. In general, bio-related substances include, but are not limited to the following: drugs, proteins, peptides, oligopeptides, protein mimics, fragments and analogues, enzymes, antigens, antibodies and their fragments, receptors, small molecule drugs, nucleosides, nucleotides, oligonucleotides, antisense oligonucleotides, polynucleotides, nucleic acids, aptamers, polysaccharides, proteoglycans, glycoproteins, steroids, steroids compounds, lipid compounds, hormones, vitamins, phospholipids, glycolipids, dyes, fluorescent substances, targeting factors, targeting molecules, cytokines, Neurotransmitters, extracellular matrix substances, plant or animal extracts, viruses, vaccines, cells, vesicles, liposomes, micelles, etc. The bio-related substance may be the bio-related substance itself, or the precursor, activating state, derivative, isomer, mutant, analogue, mimic, polymorph, pharmacologically acceptable salt, fusion protein, chemically modified substance, genetic recombinant substance thereof, etc., and may also be the corresponding agonist, activator, activator, inhibitor, antagonist, regulator, receptor, ligand or ligand, antibody and its fragment, acting enzyme (such as kinase, hydrolase, lyase, oxygen reductase, isomerase, transferase, deaminase, deimidase, invertase, synthase, etc.), substrates of enzymes (such as coagulation cascade protease substrates, etc.), etc. The derivatives include but are not limited to, glycosides, nucleosides, amino acids, and peptide derivatives. Chemical modification products that form new reactive groups, that is, modified products generated after modifying reactive groups and changing their types, and additionally introducing functional groups, reactive groups, amino acids or amino acid derivatives, polypeptides, etc., are all chemical modifications of bio-related substances. Before or after binding to functionalized polyethylene glycol, bio-related substances are also allowed to have target molecules, appendages, or delivery vehicles bound to them to form modified biologically related substances or complex bio-related substances. Wherein, the pharmacologically acceptable salts may be inorganic salts, such as hydrochloride, sulfate, phosphate, or organic salts, such as oxalate, malate, citrate, etc. Wherein, "drug" in the present application includes any agent, compound, composition or mixture that provides physiological or pharmacological effects in or in vitro, and often provides beneficial effects. There is no specific restriction on the types thereof, including but not limited to drugs, vaccines, antibodies, vitamins, foods, food additives, nutrients, nutraceuticals and other agents that provide beneficial effects. There is no particular restriction on the scope of physiological or pharmacological effects produced by said "drug" in vivo, which can be a systemic effect or only a local effect. There is no special restriction on the activity of the "drug", which is mainly an active substance that can interact with other substances, or an inert substance that does not interact; however, inert drugs can be converted into an active form by acting in the body or by certain stimulation. Wherein, "small molecule drugs" are bio-related substances with a molecular weight of no more than 1000 Da, or small molecule mimics or active fragments of any bio-related substances.

In the present application, "amino acid residue" includes an amino acid from which, a hydrogen atom has been removed from the amino group and/or from which, a hydroxyl group has been removed from the carboxyl group and/or from which, a hydrogen atom has been removed from the sulfhydryl group and/or from which, an amino group is protected and/or a carboxyl group is protected and/or a sulfhydryl group is protected. The amino acid residues may be referred to as amino acids in the present application.

In the present application, "targeted delivery" or "target" in verb form refers to the process by which the reagent facilitates delivery to a specific organ, tissue, cell, and/or intracellular compartment (known as the target location) so that the target location is delivered more than any other organ, tissue, cell, or intracellular compartment (known as the non-target location). Targeted delivery can be detected by methods known in the art, such as by comparing the concentration of the reagent delivered in the target cell population after systemic administration with the concentration of the reagent delivered in the non-target cell population. In some embodiments, targeted delivery results in at least two-fold higher concentrations at the target location compared to the non-target location.

In the present application, "targeted group" is a group that can interact with the complementary binding moiety at the desired location and/or under the desired conditions. For example, complementary binding moieties can be ligands and antiligands (e.g., streptavidin antibiotics and biotin, proteins A or G and the Fe region of immunoglobulins), ligands and receptors (e.g., small molecule ligands and their receptors, or sugar-exogenous lectin interactions), phage display-derived peptides, complementary nucleic acids (e.g., DNA hybrids, RNA hybrids, DNA/RNA hybrids, etc.), and aptamers. Other exemplary complementary bonding fractions include but are not limited to, those exhibiting complementary charges, hydrophobicity, hydrogen bonding, covalent bonding, van der Waals force, reactive chemistry, electrostatic interactions, magnetic interactions, etc.

In some specific embodiments of the present application, the reaction process also involves the "protection" and "deprotection" processes of related groups. To prevent a reactive group from influencing the reaction, the reactive group is usually protected. In some specific embodiments of the present application, when there are more than two reactive groups, only the target reactive groups are selectively reacted, so other reactive groups are protected. The protecting group is not only stable during the target reaction, but can be removed by conventional technical means in the art as needed.

In the present application, the "protection" of reactive groups refers to the strategy of reversibly converting the reactive groups to be protected into inert groups (non-reactive groups) by specific reagents. The part of the protected group that is different from the unprotected form is called the "protecting group". For example, -OTBS is a protected form of hydroxyl group (-OH); wherein, the -TBS group is the protecting group of the hydroxyl group.

In the present application, "hydroxyl protecting group" includes all the groups that can be used as common hydroxyl protecting groups. Hydroxy protecting group is preferably selected from the group consisting of an alkanoyl group (e.g., an acetyl group, a butyryl group), an aromatic alkanoyl group (e.g., a benzoyl group), a benzyl group, a triphenylmethyl group, a trimethylsilyl group, a t-butyldimethylsilyl group, an allyl group, an acetal group, and a ketal group. Removal of acetyl groups is generally carried out under basic conditions, most commonly by the ammonolysis with NH₃/MeOH or by the methanolysis catalyzed by methanol anion. The benzyl group can be easily removed via palladium-catalyzed hydrogenolysis in a neutral solution at room temperature, or via a reduction reaction by sodium metal in ethanol or liquid ammonia. The triphenylmethyl group is generally removed via catalytic hydrogenolysis. The trimethylsilyl groups are generally removed using reagents containing fluoride ions (e.g., tetrabutylammonium fluoride/anhydrous THF, etc.). The t-butyldimethylsilyl ether is relatively stable and can withstand ester hydrolysis conditions with alcoholic potassium hydroxide and mild reduction conditions (e.g., Zn/CH₃OH and the like) so that it can be removed by fluoride ions (e.g., Bu₄N⁺F⁻) in THF solution or by aqueous acetic acid at room temperature. The protection of glycols preferably forms dioxolane, dioxane, cyclic carbonate or cyclic borate.

In the present application, "carboxyl protecting group" refers to the protecting group which can be transformed into a carboxyl group via the hydrolysis or the deprotection reaction of the carboxyl protecting group itself. Carboxyl protecting group is preferably selected from the group consisting of an alkyl group (e.g., a methyl group, an ethyl group, and a butyl group) and an aralkyl group (e.g., a benzyl group), and more preferably selected from the group consisting of a butyl group (tBu), a methyl group (Me), and an ethyl group (Et). In the present application, "protected carboxyl group" refers to the group protected by an appropriate carboxyl protecting group, which is preferably selected from the group consisting of a methoxycarbonyl group, an ethoxycarbonyl group, *t*-butoxycarbonyl group, and a carbobenzyloxy group. The carboxyl protecting groups can be removed through hydrolysis catalyzed by acids or alkalis, or through pyrolysis reactions occasionally; for example, the *t*-butyl groups can be removed under mild acidic conditions, and the benzyl group can be removed by hydrogenolysis. The reagent used for removal of carboxyl protecting groups is selected from the group consisting of TFA, H₂O, LiOH, NaOH, KOH, MeOH, EtOH, and combinations thereof, and preferably selected from the group consisting of the combination of TFA and H₂O, the combination of LiOH and MeOH, and the combination of LiOH and EtOH. A protected carboxyl group can undergo deprotection and then produce the corresponding free acid; the deprotection is conducted in the presence of an alkali, and the alkali forms pharmaceutically acceptable salt with the free acid produced via the deprotection.

In the present application, "amino protecting group" as same as "amine protecting group" that includes all the groups which are used as amino/amine protecting groups generally, such as an aryl C₁₋₆ alkyl group, a C₁₋₆ alkoxy C₁₋₆ alkyl group, a C₁₋₆ alkoxycarbonyl group, an aryloxycarbonyl group, a C₁₋₆ alkylsulfonyl group, an alkoxycarbonyl group, a silyl group, etc. An amino protecting group is preferably selected from the group consisting of a *t*-butoxycarbonyl group (Boc), a*p*-methoxybenzyloxycarbonyl group (Moz), and a 9-fluorene-methylene-carbonyl (Fmoc). The reagent used for removal of amino protecting groups is selected from the group consisting of TFA, H₂O, LiOH, NaOH, KOH, MeOH, EtOH, and combinations thereof, and preferably selected from the group consisting of the combination of TFA and H₂O, the combination of LiOH and MeOH, and the combination of LiOH and EtOH. The reagent used for removal of the protecting group of Boc can be TFA or HCl/EA, preferably TFA. The reagent used for the removal of the protecting group of Fmoc can be the *N,N*-dimethylformamide (DMF) solution containing 20% piperidine.

In the present application, the hydroxyl groups protected by hydroxyl protecting groups are not particularly limited, e.g., alcoholic hydroxyl groups, phenolic hydroxyl groups, and the like. The amino groups protected by amino protecting groups are not particularly limited, such as those from primary amines, secondary amines, hydrazines, and amides. Amine groups in the present application are not particularly limited, including but not limited to primary amine groups, secondary amine groups, tertiary amine groups, and quaternary ammonium ions.

In the present application, the deprotection of protected hydroxyl groups is related to the types of hydroxyl protecting groups. The types of hydroxyl protecting groups are not particularly limited; for example, benzyl groups, silyl ether, tert-butyl groups can be used to protect terminal hydroxyl groups, and the corresponding deprotection methods include the following:

### A: deprotection of benzyl protection groups

The deprotection of benzyl groups can be achieved via hydrogenation using a hydrogenative reducing agent and a hydrogen donor. As used herein, the water content should be less than 1% to facilitate the reaction.

The hydrogenative reduction agent is not particularly limited, preferably palladium or nickel. The agent carrier is not particularly limited, preferably alumina or carbon, and more preferably carbon. The amount of palladium is 1 to 100 wt% of that of compounds containing protected hydroxyl groups, preferably 1 to 20 wt%.

The reaction solvent is not particularly limited, as long as it allows the reagents and the products to be dissolved. Preferable solvents include methanol, ethanol, ethyl acetate, tetrahydrofuran, and acetic acid; wherein, methanol is more preferable. The hydrogen donor is not particularly limited, preferably hydrogen gas, cyclohexene, 2-propanol, ammonium formate, or the like. The reaction temperature is preferably 25 to 40 °C. The reaction time is not particularly limited, which is negatively correlated with the amount of catalyst used and preferably 1 to 5 h.

### B: deprotection of acetal and ketal groups

Acetal or ketal compounds used to protect hydroxyl groups are preferably ethyl vinyl ether, tetrahydropyran, acetone, 2,2-dimethoxypropane, benzaldehyde, etc. The deprotection of acetal and ketal protecting group is achieved under acidic conditions, and the pH of the solution is preferably 0 to 4. There is no special restriction on acid, but acetic acid, phosphoric acid, sulfuric acid, hydrochloric acid, and nitric acid are preferred, and hydrochloric acid is preferred. There is no special restriction on the reaction solvent, as long as the reactants and products can be dissolved, and water is preferred. The reaction temperature is preferably 0 to 30 °C.

### C: deprotection of silyl ether protecting group

Compounds used for this type of hydroxyl protection include trimethylsilyl ether, triethylsilyl ether, tert-butyldimethylsilyl ether, tert-butyldiphenylsilyl ether, and the like. The deprotection of such silyl ethers uses compounds containing fluoride ions; wherein, the compound is preferably tetrabutylammonium fluoride, tetraethylammonium fluoride, hydrofluoric acid, or potassium fluoride, and more preferably tetrabutylammonium fluoride or potassium fluoride. The amount of the fluorine-containing compound is 5 to 20 folds of that of the protected hydroxyl group and preferably 8 to 15 folds of that of the initiator. When the amount of the fluorine-containing compound used is less than 5 molar equivalents per molar equivalent of protected hydroxyl groups, the deprotonation might not be complete. When the amount of deprotection reagent used exceeds 20 molar equivalents per molar equivalent of the initiator, the excess reagent tends to cause difficulty in the purification process and result in side reactions in subsequent steps. The reaction solvent is not particularly limited as long as it can dissolve the reagents and the products, preferably an aprotic solvent, and more preferably tetrahydrofuran or dichloromethane. The reaction temperature is preferably 0 to 30 °C; when it is lower than 0 °C, the reaction rate is relatively slow, and the protective group cannot be completely removed.

### D: deprotection of t-butyl protection groups

The deprotection of the tert-butyl group is carried out under an acidic condition, and the pH of the solution is preferably 0 to 4. The acid is not particularly limited but is preferably acetic acid, phosphoric acid, sulfuric acid, hydrochloric acid or nitric acid, and more preferably hydrochloric acid. The reaction solvent is not particularly limited as long as it can dissolve the reagents and the products, preferably water. The reaction temperature is preferably 0 to 30 °C.

In the present application, "carboxyl activation" refers to the activation of carboxyl groups with carboxyl activators, and the carboxyl groups can promote better progress of the condensation reaction after activation, such as: inhibiting the generation of racemic impurities in the condensation reaction, catalyzing the acceleration of the reaction speed, etc. "Carboxyl activating group" is the residue of a carboxyl activator. The carboxyl activator is selected from the group consisting of *N*-hydroxysuccinimide (NHS), 1-ethyl-(3-dimethylaminopropyl) carboyldiimide hydrochloride (EDCI), *N*-hydroxy-5-norbornene-2,3-dicarboxylimide (HONb), *N,N*-dicyclohexylcarbodiimide (DCC) and the combinations thereof, preferably the combination of NHS/EDCI, NHS/DCC, HONb/DCC, and most preferably the combination of NHS/EDCI.

In the present application, the condensing agent is not particularly limited, preferably *N,N*'-dicyclohexylcarbodiimide (DCC), 1-ethyl-(3-dimethylaminopropyl)carbodiimide hydrochloride (EDC·HCl), 2-(7-azobenzotriazole-*N*,*N*,*N'*,*N'*-tetramethyluronium hexafluorophosphate (HATU), or 2-(1*H*-benzotriazol-1-yl)-1,1,3,3-tetramethyluronium hexafluorophosphate (HBTU), and most preferably DDC. Generally, the molar equivalent of the condensing agent is 1 to 20 folds of that of the carboxylic acid, preferably 5 to 10 folds, and suitable catalysts such as 4-dimethylaminopyridine (DMAP) can be added to the reaction.

In the present application, the oxidizing agent is not particularly limited as long as it is a compound or a combination of multiple compounds capable of increasing the valence of the substrate, preferably phenyliodine(III) bis(trifluoroacetate), 1,4-benzoquinone, benzyl trimethyl ammonium tribromide, pyridinium dichromate, ozone, oxygen, hydrofluoric acid, sodium hypochlorite, cobaltic acetate, cobalt acetate, manganous acetate, palladium(II) acetate, cupric acetate, monoperoxyphthalic acid, iodine, *N*-iodosuccinimide, iodoxybenzene, 2-iodylbenzoic acid, dimethyldioxirane, dimethyl sulfoxide-oxalyl chloride, DDQ, dichlorotris(triphenylphosphine)ruthenium, manganese dioxide, (diacetoxyiodo)benzene, periodic acid, sodium periodate, sodium periodate-osmium tetraoxide, potassium permanganate, sodium perborate, perbenzoic acid, dibenzoyl peroxide, nickel peroxide, hydrogen peroxide, cumyl hydroperoxide, 1-butyl hydroperoxide, peracetic acid, *m*-chloroperbenzoic acid, *N*-chlorosuccinimide, pyridinium chlorochromate, palladium chloride-cupric chloride, urea hydrogen peroxide adduct, triphenylcarbenium tetrafluoroborate, tributyltin oxide, cobalt trifluoride, vanadium oxytrifluoride, chromium trioxide, manganese triacetate, TEMPO, diammonium cerium nitrate, bromine, pyridine *N*-oxide, silver oxide, *O*-ethylperoxycarbonic acid, manganese acetyllacetonate, vanadyl acetylacetonate, aluminium isopropoxide, peroxymonosulfate, dichloroiodobenzene, the like, or any combination thereof, and more preferably oxygen, sodium hypochlorite, hydrogen peroxide, dichloroiodobenzene, peroxymonosulfate, the like, or any combination thereof; the molar equivalent of the oxidizing agent is 1 to 50 folds of that of the hydroxyl group of the intermediate compound, preferably 1 to 20 folds, and more preferably 5 to 10 folds.

In the present application, the reducing agent is not particularly limited as long as it can reduce the Schiff base formed by the reaction of an amine with an aldehyde or ketone to an amino group, preferably sodium borohydride, sodium cyanoborohydride, lithium aluminum hydride, borane, diborane, diisobutylaluminum hydride, diisopinocampheylborane, lithium borohydride, zinc borohydride, borane-pyridine, borane-methyl sulfide, borane-tetrahydrofuran, the like, or any combination thereof, and more preferably sodium cyanoborohydride; the molar equivalent of the reducing agent is 1 to 50 folds of that of the amino group to be modified, preferably 1 to 20 folds, and more preferably 5 to 10 folds.

In the present application, the reaction temperature is 0 to 200 °C, preferably 0 to 100 °C, and more preferably 0 to 25 °C; the reaction time is preferably 10 min to 48 h, and more preferably 30 min to 24 h. The obtained product can be purified by purification means such as extraction, recrystallization, adsorption treatment, precipitation, reverse precipitation, membrane dialysis, and supercritical extraction.

In the present application, the reaction solvent can be absent or an aprotic solvent including toluene, benzene, xylene, acetonitrile, ethyl acetate, diethyl ether, tert-butyl methyl ether, tetrahydrofuran, chloroform, dichloromethane, dimethylsulfoxide, dimethylformamide, and dimethylacetamide; the aprotic solvent is preferably tetrahydrofuran, dichloromethane, dimethylsulfoxide, or dimethylformamide.

In the present application, the bases used in reactions are generally organic bases (such as triethylamine, pyridine, 4-dimethylaminopyridine, imidazole or diisopropyl ethylamine); preferably Triethylamine, or pyridine. The molar equivalent of the base is 1 to 50 folds of that of carboxylic acids, preferably 1 to 10 folds, and more preferably 2 to 3 folds.

In the present application, the repeating unit of the polyethylene glycol component is an oxyethylene unit, namely -CH₂CH₂O- or - OCH₂CH₂-, which is also denoted as EO unit, the number of repeating units is also denoted as the number of EO units; the average number of repeating units is also denoted as the average number of EO units; and the average number of preferred units is averaged.

In the case of polydispersity, the molecular weight/degree of polymerization of a single molecule of a compound, the number average molecular weight/number average polymerization degree of the compound components in a macroscopic substance is "equal" or "identical" (including other forms of equivalent expression), in the case of no special designation, is not limited to strict equality in numerical value, but the exponential value is close or approximately equal, and the preferred deviation of the similarity or approximate equality does not exceed ±10%, usually based on a preset value.

In the present application, for the case of monodispersion, the number of vinyl oxide units in a single compound molecule and the general formula is the same or equal means that they are strictly equal in numerical terms; for example, the number of EO units of a certain PEG component is set to 11, the set value equal to 12 does not fall into the setting range; Limitations of the purification method may result in the macroscopic product containing EO element array components other than the target EO element array component, in which case a monodisperse macroscopic product containing the target component is considered to be obtained when the average EO cell deviates from the preset number of EO cells by no more than ±5% (base≥10) or no more than ±0.5 (base<10), and when the content of the components that meet the range of EO cell number or EO cell average reaches a certain percentage (preferably ≥ 90%, and more preferably > 95%, more preferably greater than 96%, more preferably greater than 98%, more preferably 99 to 100%), and also regarded as obtaining a monodisperse macroscopic product containing the target component. Even if the above-mentioned content ratio is not reached, as long as the preparation method of the present application or a similar method that adopts the preparation idea of basically the same, the product with insufficient content obtained for any reason, the component in the form of the main product, the co-product or the by-product, whether or not separation and purification, are within the scope of the present application.

In the present application, when the molecular weight of the general formula of a polydisperse component is described by Da, kDa, the number of repeating units, and the number of EO units, for a single compound molecule, the molecular weight value is allowed to fall within a certain range of the given value (including the endpoint, preferably 10% ± When the preset molecular weight of the compound general formula of the monodisperse component is described by the number of oxyethylene units, there is no range fluctuation and is a discrete point, but the preparation product may fluctuate the average number of EO units within a certain range due to the non-uniformity of molecular weight (no more than ±10% or ±1, preferably no more than ±5% or ±0.5). For example, the molecular weight of mPEG (methoxy polyethylene glycol unit) is 5 kDa, which means that the molecular weight value of a single molecule in the general formula is between 4500 to 5500 Da, and the average molecular weight of the corresponding components of the corresponding prepared product is 5 kDa, that is, the average molecular weight value is 4500 to 5500Da. If the design mPEG has 22 oxyethylene units, the number of EO units of all compound molecules in the general formula should be strictly 22, but the preparation product may be a mixture of compounds with 20, 21, 22, 23, and 24 EO units, and the average number of EO units falls within the range of 22±2.2 (preferably the range of 22±1.1), and components whose molecular weight fall within this range can be considered as target components for purity calculation.

In the present application, unless otherwise specified, "mPEG" is a methoxy-terminated polyethylene glycol chain segment, and its structural formula represented by 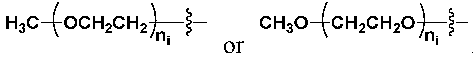 wherein, nᵢ is the degree of polymerization of the polyethylene glycol chain, which is selected from the integer from 1 to 250.

In the present application, the PDI<1.005 of products can be regarded as monodisperse, and can be recorded as PDI=1.

In the present application, the number of repeating units in the "single-chain component" is at least two.

In the present invention, the "lipid" includes but is not limited to the esters of fatty acids, and is generally characterized by poorer solubility in water and better solubility in many nonpolar organics. Although lipids usually have poorer solubility in water, lipids of particular types (e.g., lipids modified with polar groups, such as DMG-PEG2000) have limited water solubility, and can be dissolved in water under certain conditions. The known types of lipids include biomolecules such as fatty acids, waxes, sterols, fat-soluble vitamins (e.g., vitamins A, D, E, and K), monoglycerides, diglycerides, triglycerides, and phospholipids.

In the present invention, lipids include simple esters, compound esters, and derived lipids. Said simple esters are esters formed by fatty acids and alcohols, which can also be classified into three subcategories: fats, oils, and waxes. Said compound esters are "lipoid compounds" which are also termed "lipoids", including phospholipids, sphingolipids, glycolipids, steroids, sterols, and lipoproteins. Said derived lipids include derivatives of simple and compound lipids, having the general properties of lipids.

In the present invention, lipids can be synthetic or derived (separated or modified) from natural sources or compounds.

In the present invention, the term "cation" refers to a corresponding structure that is positively charged either permanently or non-permanently in response to certain conditions (e.g., pH). Therefore, cations include not only permanent cations but also those cationisable. Permanent cations refer to the corresponding compounds, groups, or atoms that are positively charged under conditions of any pH value or hydrogen ion activity of its environment. As a typical example, the presence of a quaternary nitrogen atom results in a positive charge. When a compound carries multiple positive charges, it can also be termed a permanent cation. A cationisable substance refers to a compound, group, or atom that is positively charged at a lower pH and uncharged at a higher pH of its environment. Moreover, in non-aqueous environments where the pH cannot be determined, a cationisable compound, group, or atom is positively charged at a high concentration of hydrogen ions and uncharged at a low concentration or activity of hydrogen ions. It depends on the individual properties of the cationisable or polycationisable compound, in particular the pKa of the respective cationisable group or atom, at which pH or hydrogen ion concentration said compound is charged or uncharged. In diluted aqueous environments, the fraction of cationisable compounds, groups, or atoms that are positively charged may be estimated using the so-called Henderson-Hasselbalch equation which is well-known to a person skilled in the art. In some embodiments, a cationisable compound or its moiety is positively charged at the physiological pH (e.g., about 7.0-7.4). In some preferred embodiments, a cationisable compound or its moiety is neutral at the physiological pH (e.g., about 7.0-7.4), but becomes positively charged at a lower pH (e.g., about 5.5-6.5). In some embodiments, the preferred range of pKa of the cationisable compound or its moiety is from about 5 to about 7.

In the present application, "cationic lipid" means a lipid that is positively charged at any pH or hydrogen ion activity of the environment in which it is located, or a lipid that is positively charged in response to the pH or hydrogen ion activity of the environment in which it is located (e.g., the environment in which it is intended to be used). Thus, the term "cation" covers the range of "permanent cation" and "cationizable". In some embodiments, the positive charge in cationic lipid arises from the presence of quaternary nitrogen atoms. In some embodiments, cationic lipid include zwitterionic lipid that are positively charged in the environment in which they are intended to be administered (e.g., at endosomal pH). In some embodiments, if a lipid composition contains a cationizable lipid, the pH is preferably 1 to 9, 4 to 9, 5 to 8, or 6 to 8, and preferably at an endosome pH (e.g., about 5.5 to 6.5), wherein approximately 1% to 100% of the cationizable lipid is cationized.

Cationic lipids include, but are not limited to *N,N*-dioleyl-*N,N*-dimethylammonium chloride (DODAC), *N*,*N*-distearyl-*N*,*N*-dimethylammonium bromide (DDAB), *N*-(1-(2,3-dioleoyloxy)propyl)-*N*,*N*,*N*-trimethylammonium chloride (DOTAP), *N*-(1-(2,3-dioleyloxy)propyl)-*N*,*N*,*N*-trimethylammonium chloride (DOTMA), *N*,*N*-dimethyl-2, 3-Dioilyloxypropylamine (DODMA), 3-(didodecylamino)-*N*₁,*N*₁,4-tridodecyl-1-piperazineethanamine (KL10), *N*-[2(didodecylamino)ethyl]-*N*₁,*N*₄,*N*₄-tridodecyl-1,4-piperazinediethanamine (KL22), 14,25-ditridecyl-15,18,21,24-tetraaza-octatriacontane (KL25), 1,2-Dileutheroxy-N, *N*-Dimethylaminopropane (DLin-DMA), 2,2-Dilinoleyl-4-dimethylaminomethyl-[1,3]-dioxolane (DLin-K-DMA), heptatriaconta-6,9,28,31-tetraen-19-yl 4-(dimethylamino)butanoate (DLin-MC3-DMA) and 2,2-dilinoleyl-4-(2dimethylaminoethyl)-[1,3]-dioxolane (DLin-KC2-DMA), ((4-hydroxybutyl)azanediyl)bis(hexane-6,1-diyl)bis(2-hexyldecanoate) (ALC-0315), heptadecan-9-yl 8-((2-hydroxyethyl) (6-oxo-6-(undecyloxy) hexyl) amino) octanoate) (SM102) and any mixture thereof, or any of the disclosed cationic lipids of CN113402405A and their combinations.

In the present application, "PEG-lipid" (i.e., PEGylated lipid) refers to the molecules containing both a lipid and a PEG moiety. In addition to the structural general formula (1) of the present application, PEGylated lipids include, but are not limited to 1,2-dimyristoyl-sn-glycerol methoxypolyethylene glycol (PEG-DMG), 1,2-distearoyl-sn-glycero-3-phosphoethanolamine-N-[amino(polyethylene glycol)] (PEG-DSPE), PEG-cholesterol, PEG-diacylglycamide (PEG-DAG), PEG-dialkyloxypropyl (PEG-DAA), PEG500-dipalmitoylphosphatidylcholine, PEG2000-dipalmitoylphosphatidylcholine, PEG500-stearylphosphatidylethanolamine PEG2000-distearylphosphatidylethanolamine, PEG500-1,2-oleoylphosphatidylethanolamine, PEG2000-1,2-oleoylphosphatidylethanolamine, PEG2000-2,3-distearoylglycerol (PEG-DMG), and combinations thereof.

In the present application, "neutral lipid" refers to any lipid substance that is uncharged or exists in the form of neutral zwitterion at the chosen pH, preferably phospholipid. The neutral lipid can be synthetic or natural.

In the present application, "steroid lipid" refers to a steroid or steroid analog.

In the present application, "N/P ratio" refers to a molar ratio of nitrogen atoms in cationic lipid to phosphates in nucleic acids.

In the present application, "nucleic acid" refers to DNA, RNA, or the modified form thereof.

In the present application, "RNA" refers to a ribonucleic acid that may be naturally or non-naturally occurring. For example, an RNA may include modified and/or non-naturally occurring components such as one or more nucleobases, nucleosides, nucleotides, and linkers. An RNA may include a cap structure, a chain-terminating nucleoside, a stem-loop, a polyA sequence, and/or a polyadenylation signal. An RNA may have a nucleotide sequence encoding a polypeptide of interest. For example, an RNA may be a messenger RNA (mRNA). Translation of an mRNA encoding a particular polypeptide, for example, in vivo translation of an mRNA inside a mammalian cell, may produce the encoded polypeptide. RNAs may be selected from the non-limiting group consisting of small interfering RNA (siRNA), asymmetrical interfering RNA (aiRNA), microRNA (miRNA), Dicer-substrate RNA (dsRNA), small hairpin RNA (shRNA), mRNA (messenger RNA), single guide RNA (sgRNA), cas9 mRNA, and mixtures thereof. In the present application, FLuc mRNA can express luciferase protein which emits bioluminescence in the presence of fluorescein substrate, so FLuc is commonly used in mammalian cell culture to measure gene expression and cell activity. In the present application, suitable assays for determining the level of target gene expression include but are not limited to dot blots, northern blots, in situ hybridization, ELISA, immunoprecipitation, enzyme function, and phenotypic assays.

In the present application, "transfection" refers to the introduction of a species (e.g., an RNA) into a cell. Transfection may occur, such as, in vitro, ex vivo, or in vivo.

In the present application, "antigen" typically refers to a substance that can be recognized by the immune system, preferably recognized by the adaptive immune system, and trigger an antigen-specific immune response, for example, forming antibodies and/or antigen-specific T cells as a part of the adaptive immune response. Typically, the antigen may be or may contain a peptide or a protein that can be presented to T cells by MHC. In the sense of the present application, the antigen may be a translated product of the provided nucleic acid molecule (preferably mRNA as defined herein). In this context, fragments, variants, and derivatives of peptides and proteins containing at least one epitope are also defined as antigens.

In the present application, "delivery" refers to delivering an entity to the target; for example, delivering drugs and/or therapeutic agents and/or prophylactic agents to subjects, the subjects are tissues and/or cells of humans and/or other animals.

In the present application, "lipid composition" is a composition containing a variety of lipid components.

In the present application, "lipid nanoparticle" or "LNP" refers to nanoscale (e.g., 1 nm to 1000 nm) particles containing one or more types of lipid molecules. In the present application, LNP may further contain at least one drug molecule. For example, "LNP pharmaceutical composition" is the LNP that encapsulates the drug molecule, and "LNP-mRNA" is the LNP that encapsulates the mRNA.

In the present application, "formulation of lipid pharmaceutical composition" means a pharmaceutically acceptable carrier containing in addition to an LNP containing an encapsulated drug molecule.

In the present application, "pharmaceutically acceptable carrier" refers to a diluent, adjuvant, excipient, or vehicle administered together with the therapeutic agent, which is, within the scope of reasonable medical judgment, suitable for contact with tissues of human and/or other animals without causing excessive toxicity, irritation, allergic reaction, or other problems or complications corresponding to reasonable benefit/risk ratio. Pharmaceutically acceptable carriers that can be used in the pharmaceutical composition in the present application include but are not limited to sterile liquids, such as water and oil, including those from petroleum, animal, vegetable, or synthesis, e.g., peanut oil, soybean oil, mineral oil, sesame oil, etc. When the pharmaceutical composition is administered intravenously, water is an exemplary carrier. Physiological saline, glucose, and aqueous glycerol solution can also be used as liquid carriers, especially as an injection. Suitable pharmaceutical excipients include starch, glucose, lactose, sucrose, gelatin, maltose, chalk, silica gel, sodium stearate, glyceryl monostearate, talc, sodium chloride, defatted milk powder, glycerol, propylene glycol, water, ethanol, etc. The composition can also contain a small amount of humectant, emulsifier, or pH buffer as needed. Oral formulation can contain standard carrier, such as pharmaceutical-grade mannitol, lactose, starch, magnesium stearate, sodium saccharin, cellulose, magnesium carbonate, etc. Specifically, excipients include but are not limited to anti-adherents, antioxidants, binders, coatings, compression aids, disintegrants, dyes (pigments), emollients, emulsifiers, fillers (diluents), film formers or coatings, flavors, fragrances, glidants (flow enhancers), lubricants, preservatives, printing inks, sorbents, suspending or dispersing agents, sweeteners, and waters of hydration. More specifically, excipients include but are not limited to butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, and xylitol.

In the present application, pharmaceutical compositions can act systematically or locally. For this purpose, they can be administered by appropriate routes such as injection (e.g., intravenous, intraarterial, subcutaneous, intraperitoneal, or intramuscular injection, including instillation) or transdermal delivery, and can also be administered by oral, buccal, transnasal, transmucosal, topical routes, in the form of ophthalmic preparation, or by inhalation. Regarding these routes of administration, the pharmaceutical compositions of the present application can be administered in suitable dosage forms. The dosage forms include but are not limited to, tablets, capsules, lozenges, hard sugar agents, powders, sprays, creams, ointments, suppositories, gels, pastes, lotions, ointments, aqueous suspensions, injectable solutions, elixirs, and syrups.

In the present application, vaccines are preventive or therapeutic materials that provide at least one antigen or antigenic function. Antigen or antigenic function can stimulate the body's adaptive immune system to provide an adaptive immune response.

### An embodiment of the present application:

### 1.1. A PEGylated lipid, wherein its structure is represented by the general formula (1):

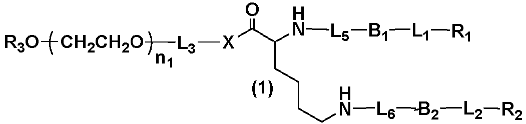

wherein,
X is -O- or -NH-;
L₁ and L₂ are each independently selected from the group consisting of a linking bond, -C(=O)-, -C(=O)O-, -OC(=O)-, -OC(=O)O-, -C(=O)NH-, -NHC(=O)-, -NHC(=O)O-, -OC(=O)NH- and -NHC(=O)NH-;
L₅ and L₆ are each independently selected from the group consisting of a linking bond, -C(=O)- and -C(=O)O-;
L₃ is a linking bond or a divalent linking group; the divalent linking group is selected from the group consisting of -(CH₂)ₜ-, -O-, -C(=O)O-, -C(=O)O-, -OC(=O)O-, -OC(=O)O-, -C(=O)NH-, -NHC(=O)-, -NHC(=O)O-, -OC(=O)NH-, -NHC(=O)NH- and any combinations thereof, wherein, t is an integer from 1 to 12;
B₁ and B₂ are each independently a linking bond or a C₁₋₁₂ alkylene group;
R₁ and R₂ are each independently a C₅₋₃₀ aliphatic hydrocarbon group; wherein, the aliphatic hydrocarbon group is an alkyl group, an alkene group or an alkyne group;
R₃ is a hydrogen atom, a C₁₋₆ alkyl group, a carbocyclic group, a heterocyclic group or -L₄-R₀₁; wherein, L₄ is a divalent linking group and R₀₁ is a functional group that can react with bio-related substance;
n₁ is an integer from 20 to 1000.

In the present application, the compound represented by general formula (1) can exist in a non-solvated or solvated form, including a hydrated form. Generally speaking, for the present application, a solvated form with a pharmaceutically acceptable solvent (such as water, ethanol, etc.) is equivalent to a non-solvated form.

In the present application, the compound represented by general formula (1) and a salt or solvate thereof may exist in their tautomeric forms, including ketone-enol interchange, amide-iminoic acid interchange, lactam-lactimide interchange, enamine-imine interchange, proton transfer interchange and valence interchange.

In the present application, a compound represented by general formula (1) shall contain a salt, tautomer, stereoisomer or solvate thereof.

### 1.2.1. X

In the present application, X is -O- or -NH-.

### 1.2.2. Polyethylene glycol chains

In a specific embodiment of the present application, the number of average molecular weight of polyethylene glycol chain preferably corresponds to 900, 1000, 1500, 2000, 2500, 3000, 3350, 3500, 4000, 5000, 5500, 6000, 6500, 7000, 7500, 8000, 8500, 9000, 9500, 10000 or 11000.

In a specific embodiment of the present application, the polyethylene glycol chain is preferably polydisperse or monodisperse, satisfying any of the following cases:
Case (1): the polyethylene glycol chain is polydisperse, and the number average degree of polymerization of n₁ is preferably an integer from 20 to 100, more preferably an integer from 20 to 60, and more preferably an integer from 40 to 60;
Case (2): the polyethylene glycol chain is monodisperse, and the number of EO units is preferably 20 to 70, more preferably 20 to 60, and more preferably 20, 22, 24, 26, 27, 28, 29, 30, 32, 34, 36, 38, 40, 42, 44, 45, 46, 48, 50, 52, 54, 56, 58, or 60.

### 1.2.3. L₃

In the present application, L₃ is a linking group or a divalent linking group.

In a specific embodiment of the present application, L₃ is preferably selected from the group consisting of a linking bond, -(CH₂)ₜₘ-, -C(=O)(CH₂)ₜₘ-, -C(=O)NH(CH₂)ₜₘ-, -C(=O)O(CH₂)ₜₘ-, -(CH₂)ₜₘC(=O)NH(CH₂)ₜₘ-, -(CH₂)ₜₘNHC(=O)(CH₂)ₜₘ-, -(CH₂)ₜₘC(=O)O(CH₂)ₜₘ- and -C(=O)(CH₂)ₜₘC(=O)NH(CH₂)ₜₘ-; wherein, the left end of L₃ is connected to the PEG chain, and tm is independently an integer from 1 to 6 at each occurrence;
L₃ is more preferably selected from the group consisting of a linking bond, -(CH₂)₂₋₃-, -C(=O)(CH₂)₂₋₃-, -C(=O)NH(CH₂)₂₋₃-, -C(=O)O(CH₂)₁₋₄-, -(CH₂)₁₋₃C(=O)NH(CH₂)₂₋₃-, -(CH₂)₂₋₃NHC(=O)(CH₂)₁₋₃-, -(CH₂)₁₋₃C(=O)O(CH₂)₁₋₄- and -C(=O)(CH₂)₂₋₃C(=O)NH(CH₂)₂₋₃-;
L₃ is more preferably selected from the group consisting of a linking bond, -CH₂CH₂-, -CH₂CH₂CH₂-, -C(=O)CH₂-, -C(=O)CH₂CH₂-, -C(=O)NHCH₂CH₂-, -C(=O)OCH₂CH₂CH₂-, -C(=O)OCH₂CH₂-, -C(=O)OCH₂CH₂CH₂-, -CH₂C(=O)NHCH₂CH₂-, -CH₂C(=O)NHCH₂CH₂CH₂-, -CH₂CH₂C(=O)NHCH₂CH₂-, -CH₂CH₂C(=O)NHCH₂CH₂CH₂-, -CH₂CH₂CH₂C(=O)NHCH₂CH₂-, -CH₂CH₂CH₂C(=O)NHCH₂CH₂CH₂-, -CH₂CH₂NHC(=O)CH₂-, -CH₂CH₂CH₂NHC(=O)CH₂-, -CH₂CH₂NHC(=O)CH₂CH₂-, -CH₂CH₂CH₂NHC(=O)CH₂CH₂-, -CH₂C(=O)OCH₂CH₂-, -CH₂C(=O)OCH₂CH₂CH₂-, -CH₂CH₂C(=O)OCH₂CH₂-, -CH₂CH₂C(=O)OCH₂CH₂CH₂-, -CH₂CH₂CH₂C(=O)OCH₂CH₂-, -CH₂CH₂CH₂C(=O)OCH₂CH₂CH₂-, -C(=O)CH₂CH₂C(=O)NHCH₂CH₂-, -C(=O)CH₂CH₂C(=O)NHCH₂CH₂CH₂-, -C(=O)CH₂CH₂CH₂C(=O)NHCH₂CH₂- and -C(=O)CH₂CH₂CH₂C(=O)NHCH₂CH₂CH₂-.

### 1.2.4. L₁, L₂, B₁, B₂, L₅, L₆

In the present application, L₁ and L₂ are preferably selected from the group consisting of a linking bond, -C(=O)-, -C(=O)O-, -OC(=O)-, -OC(=O)O-, -C(=O)NH-, -NHC(=O)-, -NHC(=O)O-, -OC(=O)NH- and -NHC(=O)NH-.

In the present application, L₅ and L₆ are preferably selected from the group consisting of a linking bond, -C(=O)- and -C(=O)O-.

In the present application, B₁ and B₂ are each independently a linking bond or a C₁₋₁₂ alkylene group.

In a specific embodiment of the present application, L₁, L₂, B₁, B₂, L₅, and L₆ are preferably selected from any of the following cases:
Case (1): B₁, B₂, L₁, and L₂ are all linking bonds;
Case (2): B₁, B₂, L₁, and L₂ are not linking bonds;
Case (3): L₅, L₆, B₁, and B₂ are all linking bonds.

In a specific embodiment of the present application, B₁, B₂, L₁, and L₂ are all linking bonds, and the PEGylated lipid is preferably selected from the group consisting of the following structures: 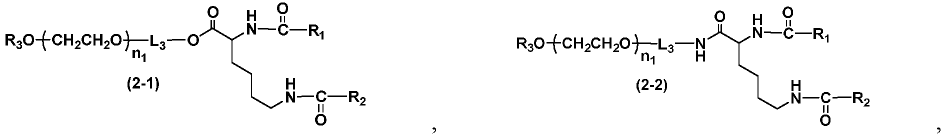 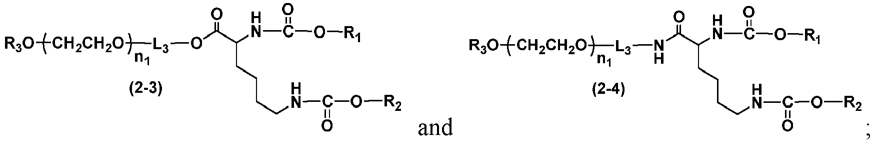
more preferably, the PEGylated lipid is represented by general formula (2-2) or formula (2-4); more preferably, the L₃ in general formula (2-2) and formula (2-4) not containing an ester bond; more preferably, the L₃ in general formula (2-2) and formula (2-4) is each independently selected from the group consisting of -(CH₂)₂₋₃-, -C(=O)(CH₂)₂₋₃-, -C(=O)NH(CH₂)₂₋₃-, -(CH₂)₁₋₃C(=O)NH(CH₂)₂₋₃-, -(CH₂)₂₋₃NHC(=O)(CH₂)₁₋₃- and -C(=O)(CH₂)₂₋₃C(=O)NH(CH₂)₂₋₃- at each occurrence; wherein, the left end of L₃ is connected with the PEG chain;
the structure of PEGylated lipid is further preferably selected from the group consisting of the following formulas: 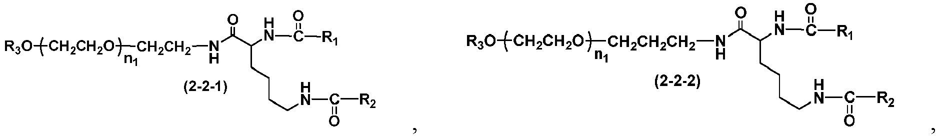 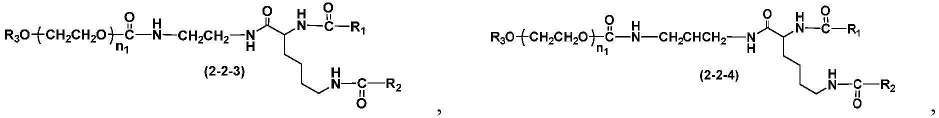 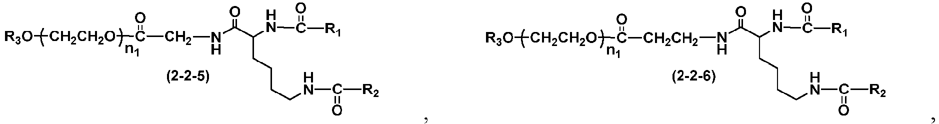 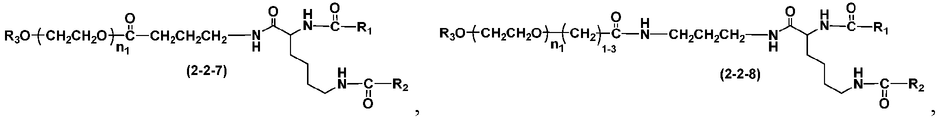 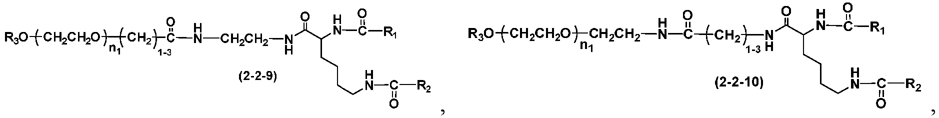 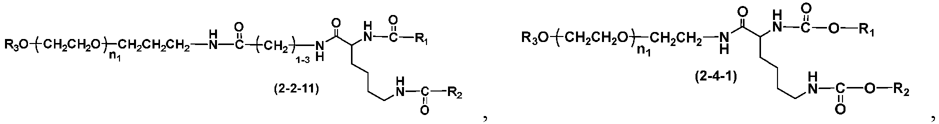 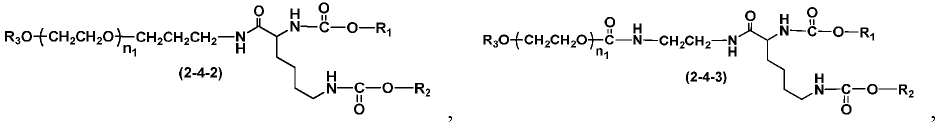 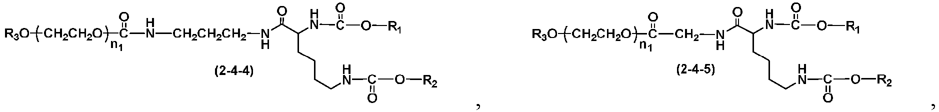 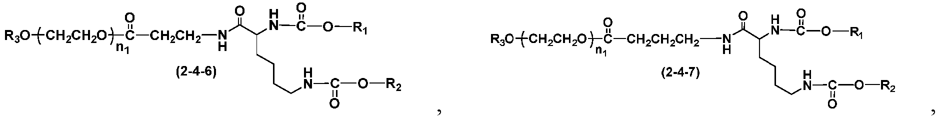 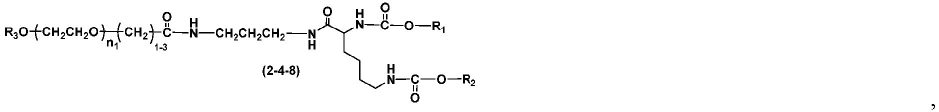 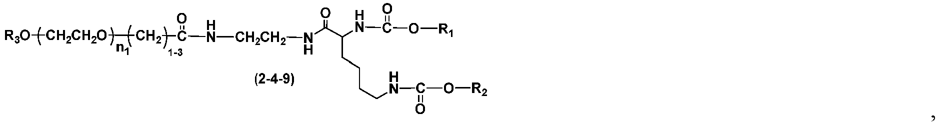 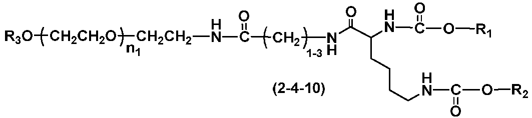 and

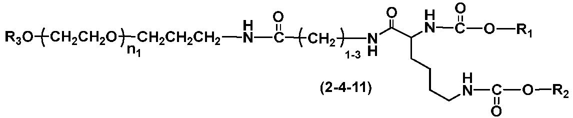

In a specific embodiment of the present application, B₁, B₂, L₁, and L₂ are preferably not linking bonds; both L₁ and L₂ are non-ester-containing divalent linking groups, and the structure of PEGylated lipid is preferably selected from the group consisting of the following formulas: 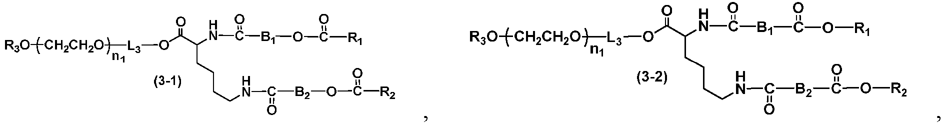 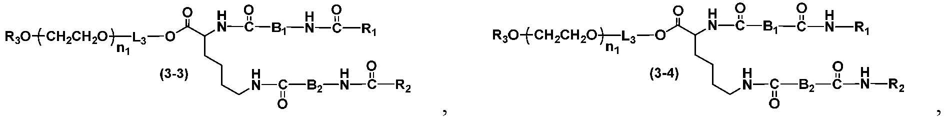 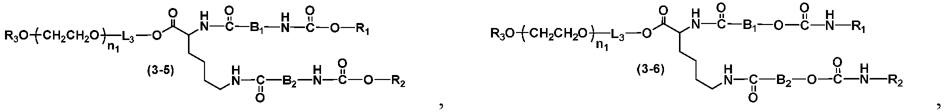 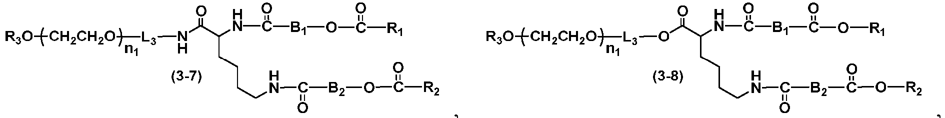 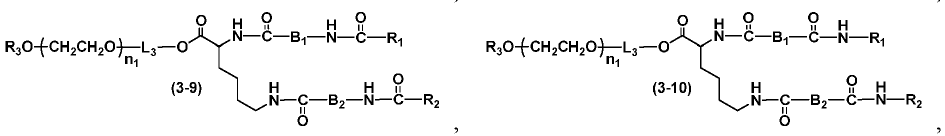 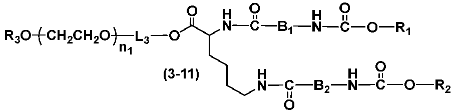 and

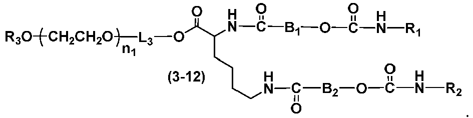

### 1.2.5. R₃

In the present application, R₃ is a hydrogen atom, a C₁₋₆ alkyl group, a carbocyclic group, a heterocyclic group or -L₄-R₀₁; wherein, L₄ is a divalent linking group and R₀₁ is a functional group that can react with bio-related substances.

In a specific embodiment of the present application, R₃ is -L₄-R₀₁; wherein, L₄ is a divalent linking group, which is selected from the group consisting of -(CH₂)ₜ-, -O-, -C(=O)-, -C(=O)O-, -OC(=O)-, -OC(=O)O-, -C(=O)NH-, -NHC(=O)-, -NHC(=O)O-, -OC(=O)NH-, -NHC(=O)NH- and any combinations thereof; t is an integer from 1 to 12; and R₀₁ is selected from any of the following cases:
Case (1): the group consisting of an epoxy group, a hydroxyl group, a protected hydroxyl group, a sulfhydryl group, a protected sulfhydryl group, a carboxyl group, a protected carboxyl group, an amino group, a protected amino group, an aldehyde group, a protected aldehyde group, an active ester group, an alkenyl group, an acrylate group, an azido group, a cyano group, a dithiopyridinyl group, an α-halogenated acetylene group, an alkyne group, 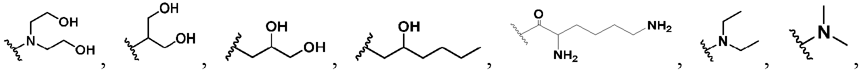 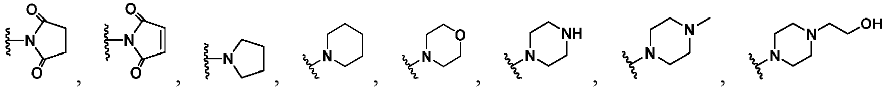 and 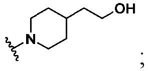
Case (2): a functional group with therapeutic targeting, and the functional group is preferably selected from the group consisting of a residue of folic acid, cholesterol, biotin, N-acetylgalactosamine and any functional derivative thereof;
   and the functional group is further preferably selected from the group consisting of the following structures:

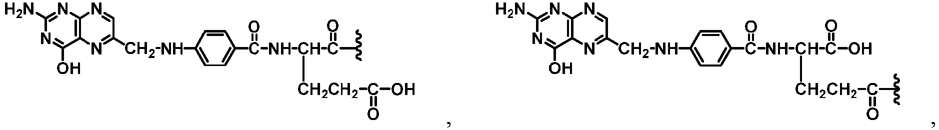

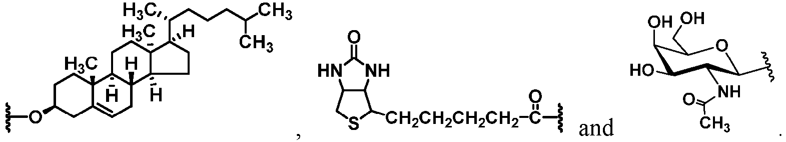

### 1.2.6. R₁, R₂

In the present application, R₁ and R₂ are each independently a C₅₋₃₀ aliphatic hydrocarbon group, more preferably a C₁₀₋₃₀ hydrocarbon aliphatic group, and more preferably a C₁₀₋₂₀ aliphatic hydrocarbon group.

In a specific embodiment of the present application, R₁ and R₂ are each independently represented by 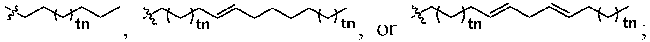 wherein, tn is independently an integer from 1 to 12 at each occurrence; R₁ and R₂ are preferably same, and selected from the group consisting of 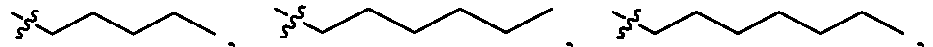 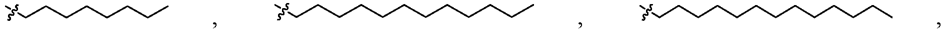 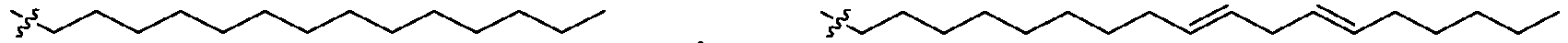 and

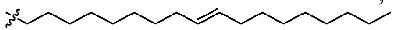

### 1.2.7. B₁, B₂

In the present application, B₁ and B₂ are each independently a linking bond or a C₁₋₁₂ alkylene group.

In a specific embodiment of the present application, B₁ and B₂ are preferably each independently selected from the group consisting of a pentylene group, a hexylene group, a heptylene group, an octylene group, a nonylene group and a decylene group.

### 1.2.8. Examples of specific structures

Some specific embodiments of the present application have finally obtained a PEGylated lipid with a structure as follows, including but not limited to any of the following structures: 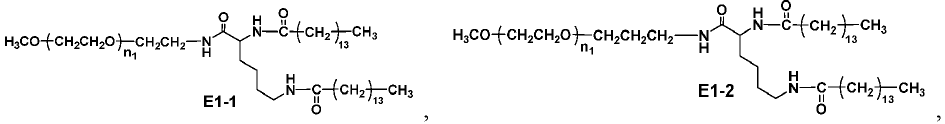 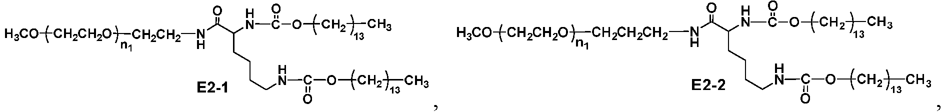 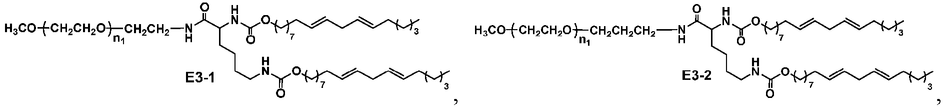 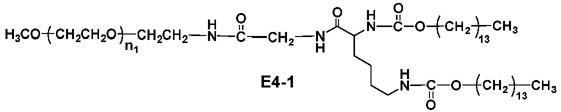 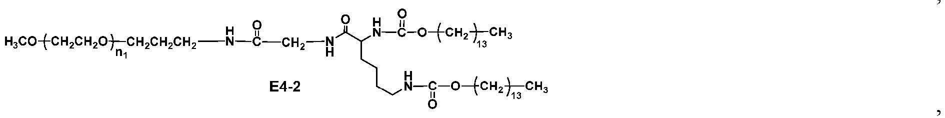 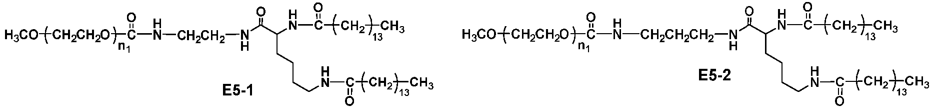 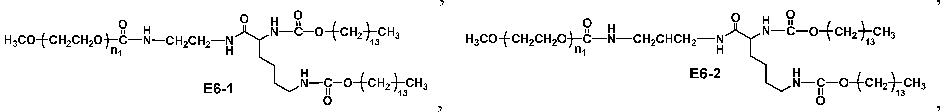 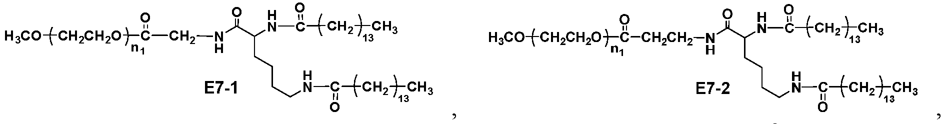 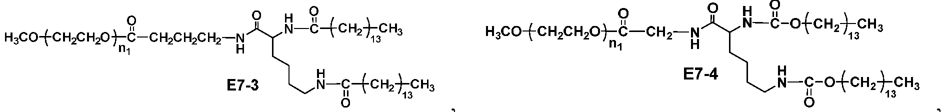 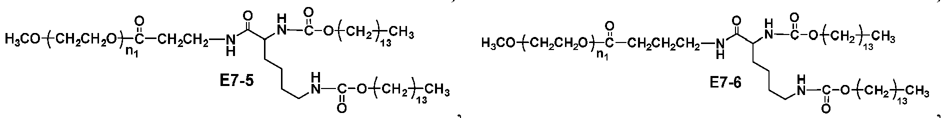 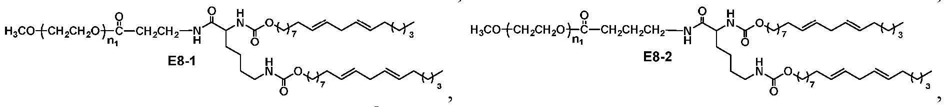 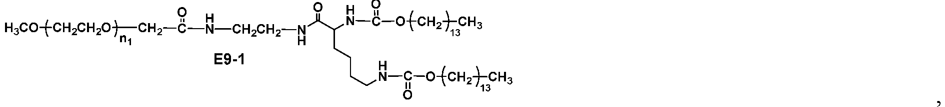 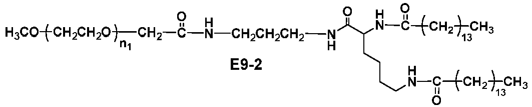 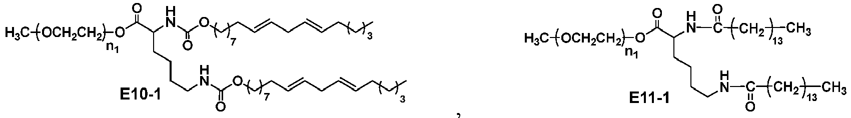 and 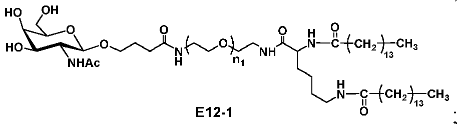 or the PEGylated lipid is selected from the group consisting of the following structures: 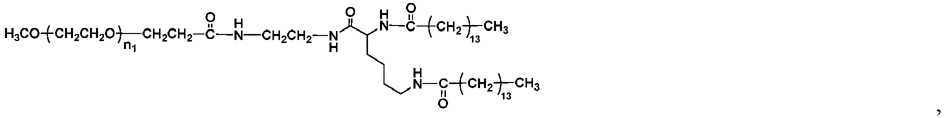 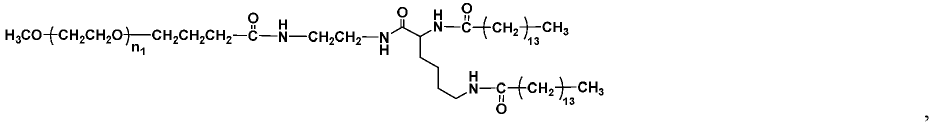 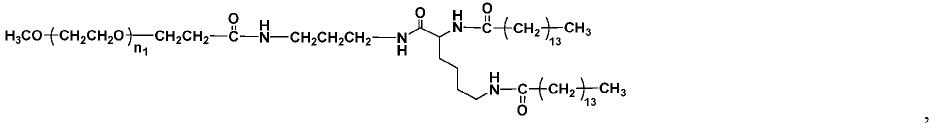 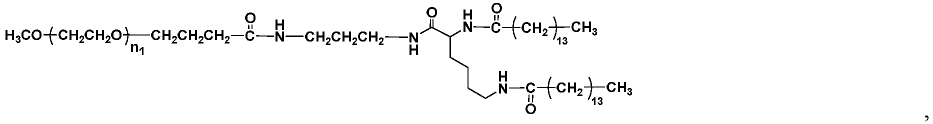 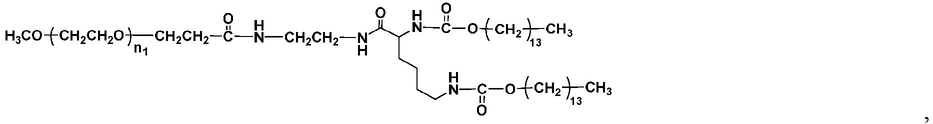 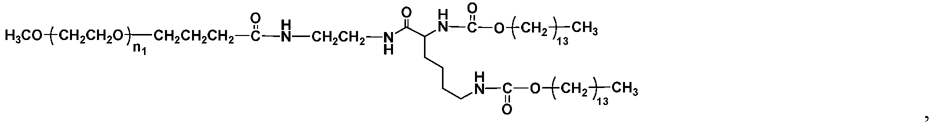 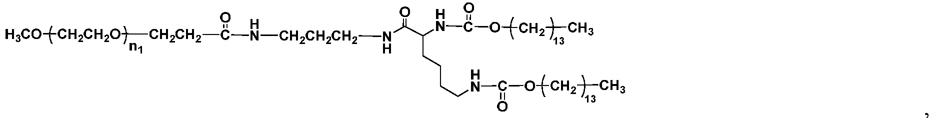 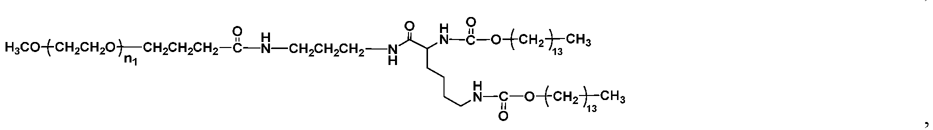 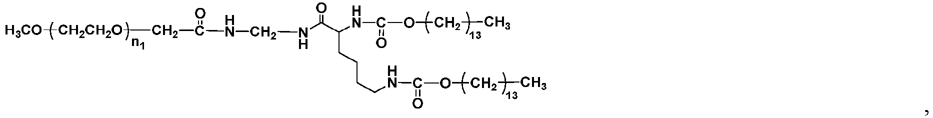 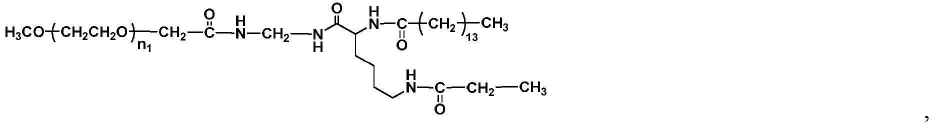 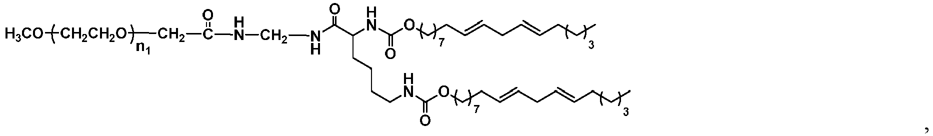 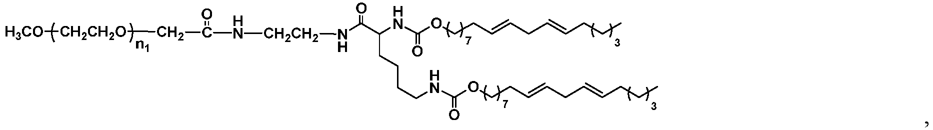 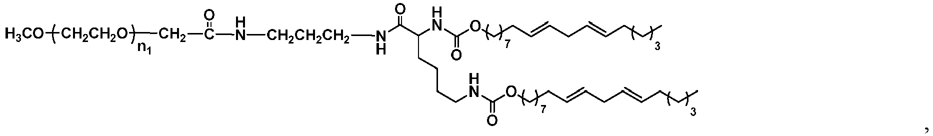 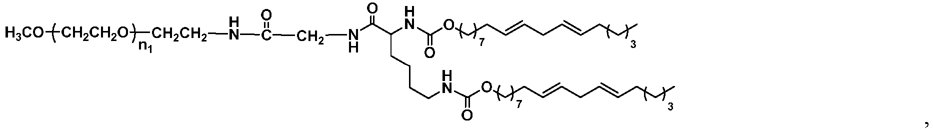 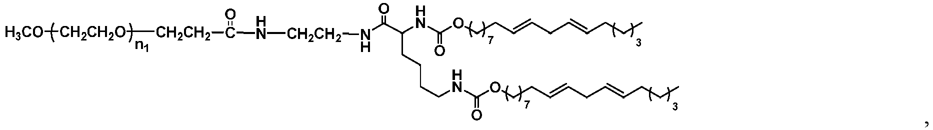 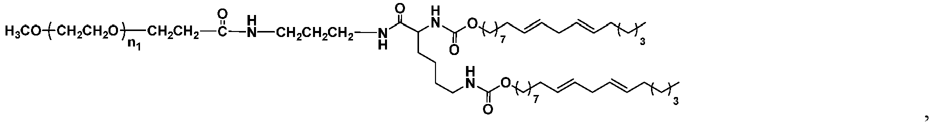 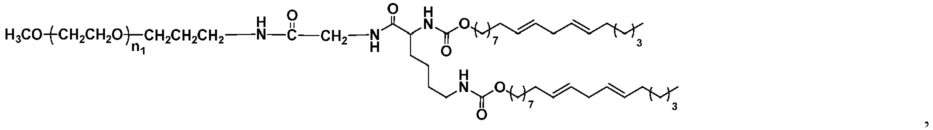 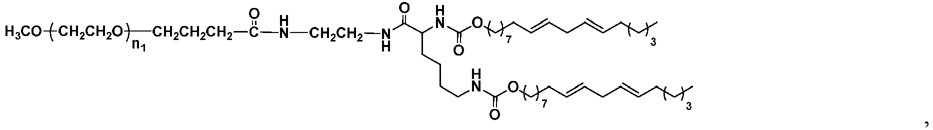 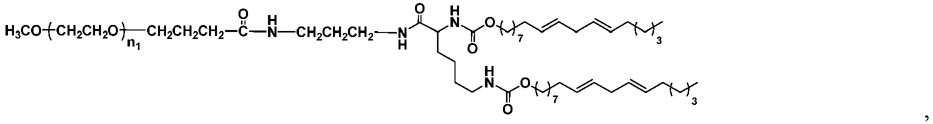 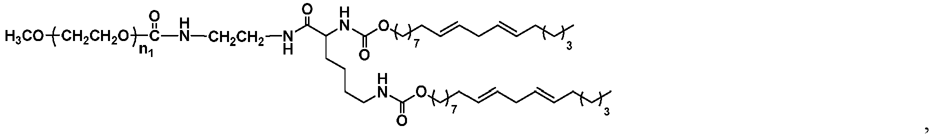 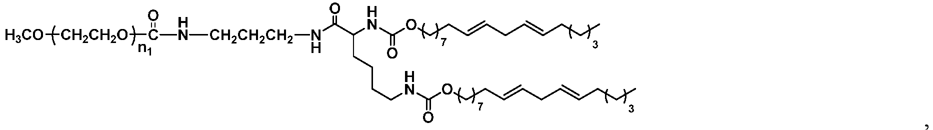 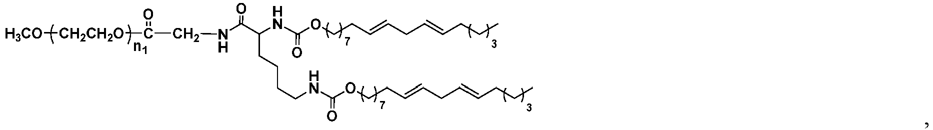 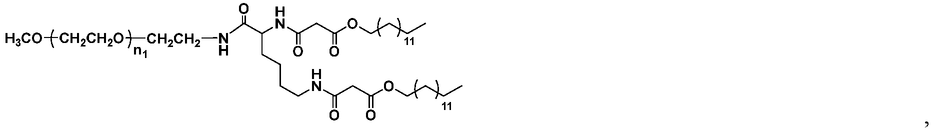 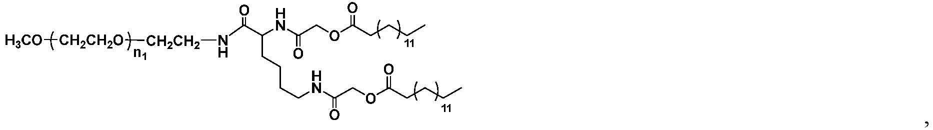 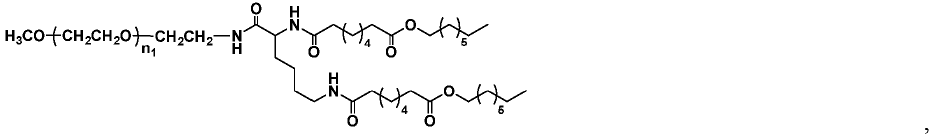 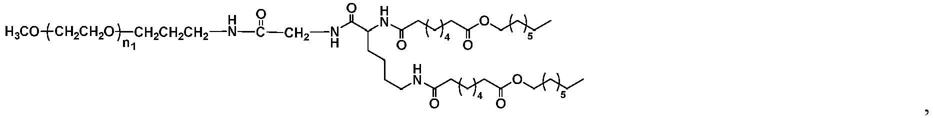 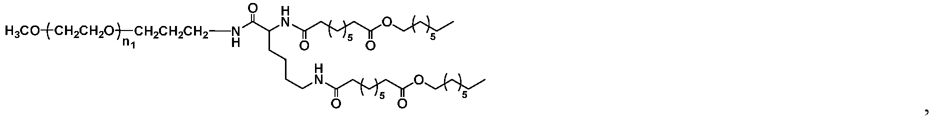 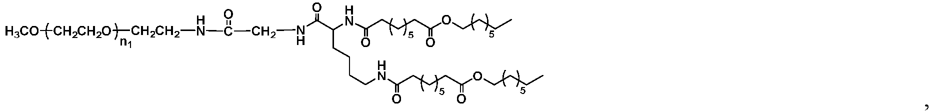 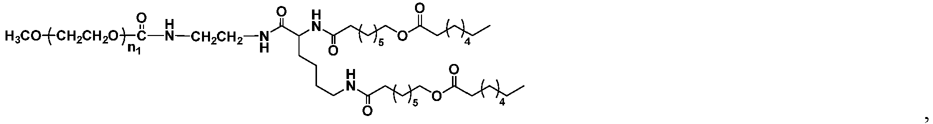 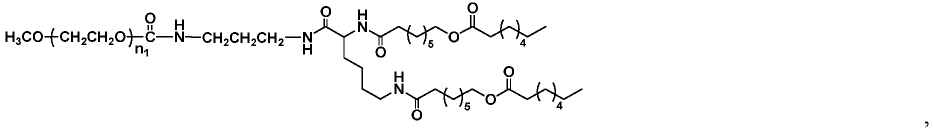 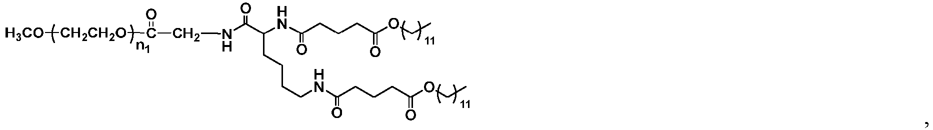 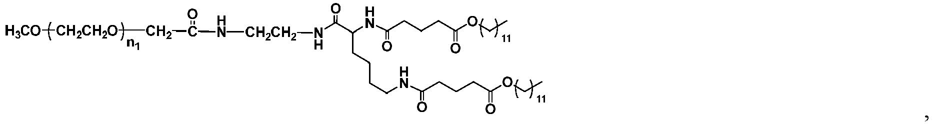 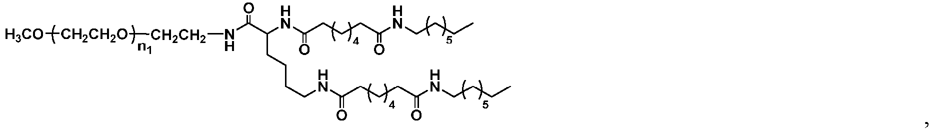 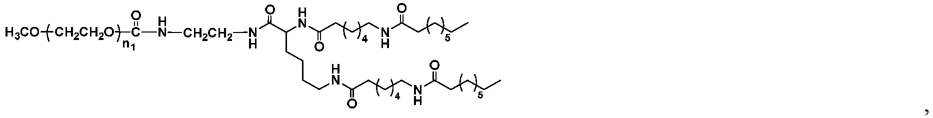 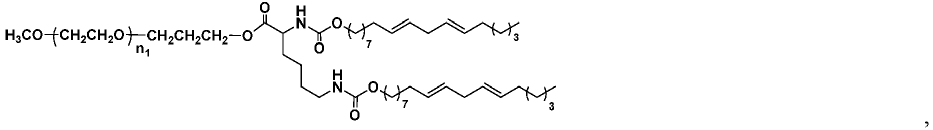 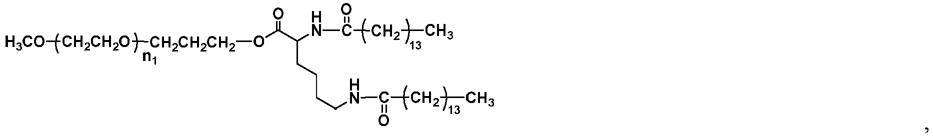 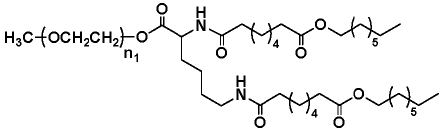 and

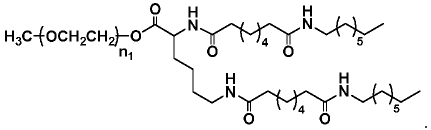

### 2. Preparation of PEGylated lipids containing lysine core

### 2.1. Small molecule intermediates IM-1, IM-2

In a specific embodiment of the present application, the preparation process of PEGylated lipids containing lysine core represented by formula (1) involves the small molecule intermediates IM-1 and IM-2, and the structures of IM-1 and IM-2 are as follows: 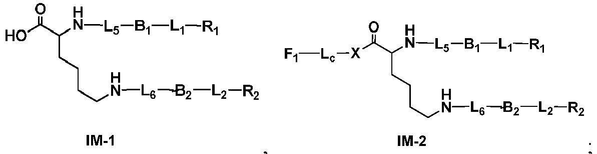 wherein, the L_{c} in IM-2 is selected from the group consisting of a linking group, a hydrocarbylene group, -OC(=O)-, -C(=O)O-, -OC(=O)O-, -OC(=O)O-, -C(=O)NH-, -NHC(=O)-, -NHC(=O)O-, -OC(=O)NH-, -NHC(=O)NH- and any combinations thereof, preferably a hydrocarbylene group; wherein, F₁ is a reactive group and the protected form thereof, capable of participating in the covalent reaction and generating a divalent linking group directly, or through deprotection or activation; F₁ is preferably selected from the group consisting of an amino group, a carboxyl group, a hydroxyl group, a halogen group, an aldehyde group, an alkenyl group, an alkyne group, a succinimidyl group, a maleimide group, a sulfhydryl group and the protected form thereof; the definitions of X, R₁, R₂, L₅, L₆, B₁, B₂, L₁ and L₂ are consistent with those described in general formula (1) and will not be repeated here.

In a specific embodiment of the present application, **IM-1** is preferably selected from the group consisting of the following general formulas: 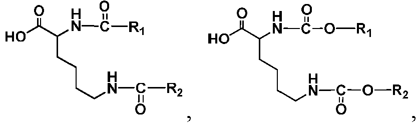 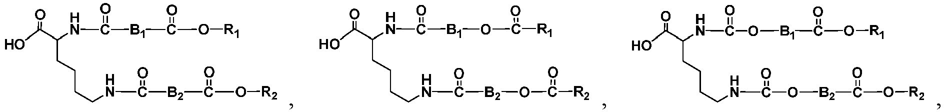 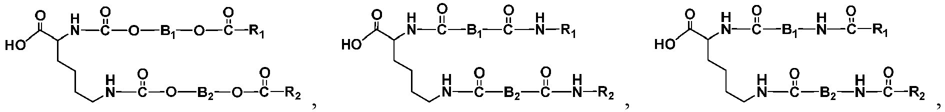 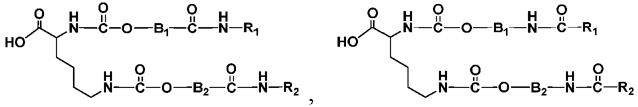 and a structure with the terminal carboxyl group being protected thereof; wherein, B₁ and B₂ are each independently a C₁₋₁₂ alkylene group at each occurrence; R₁ and R₂ are each independently a C₅₋₃₀ aliphatic hydrocarbon group at each occurrence; more specifically, IM-1 is preferably selected from the group consisting of the following structures: 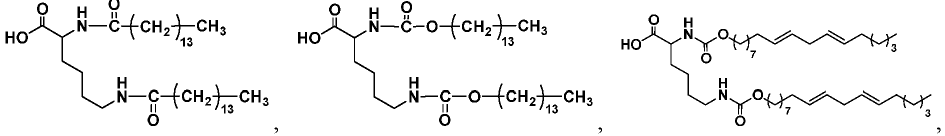 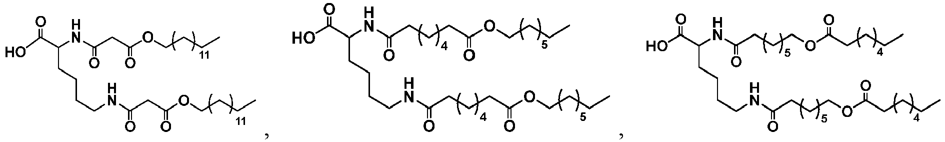 and the structure with the carboxyl group of terminal reactive groupbeing protected form thereof.

In a specific embodiment of the present application, **IM-2** is preferably selected from the group consisting of the following formulas: 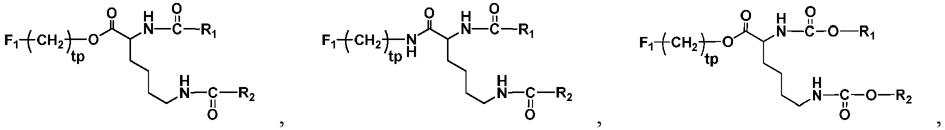 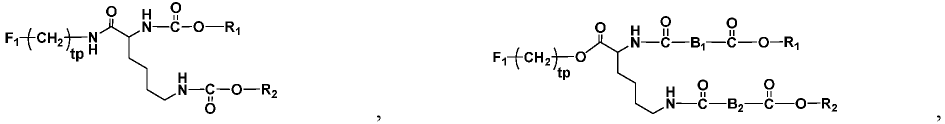 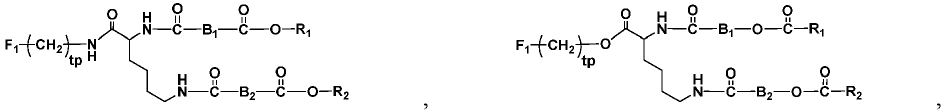 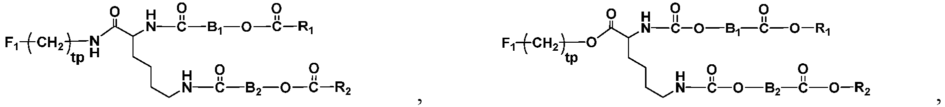 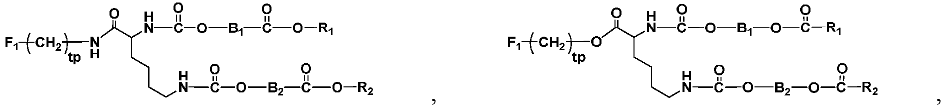 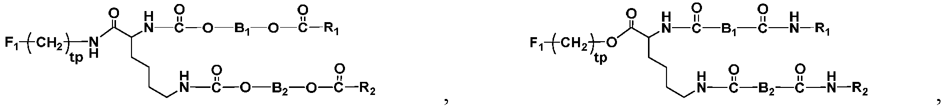 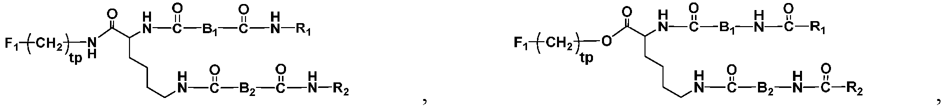 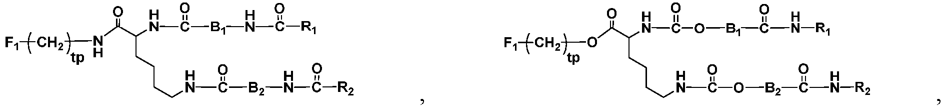 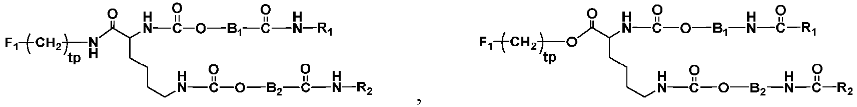 and 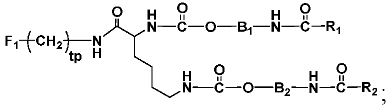 wherein, F₁ is selected from the group consisting of an amino group, a carboxyl group, a hydroxyl group, a halogen group, an aldehyde group, an alkene group, an alkyne group, a succinimidyl group, a maleimide group, a sulfhydryl group and the protected form thereof, and tp is an integer from 1 to 6, preferably an integer from 1 to 3; B₁ and B₂ are each independently a C₁₋₁₂ alkylene group at each occurrence; R₁ and R₂ are each independently a C₅₋₃₀ aliphatic hydrocarbon group at each occurrence, and more specifically, IM-2 is preferably selected from the group consisting of the following structures: 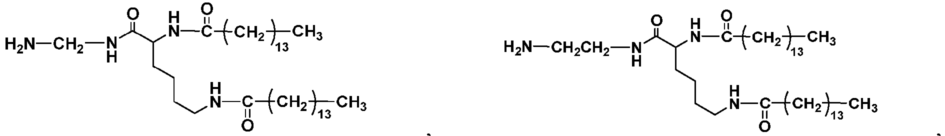 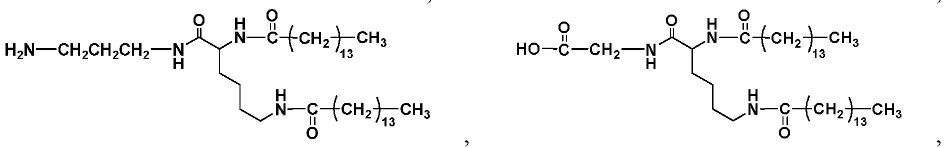 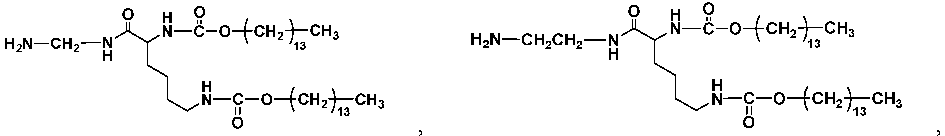 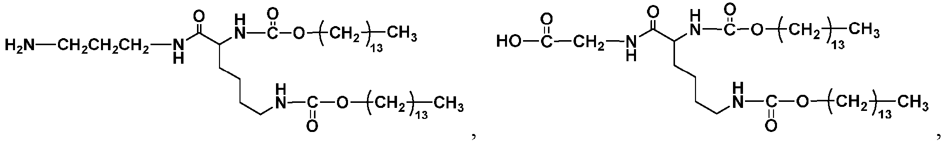 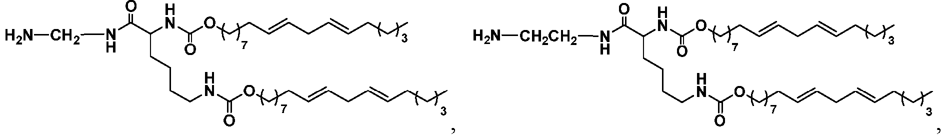 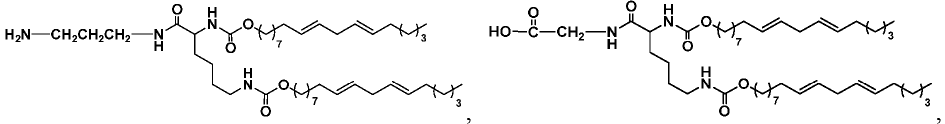 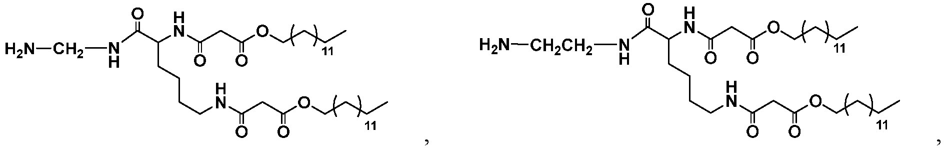 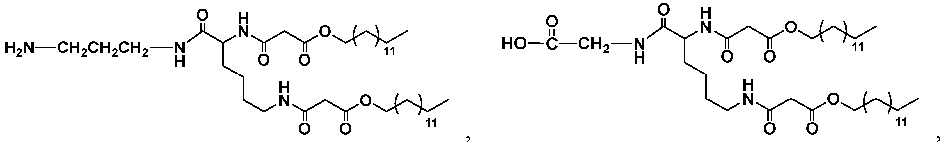 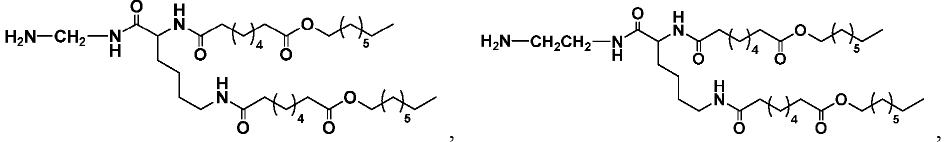 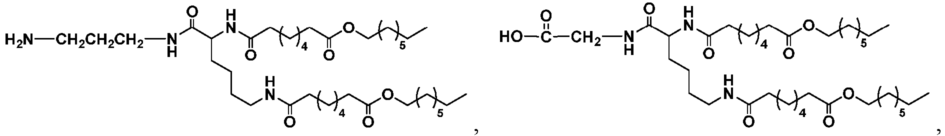 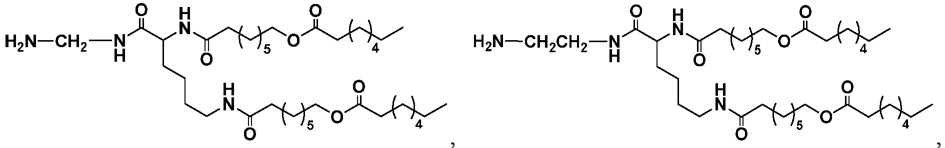 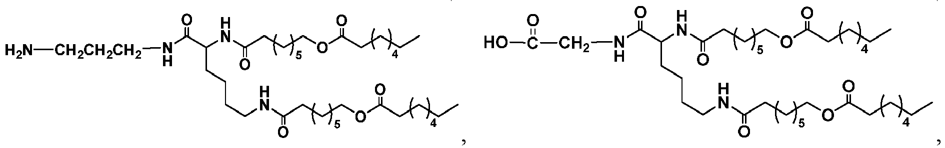 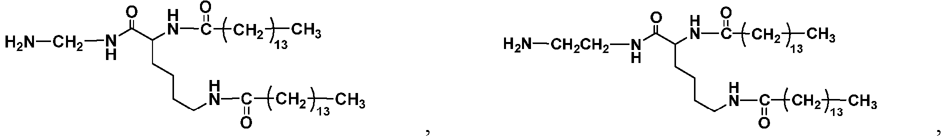 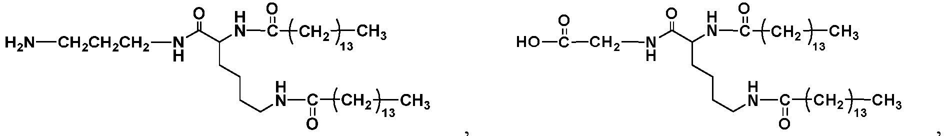 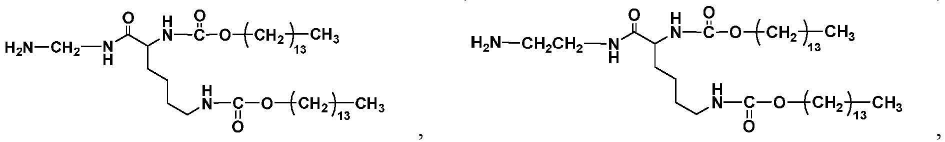 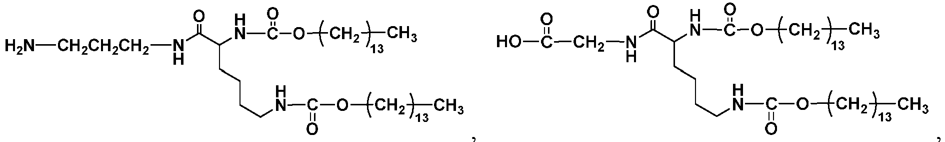 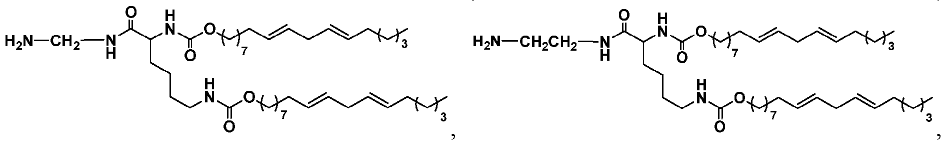 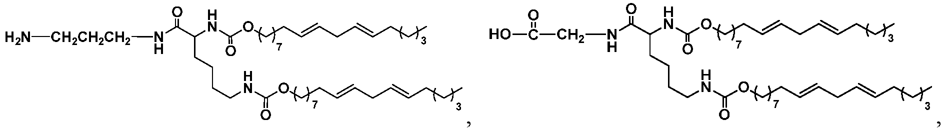 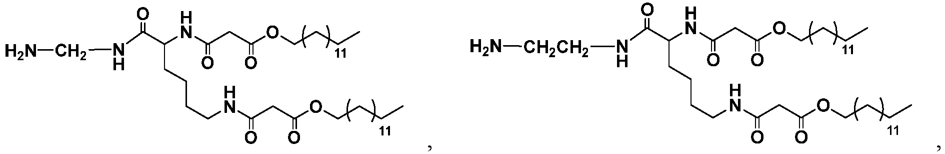 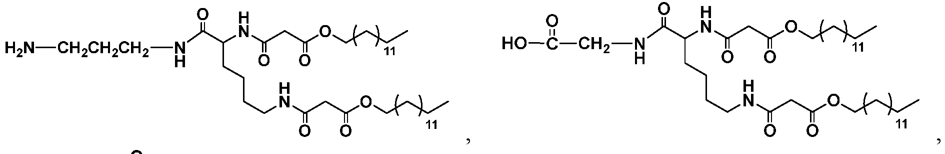 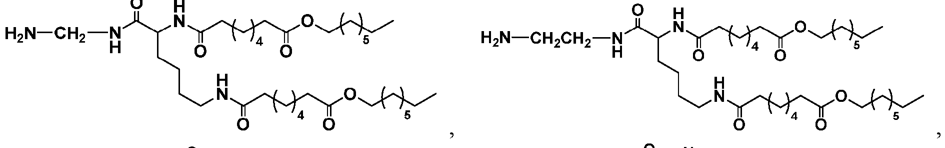 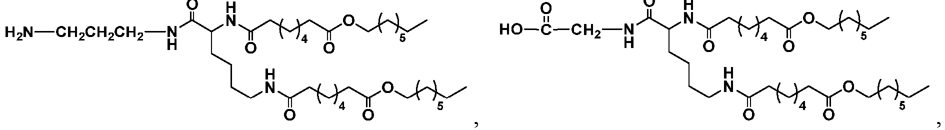 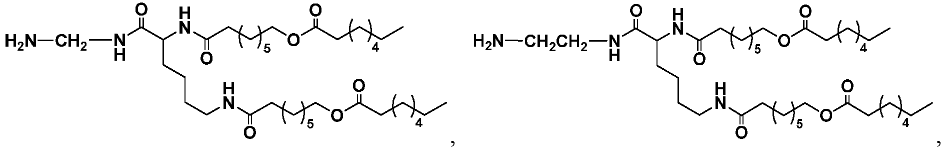 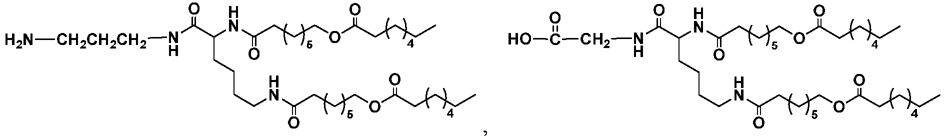 and the structure with an end reactive group being protected form thereof.

### 2.2. Activated Polyethylene Glycol Derivatives of PEG_{source1} and PEG_{source2}

In a specific embodiment of the present application, the preparation process of PEGylated lipids with a structure represented by formula (1) involves a polyethylene glycol derivative PEG_{source} containing a reactive group, and PEG_{source} can react with IM-1 or IM-2, including **PEG_{source1}** and **PEG_{source2};** the PEG_{source} is preferably selected from the group consisting of the following structures: 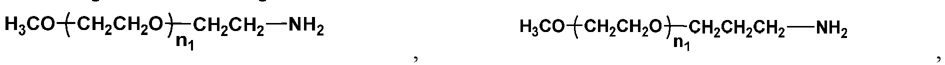 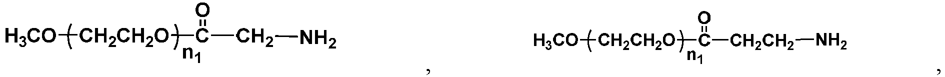 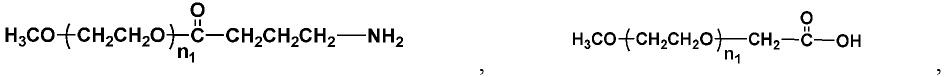 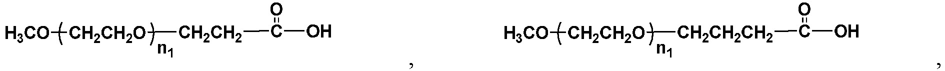 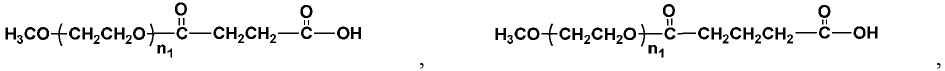 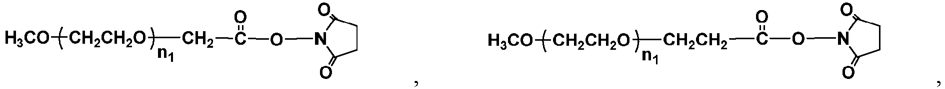 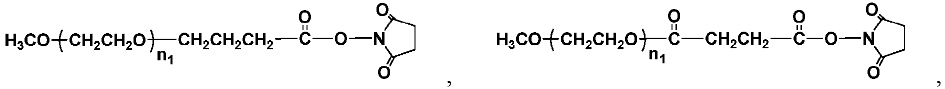 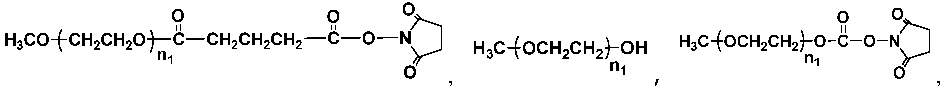 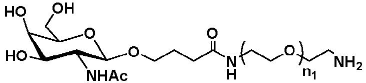 and the structure with an end reactive group being protected thereof.

### 2.3. Specific preparation methods

In the present application, the preparation of any of the aforementioned PEGylated lipids containing lysine core may adopt methods including but not limited to the following:

### 2.3.1. Method-1:

Step 1: preparation of small molecule intermediate **IM-1:** react one molecule of lysine **A-1** with small molecule **A-2** to obtain a lysine core lipid small molecule intermediate **IM-1;** wherein, F_{N} is a reactive group, which can react with an amino group to obtain a divalent linking group; the reactive group is protected or unprotected, including but not limited to an acyl chloride, a carboxyl-activated ester, a carboxyl group, a hydroxyl group, a protected carboxyl group, or a protected hydroxyl group, preferably an acyl chloride or a carboxyl-activated ester; when F_{N} is in a protected form, it should be deprotected before reacting with **A-1;**
Step 2: react the carboxyl group of the small molecule intermediate **IM-1** with the reactive group of **PEG_{source1},** a polyethylene glycol derivative containing a reactive group, to obtain a PEGylated lipid with a structure represented by the general formula **(1');** wherein, F_{c} is a reactive group that can react with a carboxyl group, preferably a hydroxyl group or an amino group;
   when R₃' is equal to R₃, the resulting structure represented by general formula **(1')** corresponds to the structure represented by the general formula **(1);**
   when R₃' is not equal to R₃, the resulting structure represented by general formula **(1')** undergoes terminal micromodification to obtain the structure represented by the general formula **(1);** the terminal micromodification is selected from the following chemical reactions: deprotection, salt complexation and decomplexation, ionization, protonation, deprotonation, or leaving group transformation;
   wherein, the definitions of X, R₁, R₂, R₃, L₅, L₆, B₁, B₂, L₁ and L₂ are the same as those described in general formula (1) and will not be repeated here, and L₅ is the same as L₆, B₁ is the same as B₂, and L₁ is the same as L₂.

### Step 1:

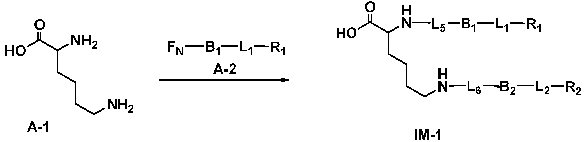

### Step 2:

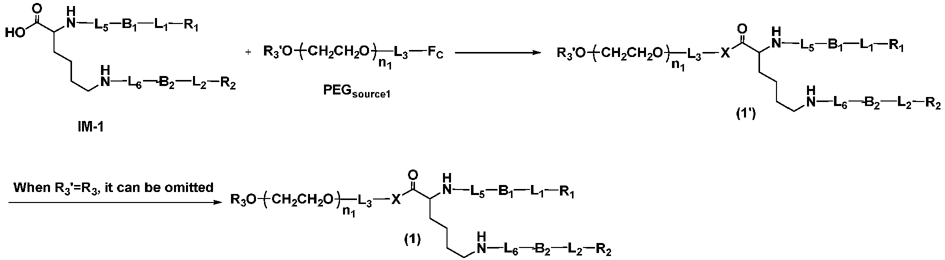

For example, in **Example** 1.1, **S1-1** corresponds to **A-1** in the above route, and **S1-2** corresponds to **A-2** in the above route, and **S1-3** corresponds to **IM-1** by substitution reaction, and **IM-1** is coupled with the active polyethylene glycol derivative PEG_{source1} (methoxy polyethylene glycol propylamine) to obtain PEGylated lipid **E1-1:**

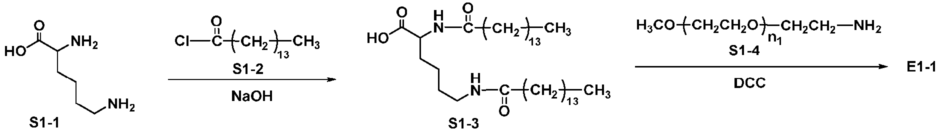

### 2.3.2. Method-2:

Step 1: preparation of small molecule intermediate **IM-1:** same as step 1 in method 1 of section 2.3.1. and will not be repeated here;
Step 2: preparation of small molecule intermediate **IM-2:** the carboxyl group of small molecule intermediate **IM-1** reacts with the reactive group F_{c} of small molecule **B-3** to obtain a small molecule intermediate **IM-2** containing a divalent linking bond L_{c}; wherein, **B-3** contains two different reactive groups F_{c} and F₁; the reactive group is protected or unprotected; F_{c} is preferably a hydroxyl group or an amino group; F₁ is any suitable reactive group, preferably a hydroxyl group, an amino group, a carboxyl group, a reactive ester or an acyl halide group; in one specific embodiment, F₁ can be a protected hydroxyl group or a protected amino group;
Step 3: the F₁ of the small molecule intermediate **IM-2** reacts with the reactive group of the active polyethylene glycol derivative **PEG_{source2}** to obtain a PEGylated lipid with a structure represented by the general formula **(1'),** and the divalent linking group formed by the reaction of F₁ and F₂ together with L_{c} and L_{d} constitutes L₃; wherein, F₂ is preferably a carboxyl group, an active ester group, an acyl halide group, a hydroxyl group or an amino group, and L_{d} is a linking group or a divalent linking group, and the divalent linking group is preferably an hydrocarbonylene group; when F₁ is a protected reactive group, it should be deprotected before reacting with **PEG_{source2};**
   when R₃' is equal to R₃, the resulting structure represented by general formula **(1')** corresponds to the structure represented by the general formula **(1);**
   when R₃' is not equal to R₃, the resulting structure represented by general formula **(1')** undergoes terminal micromodification to obtain the structure represented by general formula **(1);** the terminal micromodification is selected from the following chemical reactions: deprotection, salt complexation and decomplexation, ionization, protonation, deprotonation, or leaving group transformation;
   wherein, the definitions of X, R₁, R₂, R₃, L₅, L₆, B₁, B₂, L₁ and L₂ are the same as those described in general formula (1) and will not be repeated here, and L₅ is the same as L₆, B₁ is the same as B₂, and L₁ is the same as L₂.

### Step 1:

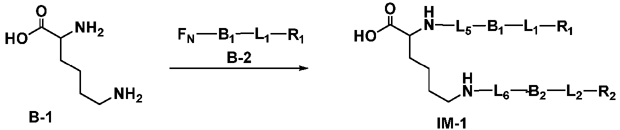

### Step 2:

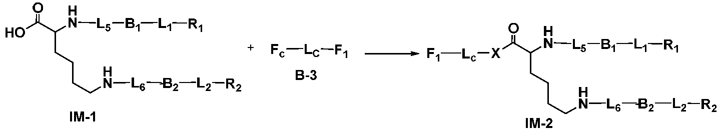

### Step 3:

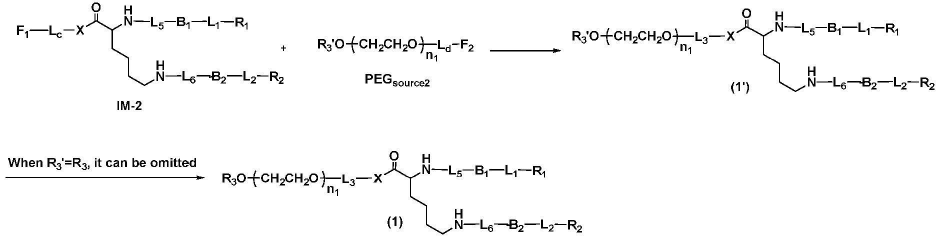

For example, in Example 5.1, S1-3 corresponds to **IM-1** in the above route, and **S5-1** corresponds to **B-3** in the above route; **S5-2** (corresponding to **IM-2**) is obtained by substitution reaction, and the PEGylated lipid **E5-1** is obtained by coupling reaction between **IM-2** and the active polyethylene glycol derivative PEG_{source2}:

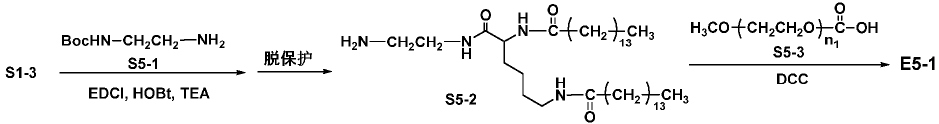

### 3. Lipid composition

In the present application, provided herein is a lipid composition containing any PEGylated lipid with a structure represented by the general formula (1).

In one specific embodiment of the present application, the lipid composition preferably contains, in addition to a PEGylated lipid with a structure represented by the general formula (1), one or more types of lipids selected from the group consisting of a phospholipid, a steroid lipid, and a cationic lipid, selected from any one of the following cases:
Case (1): contains also a phospholipid;
Case (2): contains also a steroid lipid;
Case (3): contains also a cationic lipid;
Case (4): contains also a phospholipid and steroid lipid;
Case (5): contains also a phospholipid and cationic lipid;
Case (6): contains also a steroid lipid and cationic lipid;
Case (7): contains also a phospholipid, a steroid lipid and a cationic lipid;
more preferably, it also contains a phospholipid, a steroid lipid and a cationic lipid.

In a more specific embodiment of the present application, the phospholipid in the lipid composition is preferably selected from the group consisting of 1,2-dilinoleoyl-sn-glycero-3-phosphocholine (DLPC), 1,2-dimyristoleoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphatidylcholine (DSPC), 1,2-diundecanoyl-sn-glycero-3-phosphatidylcholine (DUPC), 1-plamitoyl-2-oleoyl-sn-glycero-3-phosphocholine (POPC), 1,2-di-*O*-octadecenyl-sn-glycero-3-phosphatidylcholine (18:0 Diether PC), 1-oleoyl-2-cholesterylhemisuccinyl-sn-glycero-3-phosphocholine (OChemsPC), 1-*O*-hexadecyl-sn-glycero-3-phosphatidylcholine (C16 Lyso PC), 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholines, 1,2-dioleoyl-sn-glycero-3-phosphoethanolamine (DOPE), 1,2-diphytanyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt (DOPG), dioleoyl phosphatidylserine (DOPS), dipalmitoyl phosphatidylglycerol (DPPG), palmitoyloleoyl phosphatidylethanolamine (POPE), distearoyl phosphatidylethanolamine (DSPE), dipalmitoyl phosphatidylethanolamine (DPPE), dimyristoleoyl phosphoethanolamine (DMPE), 1-stearoyl-2-oleoyl-stearoylethanolamine (SOPE), 1-stearoyl-2-oleoyl-phosphatidylcholine (SOPC), sphingomyelin, phosphatidylcholine, phosphatidylethnolamine, phosphatidylserine, phosphatidylinositol, phosphatidic acid, palmitoyloleoyl phosphatidylcholine, lysophosphatidylcholine lysophosphatidylethanolamine (LPE), and combinations thereof; the phospholipid can be synthetic or natural.

In a specific embodiment of the present application, the steroid lipid in the lipid composition is preferably selected from the group consisting of cholesterol, coprostanol, sitosterol, ergosterol, campesterol, stigmasterol, brassicasterol tomatidine, ursolic acid, α-tocopherol, and combinations thereof.

In a more specific embodiment of the present application, the cationic lipid in the lipid composition is preferably selected from the group consisting of *N*,*N*-dioleyl-*N*,*N*-dimethylammonium chloride (DODAC), *N*,*N*-distearyl-*N*,*N*-dimethylammonium bromide (DDAB), *N*-(1-(2,3-dioleoyloxy)propyl)-*N*,*N*,*N*-trimethylammonium chloride (DOTAP), *N*-(1-(2,3-dioleyloxy)propyl)-*N*,*N*,*N*-trimethylammonium chloride (DOTMA), *N*,*N*-dimethyl-2, 3-dioilyloxypropylamine (DODMA), 3-(didodecylamino)-*N*1,*N*1,4-tridodecyl-1-piperazineethanamine (KL10), *N*-[2(didodecylamino)ethyl]-*N*1,*N*4,*N*4-tridodecyl-1,4-piperazinediethanamine (KL22), 14,25-ditridecyl-15,18,21,24-tetraaza-octatriacontane (KL25), 1,2-dileutheroxy-N, *N*-dimethylaminopropane (DLin-DMA), 2,2-dilinoleyl-4-dimethylaminomethyl-[1,3]-dioxolane (DLin-K-DMA), heptatriaconta-6,9,28,31-tetraen-19-yl-4-(dimethylamino)butanoate (DLin-MC3-DMA) and 2,2-dilinoleyl-4-(2dimethylaminoethyl)-[1,3]-dioxolane (DLin-KC2-DMA), ((4-hydroxybutyl)azanediyl)bis(hexane-6,1-diyl)bis(2-hexyldecanoate) (ALC-0315), heptadecan-9-yl-8-((2-hydroxyethyl)(6-oxo-6-(undecyloxy)hexyl)amino)octanoate) (SM102), and any combinations thereof.

In a more specific embodiment of the present application, the cationic lipid in the lipid composition is preferably selected from the group consisting of the following structures and the combinations thereof: 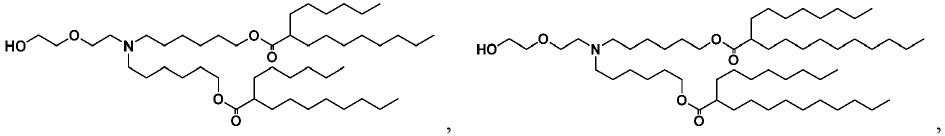 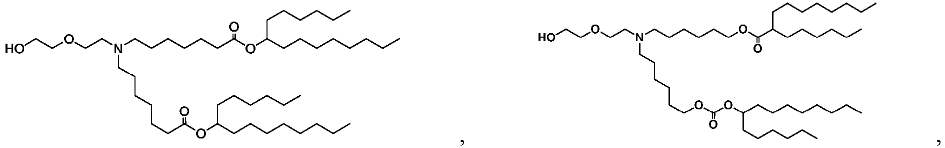 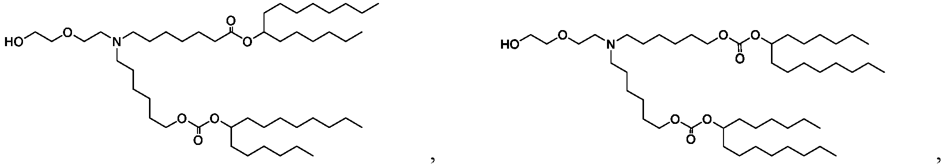 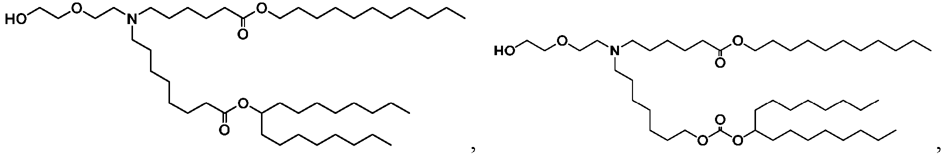 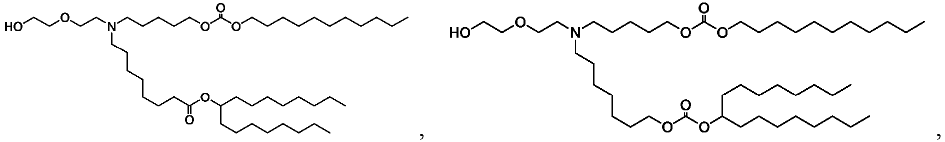 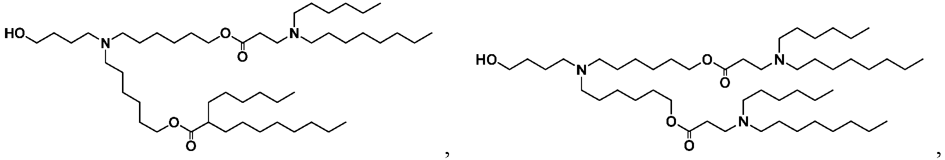 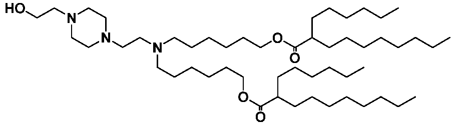 and

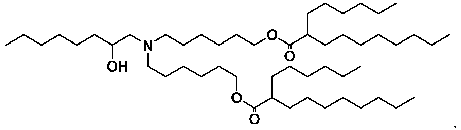

In a specific embodiment of the present application, the molar percentage of the PEGylated lipid, cationic lipid, phospholipid, or steroid lipid in the total lipids in the solution containing solvent is not limited; the percentage is the molar percentage of each lipid in the total lipids in the solution containing the solvent.

In a more specific embodiment of the present application, the lipid composition preferably contains 20-80% cationic lipids, 5-15% phospholipids, 25-55% steroid lipids and 0.5-10% PEGylated lipids.

In a more specific embodiment of the present application, the molar percentage of the PEGylated lipid in the total lipids in the solution containing solvent is preferably 1-5%; more preferably 1-3%; and more preferably about 1.5%, 1.6%, 1.7%, 1.8%, or 1.9%;
the molar percentage of the cationic lipid in the total lipids in the solution containing solvent is preferably 30-65%; and more preferably about 35%, 40%, 45%, 46%, 47%, 48%, 49%, 50%, or 55%;
the molar percentage of the phospholipid in the total lipids in the solution containing solvent is preferably 7.5-13%, and more preferably about 8%, 9%, 10%, 11%, 12%;
the molar percentage of the steroid lipid in the total lipids in the solution containing solvent is preferably 35-50%, more preferably about 40%, 41%, 42%, 43%, 44%, 45%, 46%, 47%, 48%, 49%, or 50%.

### 3.2. Preparation of lipid compositions

In the present application, the lipid composition can be prepared by the following methods, including but not limited to ethanol injection, microfluidic method, T-tube mixing method, periplasmic extrusion method, preferably ethanol injection and microfluidic method.

### 4. Lipid pharmaceutical compositions and formulations thereof

### 4.1. Lipid pharmaceutical compositions

In one embodiment of the present application, a lipid pharmaceutical composition contains any lipid composition and drug described in section 1.3 above; wherein, the lipid composition contains any of the aforementioned PEGylated lipid with a structure represented by formula (1), and the drug is selected from the group consisting of a nucleic acid drug, a gene vaccine, an antitumor drug, a small molecule drug, a peptide drug and a protein drug.

In a specific embodiment of the present application, the nucleic acid drug of the lipid pharmaceutical composition is selected from the group consisting of RNA, DNA, antisense nucleic acid, plasmid, interfering nucleic acid, aptamer, antagomir and ribozyme, the RNA is selected from any of mRNA, saRNA, circRNA, miRNA and siRNA; the nucleic acid drug is preferably selected from the group consisting of DNA, mRNA, miRNA and siRNA.

In a specific embodiment of the present application, the lipid pharmaceutical composition is preferably used as a drug and selected from the group consisting of an antineoplastic agent, an antiviral agent, an antifungal agent and a vaccine.

In a specific embodiment of the present application, the N/P ratio of the lipid composition to the nucleic acid is preferably (0.1~100): 1, more preferably (0.2~30): 1, most preferably (0.5~20): 1.

### 4.2. A lipid pharmaceutical composition preparation

In a specific embodiment of the present application, the drug in the lipid pharmaceutical composition is the nucleic acid drug, and the working solution of the formulation is deionized water, ultrapure water, phosphate buffer or normal saline, more preferably phosphate buffer or normal saline, and most preferably normal saline; the ratio of lipid composition: working solution is preferably (0.05~20) g: 100 mL, more preferably (0.1~10) g: 100 mL, most preferably (0.2~5) g: 100 mL.

In a specific embodiment of the application, a formulation of lipid pharmaceutical composition contains the aforementioned lipid pharmaceutical composition and pharmaceutically acceptable diluent or excipient; the diluent or excipient is preferably deionized water, ultrapure water, phosphate buffer or normal saline, more preferably phosphate buffer or normal saline, and most preferably normal saline.

In the present application, the preparation of the formulation of lipid pharmaceutical composition includes the following steps:
(1) equilibrate the lipid composition in the diluent or excipient;
(2) nucleic acid drugs are added to the mixture of the balanced lipid composition and the diluent or excipient for compounding;
wherein, the equilibrium time is preferably 0.1~12 h, preferably 0.2~6 h, and more preferably 0.5~3 h; the composite time is preferably 0.1~12 h, preferably 0.2~5 h, and more preferably 0.5~2 h.

### 5. Liposomes or lipid nanoparticles and preparation thereof

### 5.1. Liposomes or lipid nanoparticles

A specific embodiment of the present application, a liposome or lipid nanoparticle contains any lipid pharmaceutical composition described above.

A specific embodiment of the present application, the aforementioned lipid nanoparticle is preferably an LNP pharmaceutical composition, an LPP pharmaceutical composition or a PNP pharmaceutical composition, preferably an LNP pharmaceutical composition, more preferably an LNP-nucleic acid pharmaceutical composition, and more preferably an LNP-mRNA pharmaceutical composition.

### 5.2. Preparation of liposomes or lipid nanoparticles

In a specific embodiment of the present application, the liposomes can be prepared by the following methods, including but not limited to thin-film dispersion method, ultrasonic dispersion method, reverse-phase evaporation method, freeze-drying method, freeze-thaw method, double emulsion method and injection method, preferably by thin-film dispersion method, ultrasonic dispersion method and/or reverse-phase evaporation method.

In a specific embodiment of the present application, the lipid nanoparticle can be prepared by the following methods, including but not limited to microemulsion method, double emulsion method, high shear homogenization ultrasonic method, thin film hydration extrusion method, and microfluidic method.

In a specific embodiment of the present application, liposomes are prepared by thin-film dispersion methods that could include the following steps:
Step (1): weigh cationic lipid, steroid lipid, neutral lipid, and PEGylated lipid, fully dissolve them in organic solvents, shake them well, remove the organic solvents by vacuum rotary evaporation to obtain an oil film, and further dry with a vacuum pump to remove the organic solvents;
Step (2): add phosphate buffer with dissolved cryoprotectant, and use water-bath ultrasonication to obtain a translucent emulsion;
Step (3): add the emulsion to a high-pressure homogenizer for overpressure, and then add the over pressurized homogenized emulsion to the liposome extruder for film-passing to obtain cationic liposomes;
Step (4): optionally, dry the said liposomes in a freeze dryer to obtain a liposome powder;
   wherein, the organic solvent is preferably dichloromethane, chloroform and/or methanol; more preferably chloroform and methanol; wherein, the rotational speed of vacuum rotary evaporation is preferably 30 to 300 rpm, more preferably 50 to 200 rpm, and most preferably 100 to 170 rpm; wherein, the temperature of vacuum rotary evaporation is preferably 10 to 200 °C, more preferably 20 to 200 °C, and most preferably 40 to 80 °C;
   the time of the drying with a vacuum pump is preferably 1 to 72 h, more preferably 5 to 48 h, and most preferably 15 to 36 h;
   the mass concentration of the cryoprotectant dissolved in phosphate buffer is preferably 0.1 to 80%, preferably 1 to 50%, and more preferably 5 to 20%;
   the frequency of the water-bath ultrasonication is preferably 10 to 300 kHz, more preferably 30 to 200 kHz, and most preferably 60 to 150 kHz;
   the time of the water-bath ultrasonication is preferably 0.1 to 5 h, more preferably 0.2 to 2 h, and most preferably 0.25 to 1 h;
   the pressure of the high-pressure homogenizer is preferably 50 to 240 MPa, more preferably 80 to 200 MPa, and most preferably 100 to 150 MPa;
   the times of the overpressure of the high-pressure homogenizer is preferably any integer from 1 to 50, more preferably any integer from 3 to 20, and most preferably any integer from 5 to 10;
   the pressure of the liposome extruder is preferably 50 to 300 MPa, more preferably 80 to 250 MPa, and most preferably 120 to 200 MPa;
   the times of the film-passing of the liposome extruder is preferably any integer from 1 to 50, more preferably any integer from 3 to 30, and most preferably any integer from 5 to 20;
   the time of the drying in a freeze dryer is preferably 1 to 120 h, more preferably 5 to 72 h, and most preferably 10 to 36 h.

In the preparation methods for cationic liposomes in the present application, the ratio of cationic liposome to phosphate buffer with dissolved cryoprotectant could be 1 mg : (0.1~100) mL, preferably 1 mg : (0.3~50) mL, and more preferably 1 mg : (0.5~5) mL.

In a specific embodiment of the present application, the lipid nanoparticle is preferably prepared by the microfluidic method, the steps are as follows:
Step (1): dissolve each lipid component in the organic solvent to obtain lipid compositions dissolved in the organic phase; the organic phase is preferably ethanol;
Step (2): add core acid drugs to buffer to obtain an aqueous solution; the aqueous phase is preferably a citrate buffer salt or sodium acetate buffer;
Step (3): the organic phase solution and aqueous phase solution are mixed by microfluidic equipment to form lipid nanoparticle compositions, and purified by ultrafiltration and the like to remove organic solvents and free nucleic acid molecules.

The following specific examples are further descriptions of the preparation methods of PEGylated lipid, lipid compositions, and formulation of lipid pharmaceutical compositions, and the biological activity assays for lipid pharmaceutical compositions; the specific examples are disclosed to further illustrate the application, but should not be regarded as a limitation of the scope of the present application. Wherein, in the embodiments of preparing PEGylated lipid, the structures of end products are characterized by NMR, and the molecular weight is confirmed by GPC or MALDI-TOF.

### Example-1.1: PEGylated lipid (E1-1)

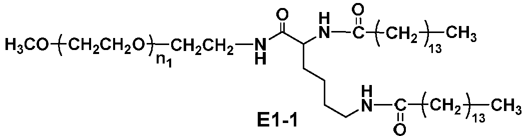

The preparation process is as follows:
Step a: The lysine (**S1-1**, 0.58 g, 4.0 mmol) in a mixed solution of acetone and water (2:1, v/v). The pH was adjusted to 8.5-9.5 by dropwise addition of 10% (wt%) NaOH aqueous solution. Under ice bath conditions, tetradecyl chloride (**S1-2,** 1.35 g, 5.2 mmol) was added dropwise with stirring, and during this process, 10% NaOH aqueous solution was added dropwise to keep the pH 7.5-11.5. The mixture was then stirred in an ice bath for 2.5 h, and after the reaction was finished, the solution was allowed to rest at room temperature overnight. The solvent was removed by rotary evaporation, and a mixed solution of concentrated hydrochloric acid and water (1:1, v/v) was added to the residue to adjust the pH 1-2. The precipitated solid was filtered, and the filtrate was washed with deionized water until the pH was adjusted to 7, then washed with petroleum ether three times, and finally recrystallized with methanol to obtain the small molecule lipid intermediate **S1-3** (1.75 g).
Step b: The monoaminomethoxy-terminated PEG2000 (**S1-4**, 2.00 g, 1.0 mmol, mPEG-CH₂CH₂NH₂, M_{w}≈2000, n₁≈45, PDI=1.02) was dissolved in a mixed solution of anhydrous DMF and anhydrous DCM (1:8, v/v), 4-(dimethylamino)pyridine (**DMAP,** 0.32 g, 2.6 mmol), **S1-3** (1.19 g, 2.0 mmol) and 1-ethyl-(3-dimethylaminopropyl)carboyldiimide (**EDC**, 0.38 g, 2.0 mmol) were slowly added to the solution under ice bath, then the solution was stirred overnight at room temperature. After completion of the reaction, the filtrate was concentrated, dissolved with a small amount of DCM, then slowly added dropwise to the ether solution, filtered, collected the precipitate and dissolved with a small amount of DCM again, and then slowly added dropwise to the ether solution, filtered and collected the precipitate, and finally dried to obtain the PEGylated lipid **E1-1** (1.95 g). ¹H NMR (400 MHz, CDCl₃) δ: 4.39-4.32 (m, 1H, >C*H*-), 3.83-3.48 (m, PEG-H), 3.45-3.41 (m, 2H, PEG-CH₂C*H*₂NH-), 3.34 (s, 3H, -OC*H*₃), 3.29-3.14 (m, 2H; -CH₂CH₂CH₂C*H*₂NH-), 2.22-2.12 (m, 4H, -C(=O)C*H*₂CH₂-), 1.63-1.21 (m, 54H, >CHC*H*₂C*H*₂C*H*₂CH₂-, 6H; -C(=O)CH₂(C*H*₂)₁₂CH₃, 48H), 0.88 (t, 6H, -CH₂C*H*₃). The molecular weight of **E1-1** determined by GPC was approximately 2.6 kDa and PDI=1.02.

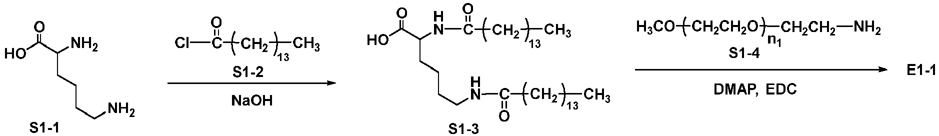

### Example-1.2: PEGylated lipid (E1-2)

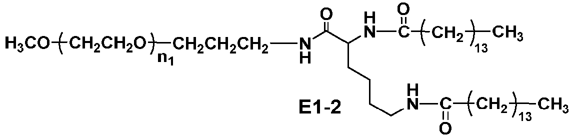

The preparation process is as follows:
Step a: mPEG-CH₂CH₂CH₂NH₂ (**S1-5**, 2.10 g, 1.0 mmol, mPEG-CH₂CH₂NH₂, Mw≈2100, n₁≈45, PDI=1.03) was dissolved in a mixed solution of anhydrous DMF and anhydrous DCM (1:8, v/v). **DMAP** (0.32 g, 2.6 mmol), **S1-3** (1.19 g, 2.0 mmol) and **EDC** (0.38 g, 2.0 mmol) were slowly added to the solution successively under ice bath, then the solution was stirred overnight at room temperature. After completion of the reaction, the filtrate was concentrated, dissolved with a small amount of DCM, and then slowly added dropwise to the ether solution, filtered, collected the precipitate, and the collected precipitate was dissolved again with a small amount of DCM, then slowly added dropwise to the ether solution, filtered, collected the precipitate, and finally dried to obtain PEGylated lipid **E1-2** (1.91 g). ¹H NMR (400 MHz, CDCl₃) δ: 4.45-4.42 (m, 1H, >C*H*-), 3.88-3.54 (m, PEG-*H*), 3.45-3.41 (m, 2H, PEG-CH₂C*H*₂NH-), 3.35 (s, 3H, -OC*H*₃), 3.28-3.12 (m, 2H, -CH₂CH₂CH₂C*H*₂NH-), 2.26-2.20 (m, 4H, -C(=O)C*H*₂CH₂-), 1.78-1.24 (m, 56H, >CHC*H*₂C*H*₂C*H*₂CH₂-, 2H; -C(=O)CH₂(C*H*₂)₁₂CH₃, 48H; PEG-CH₂C*H*₂CH₂NH-, 2H), 0.87 (t, 6H, -CH₂C*H*₃). The molecular weight of **E1-2** determined by GPC was approximately 2.6 kDa and PDI=1.03.

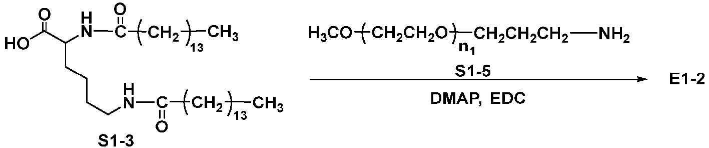

### Example-2.1: PEGylated lipid (E2-1)

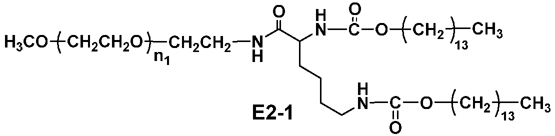

The preparation process is as follows:
Step a: **S1-1** (0.58 g, 4.0 mmol) was dissolved in anhydrous DMF (50 mL), followed by the addition of *N*,*N-*diisopropylethylamine (**DIPEA,** 25.44 mL, 145.2 mmol) and compound **S2-1** (2.44 g, 7.2 mmol), then the reaction was stirred overnight at room temperature. After completion of the reaction, the filtrate was concentrated, the crude product was dissolved with ethyl acetate (50 mL), then washed with 0.5 M citric acid (50 mL*3), NaHCO₃ (50 mL*3), and saline. The organic layer was dried with MgSO₄, concentrated, and further purified by column chromatography to obtain the small molecule lipid intermediate **S2-2** (1.68 g).
Step b: **S1-4** (2.00 g, 1.0 mmol) was dissolved in a mixture of anhydrous DMF and anhydrous DCM (1:8, v/v). **DMAP** (0.32 g, 2.6 mmol), **S2-2** (1.25 g, 2.0 mmol) and **EDC** (0.38 g, 2.0 mmol) was slowly added to the solution under an ice bath condition and stirred overnight at room temperature. After completion of the reaction, the filtrate was concentrated, dissolved with a small amount of DCM, then slowly added dropwise to the ether solution, filtered, collected the precipitate and dissolved with a small amount of DCM again, then slowly added dropwise to the ether solution, filtered, collected the precipitate, and finally dried to obtain PEGylated lipid **E2-1** (1.87 g). ¹H NMR (400 MHz, CDCl₃) δ: 4.14-4.08 (m, 1H, >C*H*-), 4.07-3.97 (t, 4H, -C(=O)OC*H*₂-), 3.83-3.53 (m, PEG-*H*), 3.46-3.43 (m, 2H, PEG-CH₂C*H*₂NH-), 3.37 (s, 3H, -OC*H*₃), 3.20-3.08 (m, 2H, -CH₂CH₂CH₂C*H*₂NH-), 1.84-1.20 (m, 54H, >CHC*H*₂C*H*₂C*H*₂CH₂-; 6H; -C(=O)OCH₂(C*H*₂)₁₂CH₃, 48H), 0.86 (t, 6H, -CH₂C*H*₃). The molecular weight of **E2-1** determined by GPC was approximately 2.7 kDa and PDI=1.02.

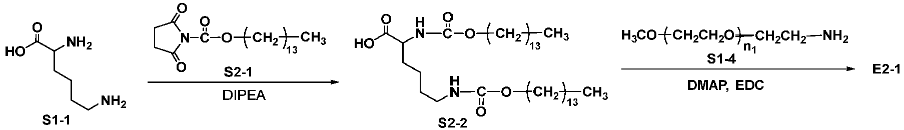

### Example-2.2: PEGylated lipid (E2-2)

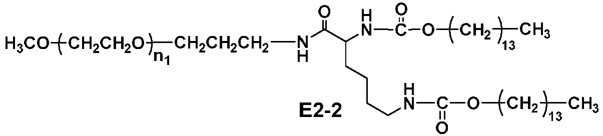

The preparation process is as follows:
**S1-5** (2.10 g, 1.0 mmol) was dissolved in a mixed solution of anhydrous DMF and anhydrous DCM (1:8, v/v). **DMAP** (0.32 g, 2.6 mmol), **S2-2** (1.25 g, 2.0 mmol) and **EDC** (0.38 g, 2.0 mmol) were slowly added under ice bath conditions, then the solution was stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E2-2** (1.92 g). ¹H NMR (400 MHz, CDCl₃) δ: 4.47-4.43 (m, 1H, >C*H*-), 4.06-4.03 (m, 4H, -C(=O)OC*H*₂-), 3.88-3.52 (m, PEG-*H*), 3.45-3.41 (m, 2H, PEG-CH₂CH₂C*H*₂NH-), 3.36 (s, 3H, -OC*H*₃), 3.29-3.11 (m, 2H, -CH₂CH₂CH₂C*H*₂NH-), 1.78-1.24 (m, 56H, >CHC*H*₂C*H*₂C*H*₂CH₂-, 6H; -C(=O)OCH₂(C*H*₂)₁₂CH₃, 48H; PEG-CH₂C*H*₂CH₂NH-, 2H), 0.89 (t, 6H, -CH₂C*H*₃). The molecular weight of **E2-2** determined by GPC was approximately 2.7 kDa and PDI=1.03.

### Example-3.1: PEGylated lipid (E3-1)

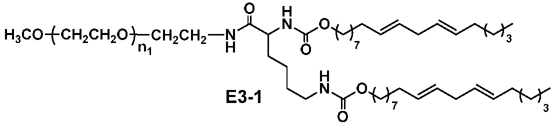

The preparation process is as follows:
Step a: **S1-1** (0.58 g, 4.0 mmol) was dissolved in anhydrous DMF (50 mL), then **DIPEA** (25.44 mL, 145.2 mmol) and compound **S3-1** (2.92 g, 7.2 mmol) was added to the solution sequentially and stirred overnight at room temperature. After completion of the reaction, the filtrate was concentrated, the crude product was dissolved with ethyl acetate (50 mL) and then washed with 0.5 M citric acid (50 mL*3), NaHCO₃ (50 mL*3), and saline. The organic layer was dried with MgSO₄, concentrated, and further purified by column chromatography to obtain the small molecule lipid intermediate **S3-2** (2.03 g).
Step b: **S1-4** (2.00 g, 1.0 mmol) was dissolved in a mixed solution of anhydrous DMF and anhydrous DCM (1:8, v/v). **DMAP** (0.32 g, 2.6 mmol), **S3-2** (1.52 g, 2.0 mmol) and **EDC** (0.38 g, 2.0 mmol) were slowly added to the solution successively under an ice bath condition and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E3-1** (2.13 g). ¹H NMR (400 MHz, CDCl₃) δ: 5.38-5.26 (m, 8H, -CH₂C*H*=C*H*CH₂C*H*=C*H*CH₂-), 4.47-4.43 (m, 1H, >C*H*-), 4.06-4.03 (m, 4H-, -C(=O)OC*H*₂-), 3.88-3.55 (m, PEG-*H*), 3.45-3.41 (m, 2H, PEG-CH₂C*H*₂NH-), 3.37 (s, 3H, -OC*H*₃), 3.29-3.13 (m, 2H, -CH₂CH₂CH₂C*H*₂NH-), 2.78-2.75 (m, 4H, -CH=CHC*H*₂CH=CH-), 2.04-1.99 (m, 8H, -C*H*₂CH=CHCH₂CH=CHC*H*₂-), 1.78-1.24 (m, 42H, -C(=O)OCH₂(CH₂)₆C*H*₂CH=CHCH₂CH=CHCH₂(C*H*₂)₃CH₃, 36H; >CHC*H*₂C*H*₂C*H*₂CH₂-, 6H), 0.88 (t, 6H, -CH₂C*H*₃). The molecular weight of **E3-1** determined by GPC was approximately 2.8 kDa and PDI=1.02.

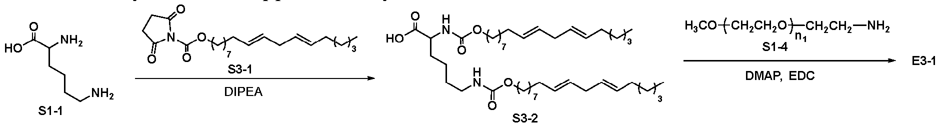

### Example-3.2: PEGylated lipid (E3-2)

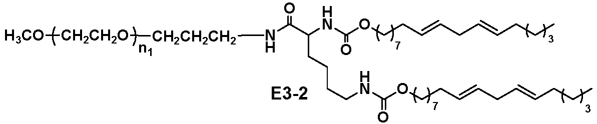

The preparation process is as follows:
**S1-5** (2.10 g, 1.0 mmol) was dissolved in a mixed solution of anhydrous DMF and anhydrous DCM (1:8, v/v). **DMAP** (0.32 g, 2.6 mmol), **S3-2** (1.52 g, 2.0 mmol) and **EDC** (0.38 g, 2.0 mmol) were slowly added to the solution successively under ice bath and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E3-2** (2.07 g). ¹H NMR (400 MHz, CDCl₃) δ: 5.38-5.26 (m, 8H, -CH₂C*H*=C*H*CH₂C*H*=C*H*CH₂-), 4.48-4.45 (m, 1H, >C*H*-), 4.06-4.03 (m, 4H, -C(=O)OC*H*₂-), 3.88-3.54 (m, PEG-*H*), 3.45-3.41 (m, 2H, PEG-CH₂CH₂C*H*₂NH-), 3.35 (s, 3H, -OC*H*₃), 3.29-3.12 (m, 2H, -CH₂CH₂CH₂C*H*₂NH-), 2.78-2.75 (m, 4H, -CH=CHC*H*₂CH=CH-), 2.04-1.98 (m, 8H, -C*H*₂CH=CHCH₂CH=CHC*H*₂-), 1.78-1.24 (m, 44H, -C(=O)OCH₂(C*H*₂)₆CH₂CH=CHCH₂CH=CHCH₂(C*H*₂)₃CH₃, 36H; PEG-CH₂C*H*₂CH₂NH-, 2H; >CHC*H*₂C*H*₂C*H*₂CH₂-, 6H), 0.88 (t, 6H, -CH₂C*H*₃). The molecular weight of **E3-2** determined by GPC was approximately 2.8 kDa and PDI=1.03.

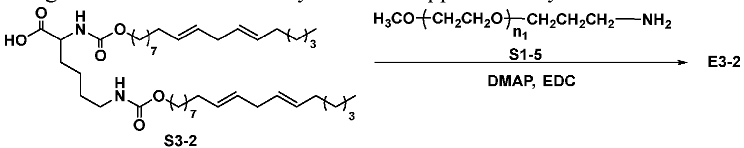

### Example-4.1: PEGylated lipid (E4-1)

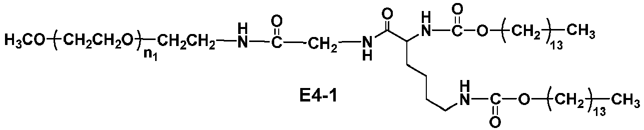

The preparation process is as follows:
Step a: tert-butyl glycinate (**S4-1**, 0.59 g, 4.5 mmol) was dissolved in dichloromethane, 1-ethyl-(3-dimethylaminopropyl) carbodiimide hydrochloride (**EDCI**, 1.15 g, 6.0 mmol) and 1-hydroxybenzotriazole (**HOBt**, 0.61 g, 4.5 mmol) were successively added to the solution at 0°C and stirred for 5 to 10 min, then the mixed solution was placed in room temperature and reacted for 1 to 3 h to obtain reaction solution A. The compound **S2-2** (1.88 g, 3.0 mmol) was dissolved in dichloromethane, and triethylamine (0.61 g, 6.0 mmol) was added to the solution at room temperature, then the reaction was stirred for 1 to 3 h to obtain reaction solution B. The reaction solution B was slowly added to reaction solution A dropwise and stirred at room temperature for 6 to 20 h. After completion of the reaction, the reaction solution was washed twice with an appropriate amount of water, twice with 10% citric acid aqueous solution, once with saturated salt water, dried with anhydrous sodium sulfate, filtered and concentrated, and the crude product was purified by column chromatography to obtain compound **S4-2** (1.86 g).
Step b: Removed the tBu protecting group. In a dry and clean round-bottom flask, a solution of trifluoroacetic acid/dichloromethane (1:2, v/v) was prepared, and a dichloromethane solution of **S4-2** (1.48 g, 2.0 mmol) was slowly added dropwise to the solution under ice bath conditions and reacted at room temperature for 2 h. After completion of the reaction, the reaction solution was concentrated, and purified water was added to the solution, shaken, extracted with dichloromethane, then the organic phase was dried with anhydrous magnesium sulfate, filtered, and the filtrate was concentrated. The small molecule lipid intermediate **S4-3** (1.26 g, 92.1%) was obtained through recrystallization.
Step c: **S1-4** (1.20 g, 0.6 mmol) was dissolved in a mixed solution of anhydrous DMF and anhydrous DCM (1:8, v/v). **DMAP** (0.19 g, 1.6 mmol), **S4-3** (0.82 g, 1.2 mmol) and **EDC** (0.23 g, 1.2 mmol) were slowly added to the solution successively under ice bath conditions, and then the solution was stirred overnight at room temperature. After completion of the reaction, the filtrate was concentrated, dissolved with a small amount of DCM, then slowly added dropwise to the ether solution, filtered, collected the precipitate and dissolved again with a small amount of DCM; the solution was slowly added dropwise to the ether solution, filtered, collected the precipitate, and finally dried to obtain PEGylated lipid **E4-1** (1.19 g). ¹H NMR (400 MHz, CDCl₃) δ: 4.46-4.44 (m, 1H, >C*H*-), 4.06-4.03 (m, 4H, -C(=O)OC*H*₂-), 3.91 (d, 2H, -NHC(=O)C*H*₂NH-), 3.88-3.54 (m, PEG-*H*), 3.46-3.40 (m, 2H, PEG-CH₂C*H*₂NH-), 3.37 (s, 3H, -OC*H*₃), 3.29-3.12 (m, 2H, -CH₂CH₂CH₂C*H*₂NH-), 1.78-1.24 (m, 54H, >CHC*H*₂C*H*₂C*H*₂CH₂-, 6H; -C(=O)OCH₂(C*H*₂)₁₂CH₃, 48H), 0.89 (t, 6H, -CH₂C*H*₃). The molecular weight of **E4-1** determined by GPC was approximately 2.7 kDa and PDI=1.02.

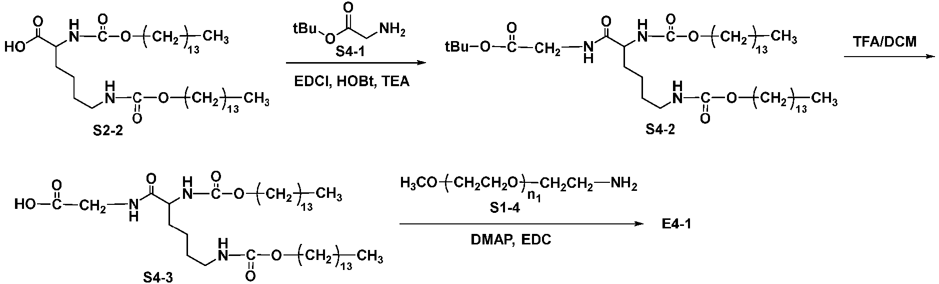

### Example-4.2: PEGylated lipid (E4-2)

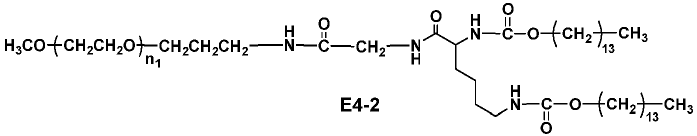

The preparation process is as follows:
**S1-5** (1.26 g, 0.6 mmol) was dissolved in a mixed solution of anhydrous DMF and anhydrous DCM (1:8, v/v). **DMAP** (0.19 g, 1.6 mmol), **S4-3** (0.82 g, 1.2 mmol) and **EDC** (0.23 g, 1.2 mmol) were slowly added to the solution successively under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E4-2** (1.21 g). ¹H NMR (400 MHz, CDCl₃) δ: 4.48-4.45 (m, 1H, >C*H*-), 4.06-4.03 (m, 4H, -C(=O)OC*H*₂-), 3.90 (d, 2H, -NHC(=O)C*H*₂NH-), 3.87-3.54 (m, PEG-*H*), 3.43-3.41 (m, 2H, PEG-CH₂CH₂C*H*₂NH-), 3.37 (s, 3H, -OC*H*₃), 3.29-3.11 (m, 2H, -CH₂CH₂CH₂C*H*₂NH-), 1.78-1.24 (m, 56H, >CHC*H*₂C*H*₂C*H*₂CH₂-, 6H; -C(=O)OCH₂(C*H*₂)₁₂CH₃, 48H; PEG-CH₂C*H*₂CH₂NH-, 2H), 0.87 (t, 6H, -CH₂C*H*₃). The molecular weight of **E4-2** determined by GPC was approximately 2.7 kDa and PDI=1.03.

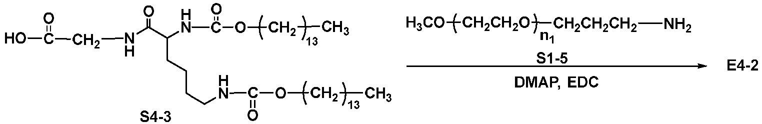

### Example-5.1: PEGylated lipid (E5-1)

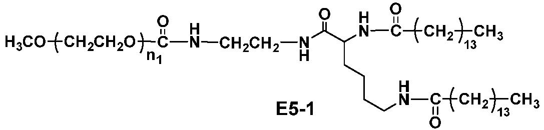

The preparation process is as follows:
Step a: **S1-3** (1.19 g, 2.0 mmol), **HOBt** (0.47 g, 2.2 mmol), O-Benzotriazole-*N*,*N*,*N'*,*N'*-tetramethyl-uronium-hexafluoro-phosphate (**HBTU,** 0.83 g, 2.2 mmol) and **DIPEA** (0.70 mL, 4.0 mmol) were added to dichloromethane (60 mL) and stirred for 30 min at room temperature to obtain reaction solution A. Ethylenediamine (**S5-1,** 6.00 g, 100.0 mmol) was dissolved in dichloromethane to obtain reaction solution B. Reaction solution A was slowly added to reaction solution B, and then the reaction was stirred at room temperature for 24 h. After completion of the reaction, the reaction solution was washed with 10% citric acid (20 mL), 1 M NaHCO₃ (20 mL) and saline (20 mL), concentrated, dried with anhydrous Na₂SO₄, filtered, concentrated the filtrate, and further purified by column chromatography to obtain the small molecule lipid intermediate **S5-2** (1.22 g, 96%).
Step b: A solution of **S5-3** (2.50 g, 1.0 mmol, mPEG-SC, Mw≈3100, n₁≈55, PDI=1.03) and triethylamine (0.70 mL, 3.2 mmol) was dissolved in anhydrous DMF (30 mL). **S5-2** (0.67 g, 1.1 mmol) was dissolved in anhydrous DMF (10 mL). The above solutions were mixed and stirred overnight at room temperature. After completion of the reaction, the reaction solution was concentrated to obtain a crude product, purified by column chromatography, concentrated, and drained by an oil pump to obtain PEGylated lipid **E5-1** (2.51 g). ¹H NMR (400 MHz, CDCl₃) δ: 4.48-4.46 (m, 1H, >C*H*-), 4.06-4.04 (m, 2H, -OCH₂C*H*₂OC(=O)-), 3.88-3.54 (m, PEG-*H*), 3.37 (s, 3H, -OC*H*₃), 3.35-3.12 (m, 4H, -NHC*H*₂C*H*₂NH-; 2H, -CH₂CH₂CH₂C*H*₂NH-), 2.28-2.20 (m, 4H, -C(=O)C*H*₂CH₂-), 1.78-1.24 (m, 54H, >CHC*H*₂C*H*₂C*H*₂CH₂-, 6H; -C(=O)CH₂(C*H*₂)₁₂CH₃, 48H), 0.88 (t, 6H, -CH₂C*H*₃). The molecular weight of **E5-1** determined by GPC was approximately 3.1 kDa and PDI=1.03.

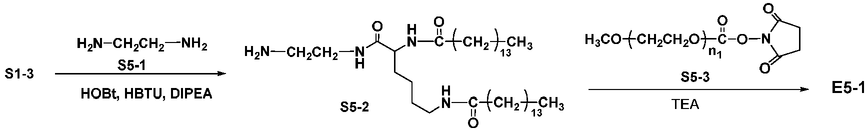

### Example-5.2: PEGylated lipid (E5-2)

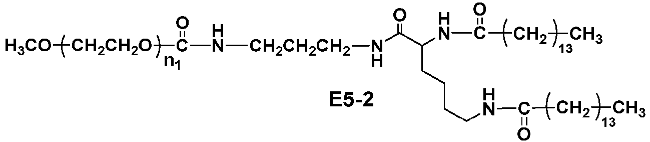

The preparation process is as follows:
Step a: **S1-3** (1.19 g, 2.0 mmol), **HOBt** (0.47 g, 2.2 mmol), **HBTU** (0.83 g, 2.2 mmol), and **DIPEA** (0.70 mL, 4.0 mmol) were added to dichloromethane (80 mL) successively and stirred at room temperature for 30 min to obtain reaction solution A. Ethylenediamine (**S5-4,** 7.40 g, 100.0 mmol) was dissolved in dichloromethane to obtain reaction solution B. Reaction solution A was slowly added to reaction solution B, and then the reaction was stirred at room temperature for 24 h. After completion of the reaction, the reaction solution was washed with 10% citric acid (30 mL), 1 M NaHCO₃ (30 mL) and saline (30 mL), concentrated, dried with anhydrous Na₂SO₄, filtered, and the filtrate was concentrated, and further purified by column chromatography to obtain the small molecule lipid intermediate **S5-5** (1.20 g, 94.6%).
Step b: A solution of **S5-3** (2.50 g, 1.0 mmol) and triethylamine (0.70 mL, 3.2 mmol) was dissolved in anhydrous DMF (30 mL). **S5-5** (0.68 g, 1.1 mmol) was dissolved in anhydrous DMF (10 mL). The above solutions were mixed and stirred overnight at room temperature. After completion of the reaction, the reaction solution was concentrated to obtain a crude product, purified by column chromatography, concentrated, and drained by oil pump to obtain PEGylated lipid **E5-2** (2.48 g). ¹H NMR (400 MHz, CDCl₃) δ: 4.49-4.46 (m, 1H, >C*H*-), 4.06-4.03 (m, 2H, -OCH₂C*H*₂OC(=O)-), 3.88-3.54 (m, PEG-*H*), 3.37 (s, 3H, -OC*H*₃), 3.35-3.12 (m, 6H, -NHC*H*₂CH₂C*H*₂NH-, 4H; -CH₂CH₂CH₂C*H*₂NH-, 2H), 2.28-2.20 (m, 4H, -C(=O)C*H*₂CH₂-), 1.78-1.24 (m, 56H, >CHC*H*₂C*H*₂C*H*₂CH₂-, 6H; -C(=O)CH₂(C*H*₂)₁₂CH₃, 48H; -NHCH₂C*H*₂CH₂NH-, 2H), 0.88 (t, 6H, -CH₂C*H*₃). The molecular weight of **E5-2** determined by GPC was approximately 3.1 kDa and PDI=1.03.

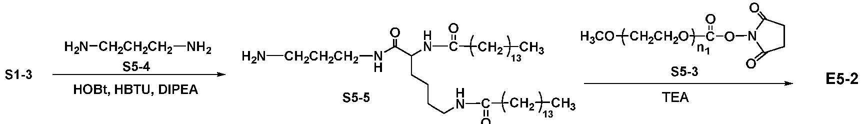

### Example-6.1: PEGylated lipid (E6-1)

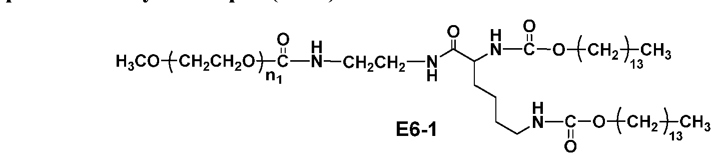

The preparation process is as follows:
Step a: **S2-2** (1.25 g, 2.0 mmol), **HOBt** (0.47 g, 2.2 mmol), **HBTU** (0.83 g, 2.2 mmol), and **DIPEA** (0.70 mL, 4.0 mmol) were added to dichloromethane (80 mL) and stirred at room temperature for 30 min to obtain reaction solution A. **S5-1** (6.00 g, 100.0 mmol) was dissolved in dichloromethane to obtain reaction solution B. Reaction solution A was slowly added to reaction solution B, and then the reaction was stirred at room temperature for 24 h. After completion of the reaction, the reaction solution was washed with 10% citric acid (30 mL), 1 M NaHCO₃ (30 mL) and saline (30 mL), concentrated and dried with anhydrous Na₂SO₄, filtered, and filtrate was concentrated, and further purified by column chromatography to obtain the small molecule lipid intermediate **S6-1** (1.27 g, 95.1%).
Step b: A solution of **S5-3** (2.50 g, 1.0 mmol) and triethylamine (0.70 mL, 3.2 mmol) were dissolved in anhydrous DMF (30 mL). **S6-1** (0.70 g, 1.1 mmol) was dissolved in anhydrous DMF (10 mL). The above solutions were mixed and stirred overnight at room temperature. After completion of the reaction, the reaction solution was concentrated to obtain crude product, purified by column chromatography, concentrated, and drained by oil pump to obtain PEGylated lipid **E6-1** (2.51 g). ¹H NMR (400 MHz, CDCl₃) δ: 4.47-4.44 (m, 1H, >C*H*-), 4.06-4.03 (m, 6H, -C(=O)OC*H*₂-, 4H; -OCH₂C*H*₂OC(=O)-, 2H), 3.87-3.55 (m, PEG-H), 3.36 (s, 3H, -OC*H*₃), 3.35-3.12 (m, 6H, -NHC*H*₂C*H*₂NH-, 4H; -CH₂CH₂CH₂C*H*₂NH-, 2H), 1.78-1.24 (m, 54H, >CHC*H*₂C*H*₂C*H*₂CH₂-, 6H; -C(=O)OCH₂(C*H*₂)₁₂CH₃, 48H), 0.88 (t, 6H, -CH₂C*H*₃). The molecular weight of **E6-1** determined by GPC was approximately 3.1 kDa and PDI=1.03.

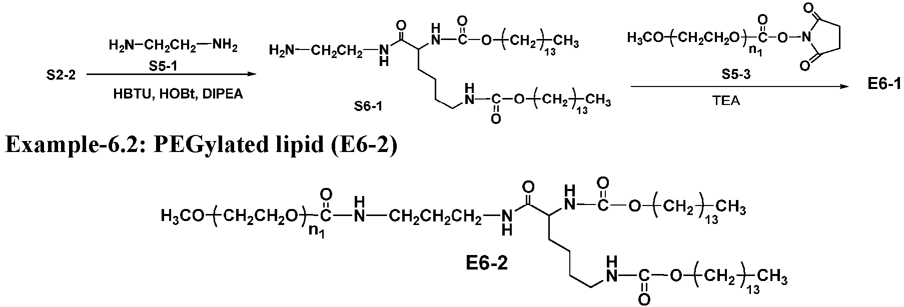

The preparation process is as follows:
Step a: **S2-2** (1.25 g, 2.0 mmol), **HOBt** (0.47 g, 2.2 mmol), **HBTU** (0.83 g, 2.2 mmol), and **DIPEA** (0.70 mL, 4.0 mmol) were added to dichloromethane (80 mL) and stirred at room temperature for 30 min to obtain reaction solution A. **S5-4** (7.40 g, 100.0 mmol) was dissolved in dichloromethane to obtain reaction solution B. Reaction solution A was slowly added to reaction solution B, and then the reaction was stirred at room temperature for 24 h. After completion of the reaction, the reaction solution was washed with 10% citric acid (30 mL), 1 M NaHCO₃ (30 mL) and saline (30 mL), concentrated and dried with anhydrous Na₂SO₄, filtered, and the filtrate was concentrated, and further purified by column chromatography to obtain the small molecule lipid intermediate **S6-2** (1.28 g, 94.3%).
Step b: A solution of **S5-3** (2.50 g, 1.0 mmol) and triethylamine (0.70 mL, 3.2 mmol) was dissolved in anhydrous DMF (30 mL). **S6-2** (0.72 g, 1.1 mmol) was dissolved in anhydrous DMF (10 mL). The above solutions were mixed and stirred overnight at room temperature. After completion of the reaction, the reaction solution was concentrated to obtain crude product, purified by column chromatography, concentrated, and drained by oil pump to obtain PEGylated lipid **E6-2** (2.60 g). ¹H NMR (400 MHz, CDCl₃) δ: 4.46-4.42 (m, 1H, >C*H*-), 4.06-4.03 (m, 6H, -C(=O)OC*H*₂-, 4H; -OCH₂C*H*₂OC(=O)-, 2H), 3.88-3.54 (m, PEG-H), 3.35-3.12 (m, 9H, -OC*H*₃, 3H; -NHC*H*₂CH₂C*H*₂NH-, 4H; -CH₂CH₂CH₂C*H*₂NH-, 2H), 1.78-1.24 (m, 56H, >CHC*H*₂C*H*₂C*H*₂CH₂-, 6H; -C(=O)OCH₂(C*H*₂)₁₂CH₃, 48H; -NHCH₂C*H*₂CH₂NH-, 2H), 0.88 (t, 6H). The molecular weight of **E6-2** determined by GPC was approximately 3.2 kDa and PDI=1.03.

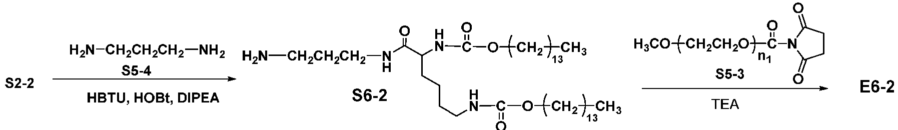

### Example-7.1: PEGylated lipid (E7-1)

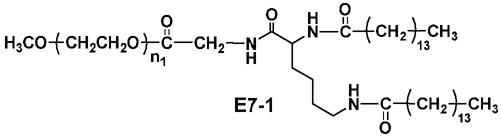

The preparation process is as follows:
**S7-1** (2.00 g, 1.0 mmol, 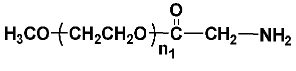 M_{w}≈2000, n₁≈44, PDI=1.03) was dissolved in a mixed solution of anhydrous DMF and anhydrous DCM (1:8, v/v). **DMAP** (0.32 g, 2.6 mmol), **S1-3** (1.19 g, 2.0 mmol) and **EDC** (0.38 g, 2.0 mmol) were slowly added successively under ice bath conditions, and stirred overnight at room temperature. After completion of the reaction, the filtrate was concentrated, dissolved with a small amount of DCM, then slowly added dropwise to the ether solution, filtered, collected the precipitate and dissolved with a small amount of DCM again, then slowly added dropwise to the ether solution, filtered, collected the precipitate, and finally dried to obtain PEGylated lipid **E7-1** (1.85 g). ¹H NMR (400 MHz, CDCl₃) δ: 4.48-4.44 (m, 1H, >C*H*-), 4.10 (d, 2H, -C(=O)C*H*₂NH-), 4.06-4.03 (m, 2H, -OCH₂C*H*₂OC(=O)-), 3.89-3.54 (m, PEG-*H*), 3.37 (s, 3H, -OC*H*₃), 3.29-3.12 (m, 2H, -CH₂CH₂CH₂C*H*₂NHCH₂-), 2.28-2.20 (m, 4H, -C(=O)C*H*₂CH₂-), 1.78-1.25 (m, 54H, >CHC*H*₂C*H*₂C*H*₂CH₂-, 6H; -C(=O)CH₂(C*H*₂)₁₂CH₃, 48H), 0.87 (t, 6H, -CH₂C*H*₃). The molecular weight of **E7-1** determined by GPC was approximately 2.6 kDa and PDI=1.03.

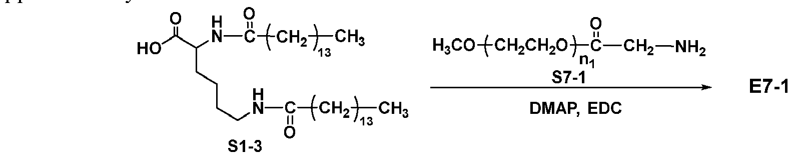

### Example-7.2: PEGylated lipid (E7-2)

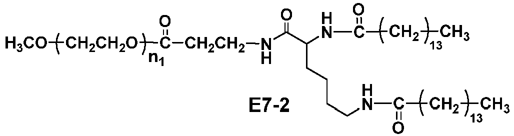

The preparation process is as follows:
Referring to the preparation process of **E7-1,** PEGylated lipid **E7-2** (1.88 g) was obtained using **S1-3** and **S7-2** ( 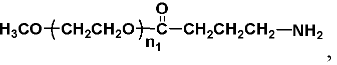 M_{w}≈2000, n₁≈44, PDI=1.02) as starting materials. The NMR hydrogen spectroscopy of **E7-2** was as follows: ¹H NMR (400 MHz, CDCl₃) δ: 4.48-4.44 (m, 1H, >C*H*-), 4.06-4.03 (m, 2H, -OCH₂C*H*₂OC(=O)-), 3.89-3.46 (m, PEG-*H*), 3.41-3.31 (m, 5H, -C(=O)CH₂CH₂C*H*₂NH-, 2H; -OC*H*₃, 3H,), 3.27-3.12 (m, 2H, -CH₂CH₂CH₂C*H*₂NHCH₂-), 2.32 (t, 2H, -C(=O)C*H*₂CH₂CH₂NH-), 2.28-2.20 (m, 4H, -C(=O)C*H*₂CH₂-), 2.00-1.82 (m, 2H, -C(=O)CH₂C*H*₂CH₂NH-), 1.75-1.25 (m, 54H, >CHC*H*₂C*H*₂C*H*₂CH₂-, 6H; -C(=O)CH₂(C*H*₂)₁₂CH₃, 48H), 0.88 (t, 6H, -CH₂C*H*₃). The molecular weight of **E7-2** determined by GPC was approximately 2.6 kDa and PDI=1.02.

### Example-7.3: PEGylated lipid (E7-3)

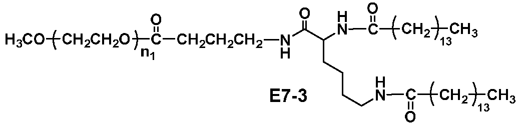

The preparation process is as follows:
Referring to the preparation process of **E7-1** PEGylated lipid **E7-3** (1.90 g) was obtained using **S1-3** and **S7-3** ( 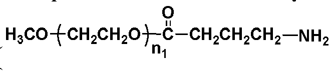 M_{w}≈2000, n₁≈44, PDI=1.03) as starting materials. The NMR hydrogen spectroscopy of **E7-3** was as follows: ¹H NMR (400 MHz, CDCl₃) δ: 4.48-4.44 (m, 1H, >C*H*-), 4.06-4.03 (m, 2H, -OCH₂C*H*₂OC(=O)-), 3.89-3.46 (m, PEG-*H*), 3.41-3.31 (m, 5H, -C(=O)CH₂CH₂C*H*₂NH-, 2H; -OC*H*₃, 3H,), 3.27-3.12 (m, 2H, -CH₂CH₂CH₂C*H*₂NHCH₂-), 2.32 (t, 2H, -C(=O)C*H*₂CH₂CH₂NH-), 2.28-2.20 (m, 4H, -C(=O)C*H*₂CH₂-), 2.00-1.82 (m, 2H, -C(=O)CH₂C*H*₂CH₂NH-), 1.75-1.25 (m, 54H, >CHCH₂C*H*₂C*H*₂CH₂-, 6H; -C(=O)CH₂(C*H*₂)₁₂CH₃, 48H), 0.88 (t, 6H, -CH₂C*H*₃). The molecular weight of **E7-3** is determined to be about 2.6 kDa and PDI=1.03.

### Example-7.4: PEGylated lipid (E7-4)

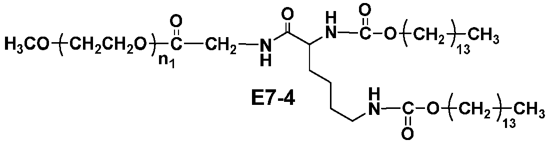

The preparation process is as follows:
**S7-1** (2.00 g, 1.0 mmol) was dissolved in a mixed solution of anhydrous DMF and anhydrous DCM (1:8, v/v). **DMAP** (0.32 g, 2.6 mmol), **S2-2** (1.25 g, 2.0 mmol) and **EDC** (0.38 g, 2.0 mmol) were slowly added successively under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E7-4** (1.61 g). ¹H NMR (400 MHz, CDCl₃) δ: 4.48-4.45 (m, 1H, *>CH*-)*,* 4.11 (d, 2H, -C(=O)C*H*₂NH-), 4.06-4.03 (m, 6H, -C(=O)OC*H*₂-, 4H; -OCH₂C*H*₂OC(=O)-, 2H), 3.88-3.54 (m, PEG-H), 3.35 (s, 3H, -OC*H*₃), 3.28-3.12 (m, 2H, -CH₂CH₂CH₂C*H*₂NHCH₂-), 1.78-1.24 (m, 54H, >CHCH₂C*H*₂C*H*₂CH₂-, 6H; -C(=O)OCH₂(C*H*₂)₁₂CH₃, 48H), 0.89 (t, 6H, -CH₂C*H*₃). The molecular weight of **E7-4** determined by GPC was approximately 2.6 kDa and PDI=1.03.

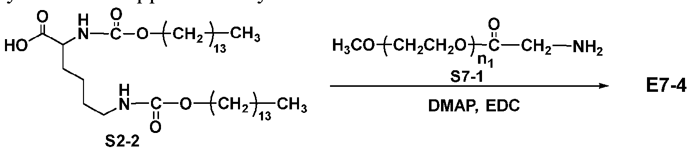

### Example-7.5: PEGylated lipid (E7-5)

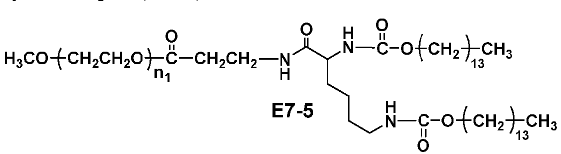

Referring to the preparation process of **E7-4,** PEGylated lipid **E7-5** (1.87 g) was obtained using **S2-2** and **S7-2** ( 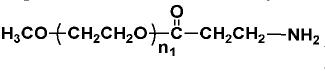 ) as starting materials and using the same molar amount. The NMR hydrogen spectra of **E7-5** was as follows: ¹H NMR (400 MHz, CDCl₃) δ: 4.48-4.45 (m, 1H, >C*H*-), 4.06-4.03 (m, 6H, -C(=O)OC*H*₂-, 4H; -OCH₂C*H*₂OC(=O)-, 2H), 3.89-3.47 (m, 176H, PEG-H; -OC(=O)CH₂C*H*₂NH-, 2H), 3.35 (s, 3H, -OC*H*₃), 3.29-3.12 (m, 2H, -CH₂CH₂CH₂C*H*₂NHCH₂-), 2.56 (t, 2H, -C(=O)C*H*₂CH₂NH-), 1.78-1.24 (m, 54H, >CHCH₂C*H*₂C*H*₂CH₂-, 6H; -C(=O)OCH₂(C*H*₂)₁₂CH₃, 48H), 0.87 (t, 6H, -CH₂C*H*₃). The molecular weight of **E7-5** determined by GPC was approximately 2.6 kDa and PDI=1.02.

### Example-7.6: PEGylated lipid (E7-6)

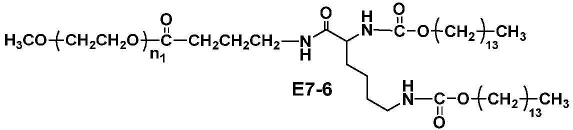

Referring to the preparation process of **E7-4,** PEGylated lipid **E7-6** (1.90 g) was obtained by using **S2-2** and **S7-3** ( 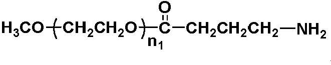 ) as starting materials and using the same molar amount. The NMR hydrogen spectra of **E7-6** was as follows: ¹H NMR (400 MHz, CDCl₃) δ: 4.48-4.45 (m, 1H, >C*H*-), 4.06-4.03 (m, 6H, -C(=O)OC*H*₂-, 4H; -OCH₂C*H*₂OC(=O)-, 2H), 3.88-3.54 (m, PEG-H), 3.41-3.32 (m, 5H, -C(=O)CH₂CH₂C*H*₂NH-, 2H; -OC*H*₃, 3H), 3.27-3.12 (m, 2H, -CH₂CH₂CH₂C*H*₂NHC(=O)-), 2.33 (t, 2H, -C(=O)CH₂C*H*₂CH₂NH-), 2.00-1.83 (m, 2H, -C(=O)CH₂C*H*₂CH₂NH-), 1.78-1.24 (m, 54H, >CHCH₂C*H*₂C*H*₂CH₂-, 6H; -C(=O)OCH₂(C*H*₂)₁₂CH₃, 48H), 0.89 (t, 6H, -CH₂C*H*₃). The molecular weight of **E7-6** determined by GPC was approximately 2.7 kDa and PDI=1.03.

### Example-8.1: PEGylated lipid (E8-1)

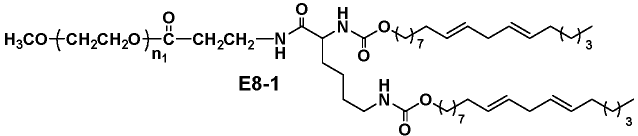

The preparation process is as follows:
**S7-2** (2.00 g, 1.0 mmol) was dissolved in a mixed solution of anhydrous DMF and anhydrous DCM (1:8, v/v). **DMAP** (0.32 g, 2.6 mmol), **S3-2** (1.52 g, 2.0 mmol) and **EDC** (0.38 g, 2.0 mmol) were slowly added successively under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E8-1** (1.96 g). ¹H NMR (400 MHz, CDCl₃) δ: 5.38-5.26 (m, 8H, -CH₂C*H*=C*H*CH₂C*H*=C*H*CH₂-), 4.48-4.44 (m, 1H, >C*H*-), 4.06-4.03 (m, 6H, -C(=O)OC*H*₂- , 4H; -OCH₂C*H*₂OC(=O)-, 2H), 3.88-3.45 (m, 176H, PEG-*H*; -OC(=O)CH₂C*H*₂NH-, 2H), 3.37 (s, 3H, -OC*H*₃), 3.29-3.12 (m, 2H, -CH₂CH₂CH₂C*H*₂NHC(=O)-), 2.78-2.74 (m, 4H, -CH=CHC*H*₂CH=CH-), 2.55 (t, 2H, -C(=O)C*H*₂CH₂NH-), 2.04-1.99 (m, 8H, -CH₂C*H*=C*H*CH₂C*H*=C*H*CH₂-), 1.78-1.24 (m, 42H, >CHCH₂C*H*₂C*H*₂CH₂-, 6H; -C(=O)OCH₂(C*H*₂)₆CH₂CH=CHCH₂CH=CHCH₂(C*H*₂)₃CH₃, 36H), 0.88 (t, 6H, -CH₂C*H*₃). The molecular weight of **E8-1** determined by GPC was approximately 2.8 kDa and PDI=1.02.

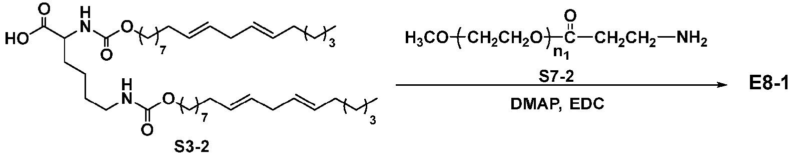

### Example-8.2: PEGylated lipid (E8-2)

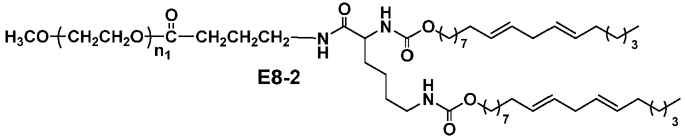

The preparation process is as follows:
**S7-3** (2.00 g, 1.0 mmol) was dissolved in a mixed solution of anhydrous DMF and anhydrous DCM (1:8, v/v). **DMAP** (0.32 g, 2.6 mmol), **S3-2** (1.52 g, 2.0 mmol) and **EDC** (0.38 g, 2.0 mmol) were slowly added to the solution successively under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E8-2** (1.97 g). ¹H NMR (400 MHz, CDCl₃) δ: 5.38-5.26 (m, 8H, -CH₂C*H*=C*H*CH₂C*H*=C*H*CH₂-), 4.46-4.43 (m, 1H, >C*H*-), 4.06-4.03 (m, 6H, -C(=O)OC*H*₂-, 4H; -OCH₂C*H*₂OC(=O)-, 2H), 3.88-3.54 (m, PEG-*H*), 3.41-3.31 (m, 5H, -C(=O)CH₂CH₂C*H*₂NH-, 2H; -OC*H*₃, 3H), 3.29-3.12 (m, 2H, -CH₂CH₂CH₂C*H*₂NHC(=O)-), 2.78-2.75 (m, 4H, -CH=CHC*H*₂CH=CH-), 2.34 (t, 2H, -C(=O)CH₂C*H*₂CH₂NH-), 2.04-1.99 (m, 8H, -CH₂C*H*=C*H*CH₂C*H*=C*H*CH₂-), 2.00-1.81 (m, 2H, -C(=O)CH₂C*H*₂CH₂NH-), 1.78-1.24 (m, 42H, >CHCH₂C*H*₂C*H*₂CH₂-, 6H; -CH₂(C*H*₂)₆CH₂-, 24H; -CH₂(C*H*₂)₃CH₃, 12H), 0.88 (t, 6H, -CH₂C*H*₃). The molecular weight of **E8-2** determined by GPC was approximately 2.8 kDa and PDI=1.03.

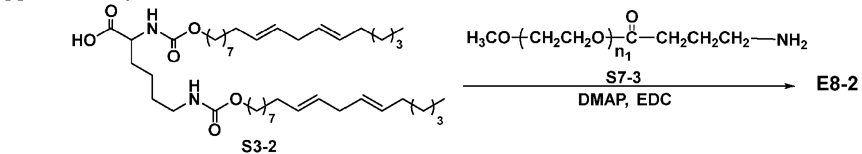

### Example-9.1: PEGylated lipid (E9-1)

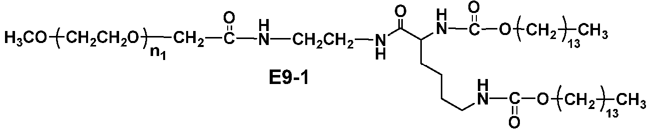

The preparation process is as follows:
**S9-1** (2.70 g, 1.0 mmol, mPEG-CM, M_{w}≈2700, n₁≈60, PDI=1.02) was dissolved in a mixed solution of anhydrous DMF and anhydrous DCM (1:8, v/v). **DMAP** (0.32 g, 2.6 mmol), **S6-1** (1.34 g, 2.0 mmol) and **EDC** (0.38 g, 2.0 mmol) were slowly added to the solution successively under ice bath conditions, and then the solution was stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, collected the precipitate, and finally dried to obtain PEGylated lipid **E9-1** (2.48 g). ¹H NMR (400 MHz, CDCl₃) δ: 4.46-4.42 (m, 1H, >C*H*-), 3.96 (s, 2H, -OC*H*₂C(=O)NH-), 4.06-4.03 (m, 4H, -C(=O)OC*H*₂-), 3.88-3.54 (m, PEG-*H*), 3.38 (s, 3H, -OC*H*₃), 3.29-3.12 (m, 6H, -NHC*H*₂C*H*₂NH-, 4H; -CH₂CH₂CH₂C*H*₂NHC(=O)-, 2H), 1.78-1.24 (m, >CHCH₂C*H*₂C*H*₂CH₂-, 6H; -C(=O)OCH₂(C*H*₂)₁₂CH₃, 48H), 0.89 (t, 6H, -CH₂C*H*₃). The molecular weight of **E9-1** determined by GPC was approximately 3.4 kDa and PDI=1.02.

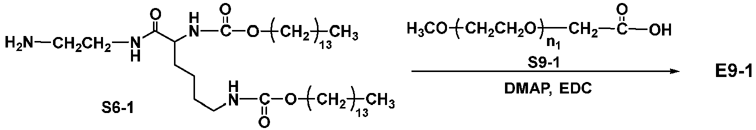

### Example-9.2: PEGylated lipid (E9-2)

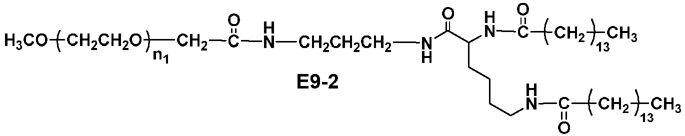

The preparation process is as follows:
**S9-1** (2.70 g, 1.0 mmol) was dissolved in a mixed solution of anhydrous DMF and anhydrous DCM (1:8, v/v). **DMAP** (0.32 g, 2.6 mmol), **S6-2** (1.36 g, 2.0 mmol) and **EDC** (0.38 g, 2.0 mmol) were slowly added to the solution successively under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, and then slowly added dropwise to the ether solution, filtered, collected the precipitate, and finally dried to obtain PEGylated lipid **E9-2** (2.44 g). ¹H NMR (400 MHz, CDCl₃) δ: 4.46-4.42 (m, 1H, *>CH*-)*,* 3.95 (s, 2H, -C(=O)C*H*₂NH-), 3.88-3.54 (m, PEG-*H*), 3.37 (s, 3H, -OC*H*₃), 3.30-3.12 (m, 6H, -NHC*H*₂CH₂C*H*₂NH-, 4H; -CH₂CH₂CH₂C*H*₂NHC(=O)-, 2H), 2.28-2.20 (m, 4H, -C(=O)C*H*₂CH₂-), 1.78-1.24 (m, 54H, >CHCH₂C*H*₂C*H*₂CH₂-, 6H; -C(=O)OCH₂(C*H*₂)₁₂CH₃, 48H; -NHCH₂C*H*₂CH₂NH-, 2H), 0.88 (t, 6H, -CH₂C*H*₃). The molecular weight of **E9-2** determined by GPC was approximately 3.4 kDa and PDI=1.02.

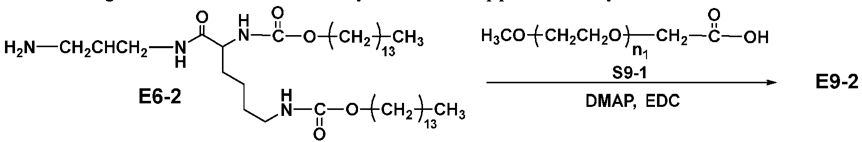

### Example-10: PEGylated lipid (E10-1)

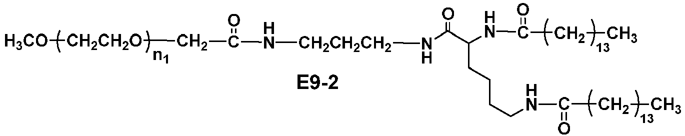

The preparation process is as follows:
**EDCI** (0.58 g, 3.0 mmol) and **DMAP** (0.12 g, 1.0 mmol) were added successively to a round-bottom flask containing **S3-2** (1.52 g, 2.0 mmol, mPEG-OH, M_{w}≈2700, n₁≈45, PDI=1.02) dissolved in dichloromethane (50 mL) and stirred overnight at room temperature. After completion of the reaction, the reaction solution was quenched with 1M HCl solution (50 mL) to separate the organic layer, and the aqueous layer was extracted with dichloromethane (50 mL*2), then the organic layer was combined, dried with anhydrous Na₂SO₄, concentrated, and purified by column chromatography to obtain PEGylated lipid **E10-1** (2.44 g, 87.2%). ¹H NMR (400 MHz, CDCl₃) δ: 5.36-5.25 (m, 8H, -CH₂C*H*=C*H*CH₂C*H*=C*H*CH₂-), 4.48-4.43 (m, 1H, >C*H*-), 4.06-4.03 (m, 6H, -C(=O)OC*H*₂-, 4H; -OCH₂C*H*₂OC(=O)-, 2H), 3.88-3.54 (m, PEG-*H*), 3.37 (s, 3H, -OC*H*₃), 3.29-3.12 (m, 2H, -CH₂CH₂CH₂C*H*₂NHC(=O)-), 2.78-2.75 (m, 4H, -CH=CHC*H*₂CH=CH-), 2.04-1.99 (m, 8H, -CH₂C*H*=C*H*CH₂C*H*=C*H*CH₂-), 1.78-1.24 (m, 42H, >CHCH₂C*H*₂C*H*₂CH₂-, 6H; -CH₂(C*H*₂)₆CH₂-, 24H; -CH₂(C*H*₂)₃CH₃, 12H), 0.88 (t, 6H, -CH₂C*H*₃). The molecular weight of **E10-1** determined by GPC was approximately 2.7 kDa and PDI=1.02.

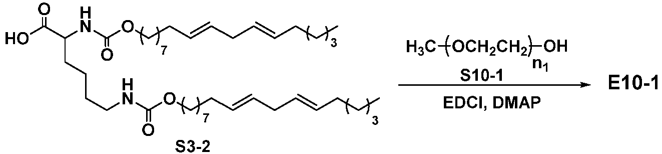

### Example-11: PEGylated lipid (E11-1)

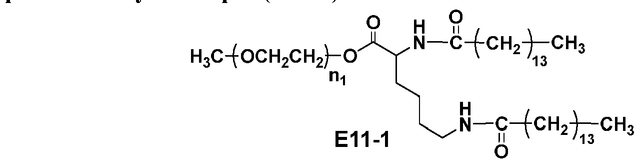

The preparation process is as follows:
**EDCI** (0.58 g, 3.0 mmol) and **DMAP** (0.12 g, 1.0 mmol) were added successively to a round-bottom flask containing **S1-3** (1.19 g, 2.0 mmol) and **S10-1** (2.00 g, 1.0 mmol) dissolved in dichloromethane (50 mL) and stirred overnight at room temperature. After completion of the reaction, the reaction solution was quenched with 1M HCl solution (50 mL) to separate the organic layer, and the aqueous layer was extracted with dichloromethane (50 mL*2), the organic layer was combined, dried with anhydrous Na₂SO₄, concentrated, and purified by column chromatography to obtain PEGylated lipid **E11-1** (2.29 g, 87%). ¹H NMR (400 MHz, CDCl₃) δ: 4.46-4.42 (m, 1H, >C*H*-), 4.06-4.03 (m, 2H, -OCH₂C*H*₂OC(=O)-), 3.88-3.54 (m, PEG-*H*), 3.37 (s, 3H, -OC*H*₃), 3.29-3.13 (m, 2H, -CH₂CH₂CH₂C*H*₂NHC(=O)-), 2.28-2.20 (m, 4H, -C(=O)C*H*₂CH₂-), 1.78-1.24 (m, 54H, >CHCH₂C*H*₂C*H*₂CH₂-, 6H; -C(=O)CH₂(C*H*₂)₁₂CH₃, 48H), 0.88 (t, 6H, -CH₂C*H*₃). The molecular weight of **E11-1** determined by GPC was approximately 2.6 kDa and PDI=1.02.

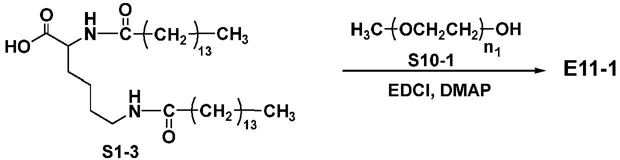

### Example-12: PEGylated lipid (E12-1)

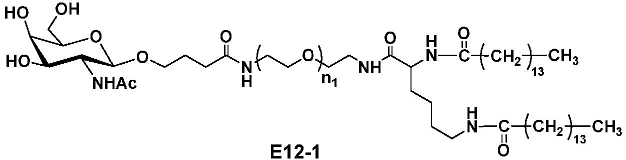

The preparation process is as follows:
Step a: Trimethylsilane trifluoromethanesulfonate (**TMSOTf,** 2.00 g, 9.0 mmol) was added to a round-bottom flask containing galactosamine pentaacetate **(S12-1,** 2.33 g, 6.0 mmol) dissolved in dichloromethane (50 mL) and stirred under a water bath at 50 °C for 90 min, then stopped heating and stirred the reaction overnight at room temperature. After completion of the reaction, the reaction solution was poured into ice-cold sodium bicarbonate solution, extracted with dichloromethane, and the organic phase was washed with water, dried with anhydrous Na₂SO₄ and concentrated to obtain compound **S12-2** (1.97 g), which was used directly for the next reaction without purification.
Step b: The above compounds **S12-2** (1.65 g, 5.0 mmol) and benzyl 4-hydroxybutyrate (**S12-3,** 1.46 g, 7.5 mmol) were dissolved in dichloromethane (20 mL), and an appropriate amount of molecular sieve was added and the reaction was stirred for 30 min. **TMSOTf** (0.56 g, 2.5 mmol) was added to the solution and stirred overnight at room temperature. After completion of the reaction, the reaction solution was added to ice-cold sodium bicarbonate solution, extracted with dichloromethane, and the organic phase was washed with water, dried with anhydrous Na₂SO₄. Compound **S12-4** (2.25 g) was obtained by concentration and purification by column chromatography.
Step c: Compound **S12-4** (1.57 g, 3.0 mmol) was dissolved in a mixture of methanol/ethyl acetate and degas with argon. Pd/C (0.15 g) was added to the mixture and the reaction was stirred overnight under balloon pressure. After completion of the reaction, the solution was filtered, rinsed with methanol, and dried in a vacuum to obtain compound **S12-5** (1.27 g, 98%).
Step d: **S12-6** (2.10 g, 1.0 mmol, M_{w}≈2100, n₁≈45, PDI=1.02) was dissolved in a mixed solution of anhydrous DMF and anhydrous DCM (1:8, v/v). **DMAP** (0.32 g, 2.6 mmol), **S12-5** (0.87 g, 2.0 mmol) and **EDC** (0.38 g, 2.0 mmol) were slowly added successively to the solution under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, and the precipitate was concentrated, finally dried to obtain **S12-7** (1.81 g).
Step e: **S12-7** (1.25 g, 0.5 mmol) was dissolved in a mixed solution of anhydrous DMF and anhydrous DCM (1:8, v/v). **DMAP** (0.16 g, 1.3 mmol), **S1-3** (0.59 g, 1.0 mmol) and **EDC** (0.19 g, 1.0 mmol) were slowly added successively to the solution under ice bath conditions and stirred overnight at room temperature. After completion of the reaction, the filtrate was concentrated, dissolved with a small amount of DCM, then slowly added dropwise to the ether solution, filtered, collected the precipitate and dissolved again with a small amount of DCM, then slowly added dropwise to the ether solution, filtered, and the precipitate was concentrated, finally dried to obtain **S12-8** (1.09 g).
Step f: Compound **S12-8** (0.90 g, 0.3 mmol) was dissolved in a mixed solution of MeOH/DCM (10 mL, 2:1), NaOMe (10 mL, 0.5 M solution in MeOH) was added to the solution and stirred overnight. After completion of the reaction, acetic acid was used to adjust the pH between 5-6, dried in a vacuum to obtain PEGylated lipid **E12-1** (0.80 g, 92%). ¹H NMR (400 MHz, CDCl₃) δ: 4.47-4.44 (m, 1H, *>CH-),* 4.34 (d, 1H, GalNAc-*H*), 3.88-3.54 (m, 186H, PEG-*H*; GalNAc-*H*, 5H; GalNAc-C*H*₂CH₂-, 1H), 3.47-3.40 (m, 2H, GalNAc-*H*, 1H; GalNAc-C*H*₂CH₂-, 1H), 3.29-3.12 (m, 6H, -CH₂CH₂CH₂C*H*₂NHC(=O)-, 2H; -CH₂C(=O)NHC*H*₂-, 2H; -C*H*₂NHC(=O)CH<, 2H), 2.28-2.20 (m, 6H, -C(=O)C*H*₂CH₂-, 4H; -C*H*₂C(=O)NHCH₂-, 2H), 2.01 (s, 3H, GalNAc-C*H*₃), 1.78-1.24 (m, 56H, >CHCH₂C*H*₂C*H*₂CH₂-, 6H; -C(=O)CH₂(C*H*₂)₁₂CH₃, 48H; GalNAc-CH₂C*H*₂CH₂C(=O)-, 2H), 0.88 (t, 6H, -CH₂C*H*₃). The molecular weight of **E12-1** determined by GPC was approximately 2.9 kDa and PDI=1.02.

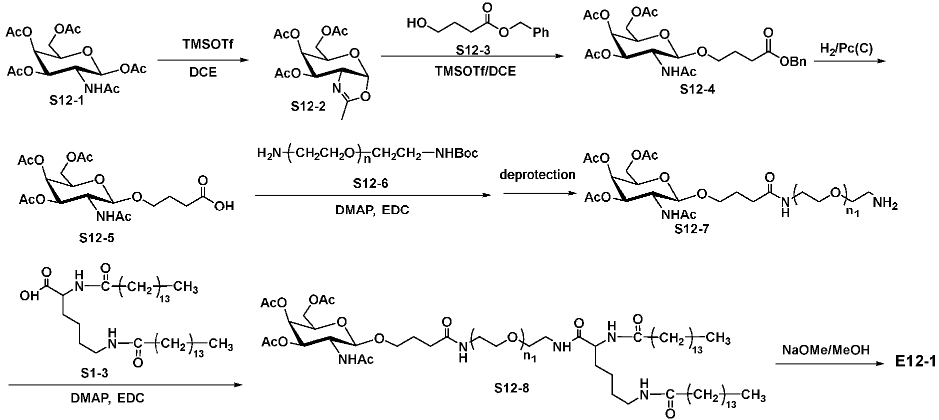

### Example 13: Preparation of LNP-mRNA pharmaceutical composition and testing of its physicochemical properties

### Example 13.1: Preparation of LNP-mRNA pharmaceutical composition

In the present embodiment, multiple sets of **LNP-mRNA** pharmaceutical compositions containing Fluc-mRNA were prepared for comparison. The phospholipid contained in each group was DSPC, the steroid lipid contained was cholesterol, and the cationic lipid was CL-1. The differences in each group were PEGylated lipid; wherein, the control groups (L-CT1 and L-CT2) contained the PEGylated lipid PEG2k-DMG (DMG) and ethylene glycol 2000 bistetradecanoate (PEG₂₀₀₀-Suc-TA₂), the experimental group series (L-1~L-25) contained the PEGylated lipid prepared in the Examples of the present application, specifically as shown in Table 1; the PEG₂₀₀₀-Suc-TA₂ was obtained with reference to the preparation method disclosed in CN114539083A, and its structure is as follows: 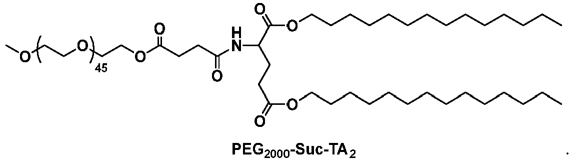 the **CL-1** was obtained with reference to the preparation method disclosed in CN113402405A, and its structure is as follows:

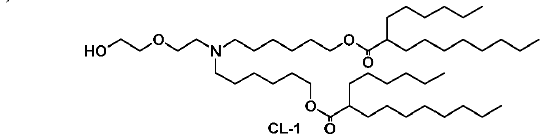

The preparation method for LNP-mRNA pharmaceutical composition is as follows:
Step a: Cationic lipid, DSPC, cholesterol and PEGylated lipid were dissolved in ethanol at a molar ratio of 48:9:42:1.5 to obtain an ethanol phase solution;
Step b: Fluc-mRNA was added into 10 to 50 mM citrate buffer (pH=4) to obtain an aqueous solution;
Step c: LNP-mRNA was prepared by mixing ethanol phase solution and aqueous phase solution, then washed by multiple DPBS ultrafiltration to remove ethanol and free molecules, and finally filtered through a sterile filter of 0.2 µm to obtain the LNP-mRNA pharmaceutical composition.

### Example 13.2: Testing of physicochemical properties of LNP-mRNA pharmaceutical compositions

Determination of encapsulation efficiency: the encapsulation efficiency of LNP-mRNA compositions was determined using the Quant-it Ribogreen RNA Quantification Kit. The results showed that the lipid composition of the present application (L-1~L-25) had a high nucleic acid encapsulation efficiency for nucleic acid drug (mRNA), all in the range of 80%-98%, and most of the encapsulation efficiency were in the range of 90%-98%. The results showed that the mRNA could be better encapsulated in the lipid compositions prepared by the PEGylated lipid in each experimental group, showing a better encapsulation efficiency than the existing PEGylated lipid, and there were also differences in the encapsulation efficiency among different PEGylated lipids.

Determination of particle size: in the present embodiment, the particle size of LNP-mRNA was determined by dynamic light scattering (DLS), and the results were shown in Table 1 below. The measured LNP-mRNA had high dimensional uniformity, and its PDI was less than 0.3. The particle size of the LNP-mRNA prepared with the lipid composition of the present application was in the range of 90-120 nm.

**Table 1: Summary table of the formulation of each lipid composition and particle size and encapsulation efficiency of the prepared LNP-mRNA**

| **LNP-mRNA** | **L-CT1** | **L-CT2** | **L-1** | **L-2** | **L-3** | **L-4** |
|---|---|---|---|---|---|---|
| **PEGylated lipid, PL** | DMG | PEG₂₀₀₀-S uc-TA₂ | E1-1 | E1-2 | E2-1 | E2-2 |
| **Cationic lipid, CL** | CL-1 | | | | | |
| **Particle size /nm** | 98 | 105 | 102 | 104 | 108 | 105 |
| **Encapsulation efficiency /%** | 91.7 | 91.4 | 90.4 | 91.7 | 92.2 | 93.5 |

| **LNP-mRNA** | **L-5** | **L-6** | **L-7** | **L-8** | **L-9** | **L-10** |
|---|---|---|---|---|---|---|
| **PEGylated lipid, PL** | E3-1 | E3-2 | E4-1 | E4-2 | E5-1 | E5-2 |
| **Cationic lipid, CL** | CL-1 | | | | | |
| **Particle size /nm** | 112 | 115 | 103 | 102 | 106 | 111 |
| **Encapsulation efficiency /%** | 91.7 | 91.4 | 92.1 | 91.9 | 89.0 | 90.7 |

| **LNP-mRNA** | **L-11** | **L-12** | **L-13** | **L-14** | **L-15** | **L-16** |
|---|---|---|---|---|---|---|
| **PEGylated lipid, PL** | E6-1 | E6-2 | E7-1 | E7-2 | E7-3 | E7-4 |
| **Cationic lipid, CL** | CL-1 | | | | | |
| **Particle size /nm** | 99 | 101 | 102 | 105 | 108 | 106 |
| **Encapsulation efficiency /%** | 92.5 | 92.3 | 91.5 | 92.6 | 92.1 | 93.2 |

| **LNP-mRNA** | **L-17** | **L-18** | **L-19** | **L-20** | **L-21** | **L-22** |
|---|---|---|---|---|---|---|
| **PEGylated lipid, PL** | E7-5 | E7-6 | E8-1 | E8-2 | E9-1 | E9-2 |
| **Cationic lipid, CL** | CL-1 | | | | | |
| **Particle size /nm** | 103 | 105 | 114 | 103 | 105 | |
| **Encapsulation efficiency /%** | 94.6 | 93.5 | 93.0 | 92.8 | 91.2 | 90.3 |

| **LNP-mRNA** | **L-23** | **L-24** | **L-25** | | | |
|---|---|---|---|---|---|---|
| **PEGylated lipid, PL** | E10-1 | E11-1 | E12-1 | | | |
| **Cationic lipid, CL** | CL-1 | CL-1 | CL-1 | | | |
| **Particle size /nm** | 112 | 103 | 107 | | | |
| **Encapsulation efficiency /%** | 92.5 | 92.2 | 91.8 | | | |

### Example 14: Biological activity assay of LNP-mRNA pharmaceutical composition formulations

### (1) Assays for Cytotoxicity (biocompatibility)

The cytotoxicity of the formulation of LNP-mRNA pharmaceutical composition was tested by commercially available cell proliferation assays (MTS, Promega). 293T cells were seeded in 96-well plates at 40,000 cells/well and cultured overnight. 293T cells were transfected with a Luciferase mRNA lipid composition formulation of 0.2 µg mRNA per well. Free Luciferase mRNA was used as a negative control and DMG and PEG₂₀₀₀-Suc-TA₂ were used as positive controls. After 24 h of transfection, the old medium was removed and replaced with a fresh medium containing MTS, incubated in the incubator for about 2 h, and the absorbance at 490 nm was detected by a microplate reader. According to the measured absorbance value, the results showed that the cell viability of the lipid composition L-1~L-25 in the experimental group were greater than or equal to 95%, that is, the cell viability of each group was selected from the group consisting of 95%, 96%, 97%, 98%, 99% and 100%, which indicated that the PEGylated lipids synthesized in Example 1-12 had good biological safety.

### (2) Study of cell transfection rate

In order to investigate the mRNA transfection rate at the cellular level of each group of LNP-mRNA compositions prepared in Example 13 of the present application, Luciferase bioluminescence was used for testing. The formulation of LNP-mRNA composition was dissolved in medium to prepare the required dose, 293T cells were used as a cell model, and 100 µL of cell suspension per well was seeded into a black-edged clear-bottom 96-well plate at a seeding density of 4×10⁴ cells/well. After seeding, the cell was incubated in a cell culture incubator for 24 h and then dosed at a dose of 0.2 ug mRNA per well. the blank control group was added with the corresponding dose of free Fluc-mRNA, and after 24 h of transfection, the old medium was removed and replaced with sodium D-luciferin (1.5 mg/ mL) substrate. After incubation for 5 min, bioluminescence was detected using a microplate reader, the stronger the fluorescence indicates the more Fluc-mRNA that was transported into the cytoplasm and translated into the corresponding fluorescent protein. The experimental results were shown in Figure 1, wherein the ordinate value was the ratio of the fluorescence intensity value of each group to the fluorescence intensity of the blank control group. The results showed that the LNP-mRNA compositions prepared by the present application had excellent in vitro transfection effects, that was, the PEGylated lipid in the control group and the experimental group were all effective delivery vehicles, and the transfection effect of most lysine-core PEGylated lipid was better than that of the currently marketed DMG and the lipid nanoparticles prepared by disclosed PEG₂₀₀₀-Suc-TA₂. Specifically, PEGylated lipid containing only amide bonds showed poorer transfection, e.g., L-1 containing the PEGylated lipid E1-1 had lower fluorescence intensity than both controls, and the PEGylated lipid containing carbamate bond was better than both controls. For example, the fluorescence intensity of L-3 of E2-1 containing carbamate bond was higher than that of L-1 of E-1 containing amide bond, and the PEGylated lipid having one ester bond connecting polyethylene glycol with lysine showed the best transfection, such as L-14, L-17, and L-19 showed the strongest fluorescence intensity. The linking group of the PEGylated lipid of the present application containing a carbamate bond or an ester bond at the appropriate position or no ester bondwere more stable, and thus showed better in vitro cell transfection efficiency. When the linkage between PEG and lysine contained a degradable ester bond at the appropriate position, the PEGylated lipid could maintain stability and showed excellent delivery efficiency.

Those described above are only embodiments of the present application and are not for limitation. Any modification of equivalent structures or equivalent routes according to the present application, which may be applied in other related art in a direct or an indirect way, should be included in the scope of the present application.

For those skilled in the art, without departing from the spirit and scope of the present application, and without unnecessary experimentation, the present application can be implemented in a wider range under equivalent parameters, concentrations, and conditions. While the present application has given particular examples, it should be understood that the present application can be further modified. In conclusion, in accordance with the principles of the present application, the present application is intended to cover any alterations, uses, or improvements of the present application, including changes departing from the scope disclosed in this application but made using conventional techniques known in the art.

## Claims

1. A PEGylated lipid, wherein the structure is represented by the general formula (1): 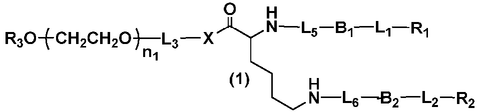 wherein,
X is -O- or -NH-;
L₁ and L₂ are each independently selected from the group consisting of a linking bond, -C(=O)-, -C(=O)O-, -OC(=O)-, -OC(=O)O-, -C(=O)NH-, -NHC(=O)-, -NHC(=O)O-, -OC(=O)NH- and -NHC(=O)NH-;
L₅ and L₆ are each independently selected from the group consisting of a linking bond, -C(=O)- and -C(=O)O-;
L₃ is a linking bond or a divalent linking group, and the divalent linking group is selected from the group consisting of -(CH₂)ₜ-, -O-, -C(=O)O-, -C(=O)O-, -OC(=O)O-, -OC(=O)O-, -C(=O)NH-, -NHC(=O)-, -NHC(=O)O-, -OC(=O)NH-, -NHC(=O)NH- and any combinations thereof; wherein, t is an integer from 1 to 12;
B₁ and B₂ are each independently a linking bond or a C₁₋₁₂ alkylene group;
R₁ and R₂ are each independently a C₅₋₃₀ aliphatic hydrocarbon group; wherein, the aliphatic hydrocarbon group is an alkyl group, an alkene group or an alkyne group;
R₃ is a hydrogen atom, a C₁₋₆ alkyl group, a carbocyclic group, a heterocyclic group or -L₄-R₀₁; wherein, L₄ is a divalent linking group and R₀₁ is a functional group that can react with bio-related substances;
n₁ is an integer from 20 to 1000.

2. The PEGylated lipid according to claim **1,** wherein the number of average molecular weight of polyethylene glycol chain corresponds to 900, 1000, 1500, 2000, 2500, 3000, 3350, 3500, 4000, 5000, 5500, 6000, 6500, 7000, 7500, 8000, 8500, 9000, 9500, 10000 or 11000.

3. The PEGylated lipid according to claim **1,** wherein the polyethylene glycol chain is polydisperse, and the number average degree of polymerization n₁ is preferably an integer from 20 to 100, more preferably an integer from 20 to 60, and more preferably an integer from 40 to 60.

4. The PEGylated lipid according to claim **1,** wherein the polyethylene glycol chain is monodisperse, and the number of EO unit is preferably 20 to 70, more preferably 20 to 60, and more preferably 20, 22, 24, 26, 27, 28, 29, 30, 32, 34, 36, 38, 40, 42, 44, 45, 46, 48, 50, 52, 54, 56, 58, or 60.

5. The PEGylated lipid according to claim **1,** wherein the L₃ is selected from the group consisting of a linking bond, -(CH₂)ₜₘ-, -C(=O)(CH₂)ₜₘ-, -C(=O)NH(CH₂)ₜₘ-, -C(=O)O(CH₂)ₜₘ-, -(CH₂)ₜₘC(=O)NH(CH₂)ₜₘ-, -(CH₂)ₜₘNHC(=O)(CH₂)ₜₘ-, -(CH₂)ₜₘC(=O)O(CH₂)ₜₘ- and -C(=O)(CH₂)ₜₘC(=O)NH(CH₂)ₜₘ-; wherein, the left end of L₃ is connected to the PEG chain, and tm is independently an integer from 1 to 6 at each occurrence;
more preferably, L₃ is selected from the group consisting of a linking bond, -(CH₂)₂₋₃-, -C(=O)(CH₂)₂₋₃-, -C(=O)NH(CH₂)₂₋₃-, -C(=O)O(CH₂)₁₋₄-, -(CH₂)₁₋₃C(=O)NH(CH₂)₂₋₃-, -(CH₂)₂₋₃NHC(=O)(CH₂)₁₋₃-, -(CH₂)₁₋₃C(=O)O(CH₂)₁₋₄- and -C(=O)(CH₂)₂₋₃C(=O)NH(CH₂)₂₋₃-;
more preferably, L₃ is selected from the group consisting of a linking bond, -CH₂CH₂-, -CH₂CH₂CH₂-, -C(=O)CH₂-, -C(=O)CH₂CH₂-, -C(=O)NHCH₂CH₂-, -C(=O)OCH₂CH₂CH₂-, -C(=O)OCH₂CH₂-, -C(=O)OCH₂CH₂CH₂-, -CH₂C(=O)NHCH₂CH₂-, -CH₂C(=O)NHCH₂CH₂CH₂-, -CH₂CH₂C(=O)NHCH₂CH₂-, -CH₂CH₂C(=O)NHCH₂CH₂CH₂-, -CH₂CH₂CH₂C(=O)NHCH₂CH₂-, -CH₂CH₂CH₂C(=O)NHCH₂CH₂CH₂-, -CH₂CH₂NHC(=O)CH₂-, -CH₂CH₂CH₂NHC(=O)CH₂-, -CH₂CH₂NHC(=O)CH₂CH₂-, -CH₂CH₂CH₂NHC(=O)CH₂CH₂-, -CH₂C(=O)OCH₂CH₂-, -CH₂C(=O)OCH₂CH₂CH₂-, -CH₂CH₂C(=O)OCH₂CH₂-, -CH₂CH₂C(=O)OCH₂CH₂CH₂-, -CH₂CH₂CH₂C(=O)OCH₂CH₂-, -CH₂CH₂CH₂C(=O)OCH₂CH₂CH₂-, -C(=O)CH₂CH₂C(=O)NHCH₂CH₂-, -C(=O)CH₂CH₂C(=O)NHCH₂CH₂CH₂-, -C(=O)CH₂CH₂CH₂C(=O)NHCH₂CH₂- and -C(=O)CH₂CH₂CH₂C(=O)NHCH₂CH₂CH₂-.

6. The PEGylated lipid according to claim **1,** wherein the L₁, L₂, L₅, L₆, B₁, and B₂ are selected from any of the following cases:
Case (1): B₁, B₂, L₁, and L₂ are all linking bonds;
Case (2): B₁, B₂, L₁, and L₂ are not linking bonds;
Case (3): L₅, L₆, B₁, and B₂ are all linking bonds.

7. The PEGylated lipid according to claim **6,** wherein the B₁, B₂, L₁, and L₂ are all linking bonds, and the PEGylated lipids are preferably selected from the group consisting of the following structures: 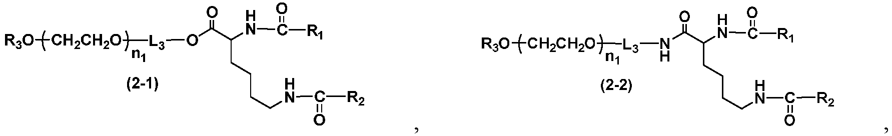 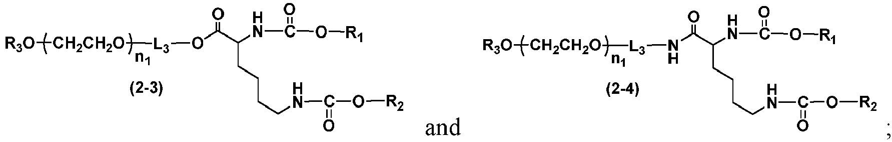 more preferably, the PEGylated lipid is represented by general formula (2-2) or formula (2-4); more preferably, the L₃ in general formula (2-2) and formula (2-4) not containing an ester bond; more preferably, the L₃ in general formula (2-2) and formula (2-4) is each independently selected from the group consisting of -(CH₂)₂₋₃-, -C(=O)(CH₂)₂₋₃-, -C(=O)NH(CH₂)₂₋₃-, -(CH₂)₁₋₃C(=O)NH(CH₂)₂₋₃-, -(CH₂)₂₋₃NHC(=O)(CH₂)₁₋₃- and -C(=O)(CH₂)₂₋₃C(=O)NH(CH₂)₂₋₃- at each occurrence; wherein, the left end of L₃ is connected with the PEG chain;
further, the structure of PEGylated lipid is preferably selected from the group consisting of the following formulas: 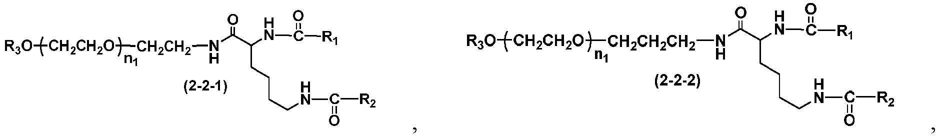 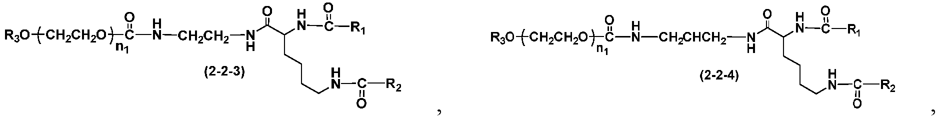 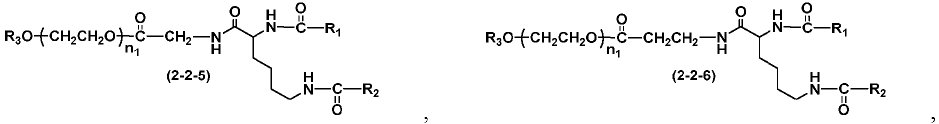 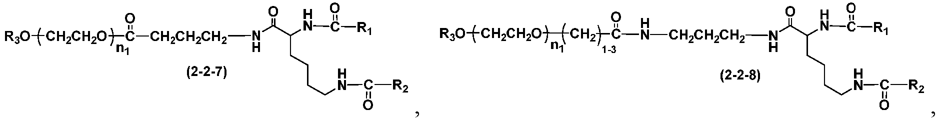 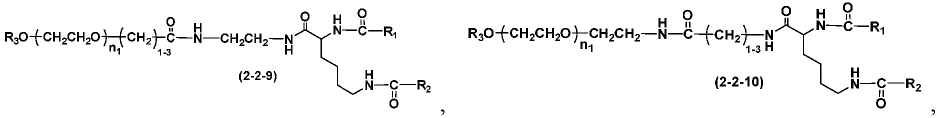 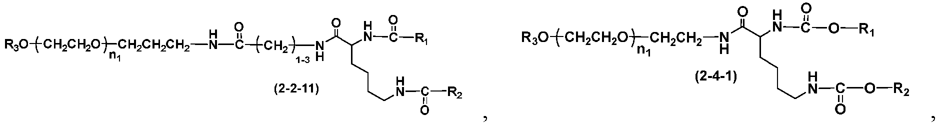 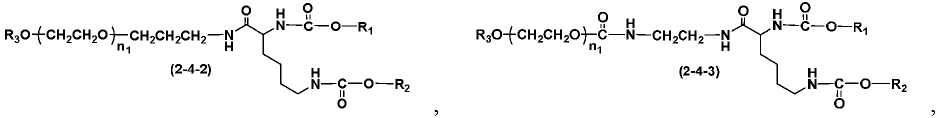 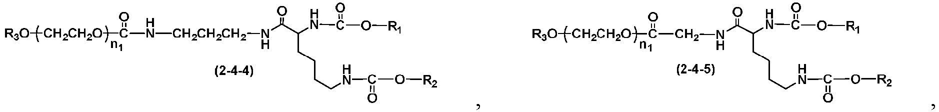 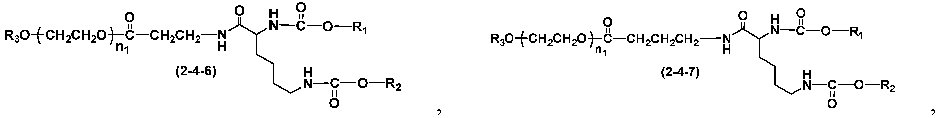 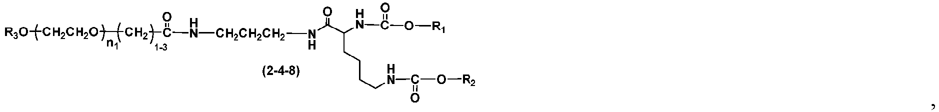 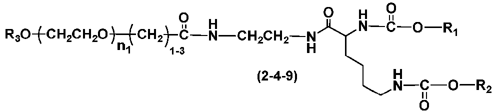 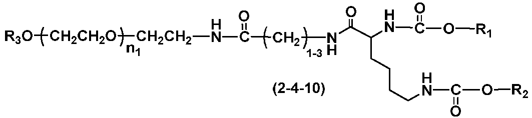 and

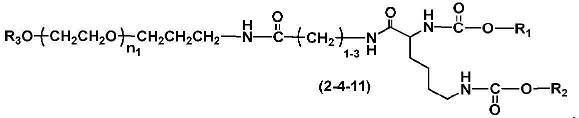

8. The PEGylated lipid according to claim 6, wherein the B₁, B₂, L₁, and L₂ are all not linking bonds; both L₁ and L₂ are non-ester-containing divalent linking groups; more preferably, the structure of PEGylated lipid is selected from the group consisting of the following formulas: 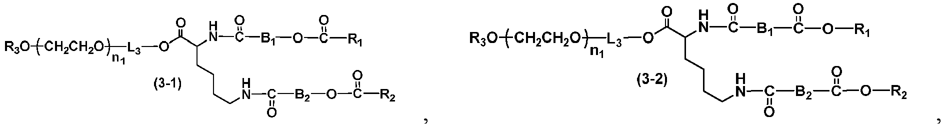 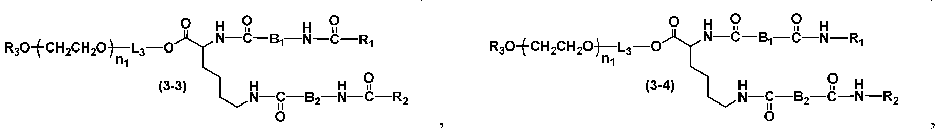 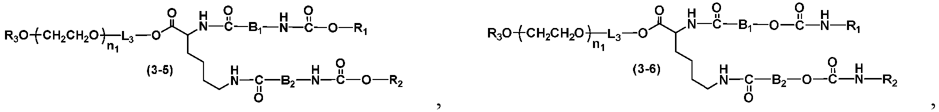 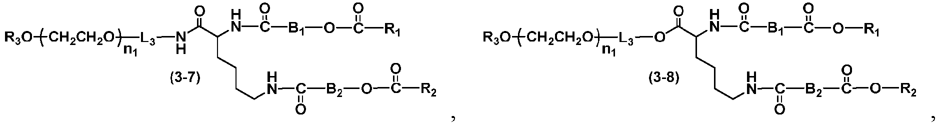 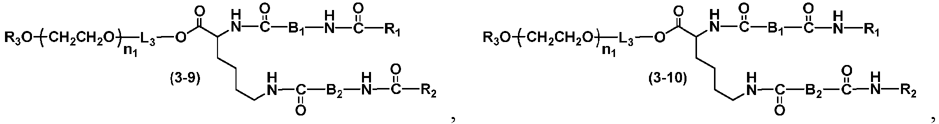 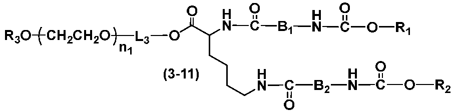 and

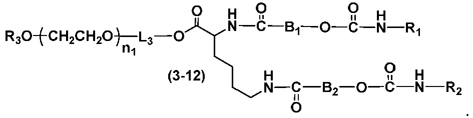

9. The PEGylated lipid according to claim **1,** wherein the R₃ is -L₄-R₀₁; wherein, L₄ is a divalent linking group, which is selected from the group consisting of -(CH₂)ₜ-, -O-, -C(=O)-, -C(=O)O-, -OC(=O)-, -OC(=O)O-, -C(=O)NH-, -NHC(=O)-, -NHC(=O)O-, -OC(=O)NH-, -NHC(=O)NH- and any combinations thereof; t is an integer from 1 to 12; and R₀₁ is selected from any of the following cases:
Case (1): the group consisting of an epoxy group, a hydroxyl group, a protected hydroxyl group, a sulfhydryl group, a protected sulfhydryl group, a carboxyl group, a protected carboxyl group, an amino group, a protected amino group, an aldehyde group, a protected aldehyde group, an active ester group, an alkenyl group, an enoate group, an azido group, a cyano group, a dithiopyridinyl group, an α-halogenated acetylene group, an alkyne group, 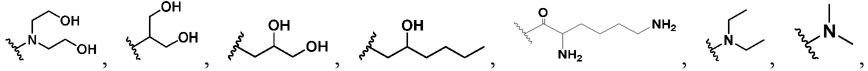 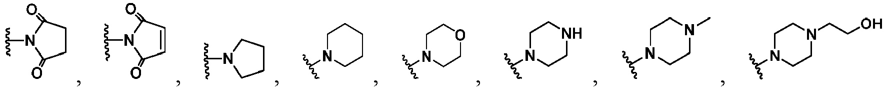 and 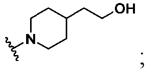
Case (2): a functional group with therapeutic targeting, and the functional group is preferably selected from the group consisting of a residue of folic acid, cholesterol, biotin, N-acetylgalactosamine and any functional derivative thereof;
further, the functional group is preferably selected from the group consisting of the following structures:

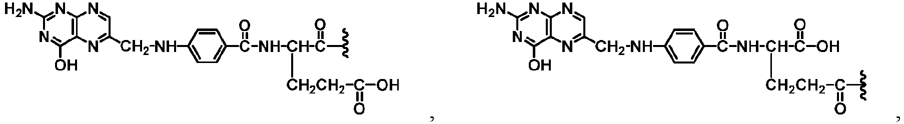

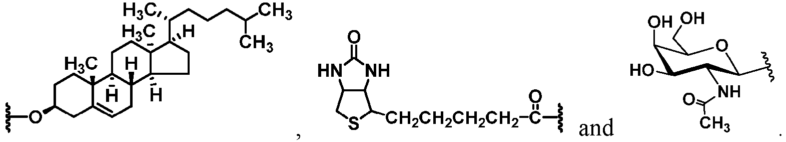

10. The PEGylated lipid according to claim 1, wherein the R₁ and R₂ are each independently represented by 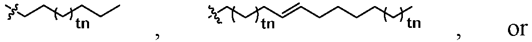 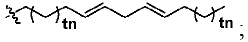 wherein, tn is independently an integer from 1 to 12 at each occurrence; R₁ and R₂ are preferably same, and selected from the group consisting of

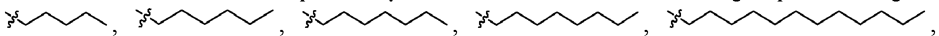

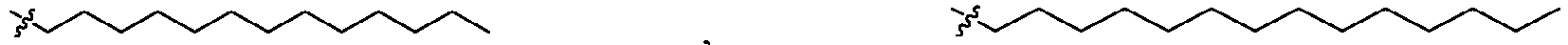

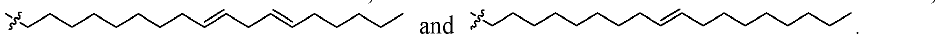

11. The PEGylated lipid according to claim **1,** wherein the B₁ and B₂ are each independently selected from the group consisting of a pentylene group, a hexylene group, a heptylene group, an octylene group, a nonylene group and a decylene group.

12. The PEGylated lipid according to claim **1,** wherein the PEGylated lipid is selected from the group consisting of the following structures: 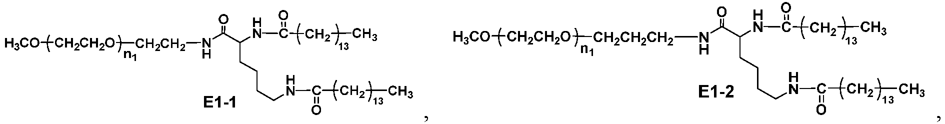 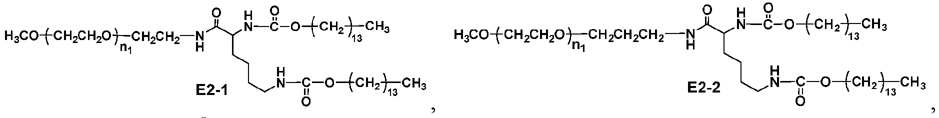 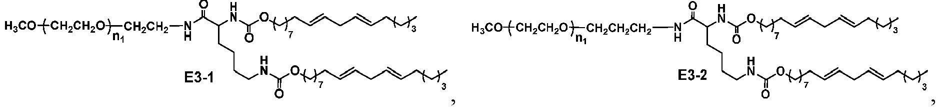 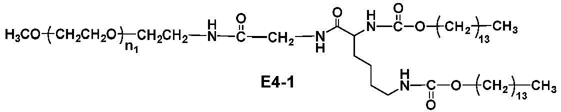 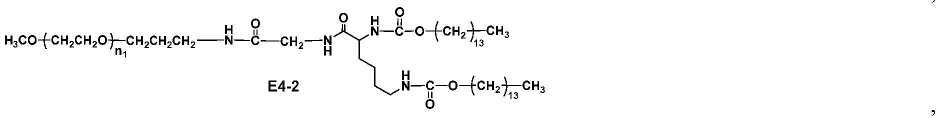 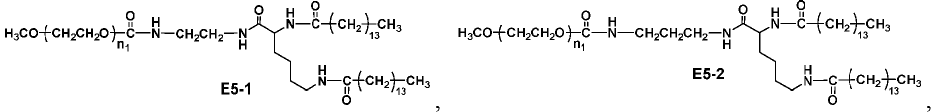 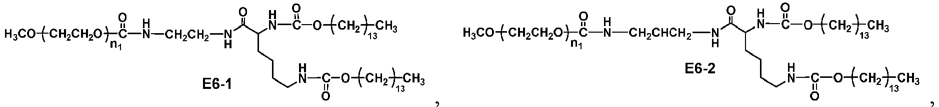 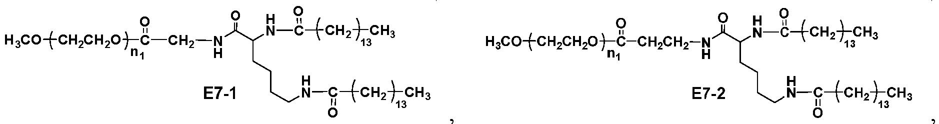 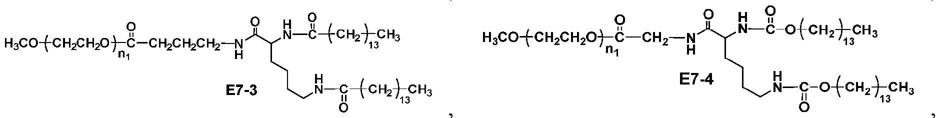 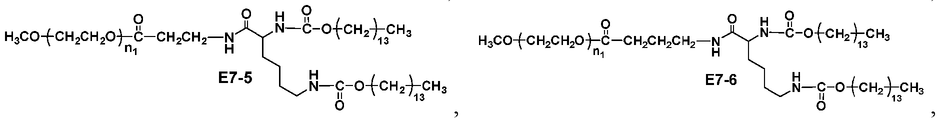 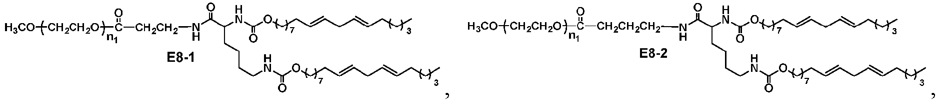 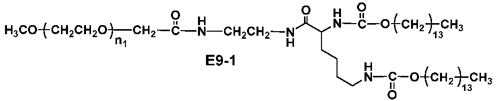 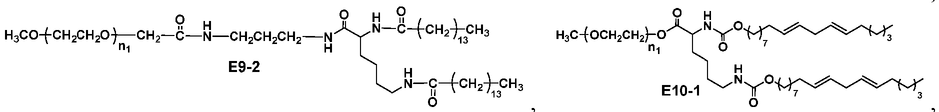 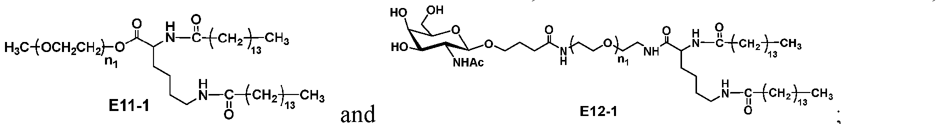 or the PEGylated lipid is selected from the group consisting of the following structures:

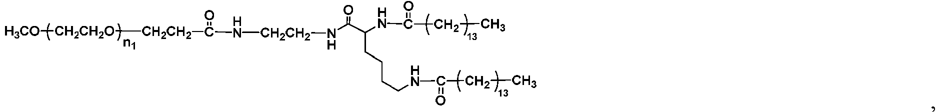

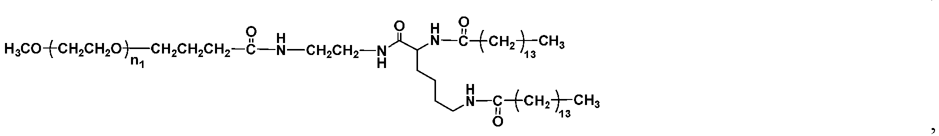

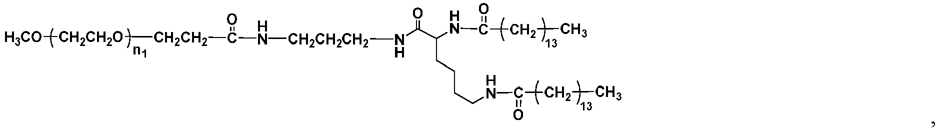

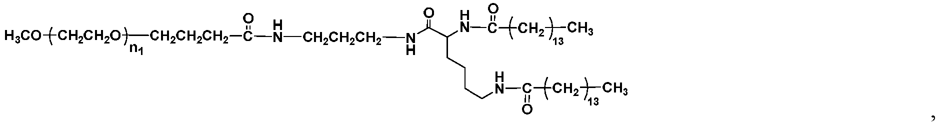

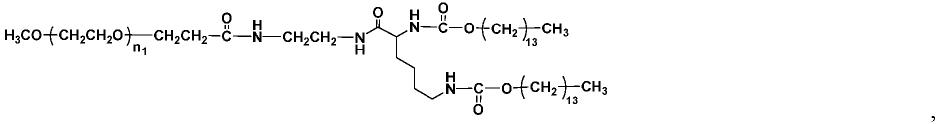

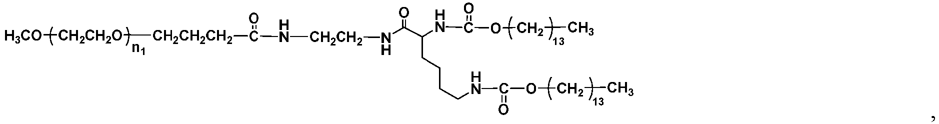

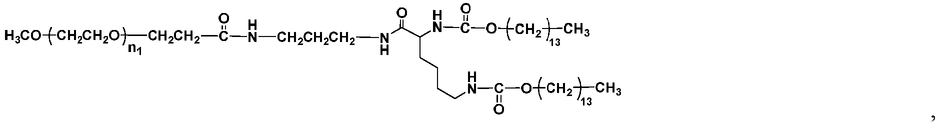

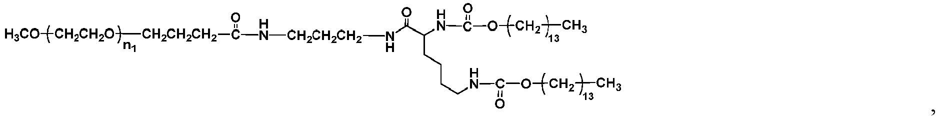

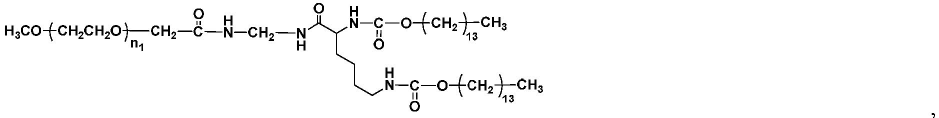

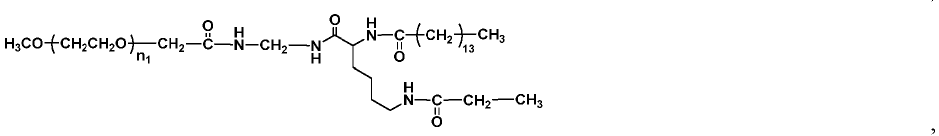

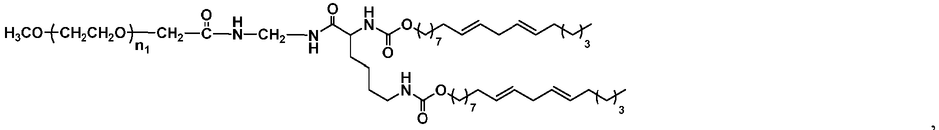

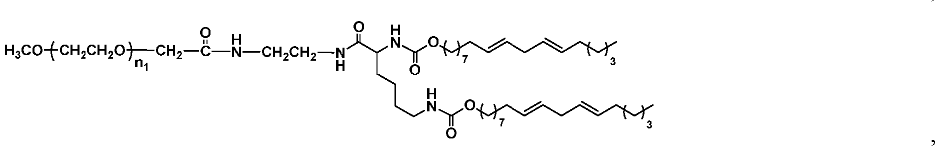

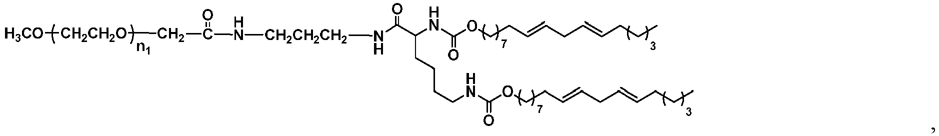

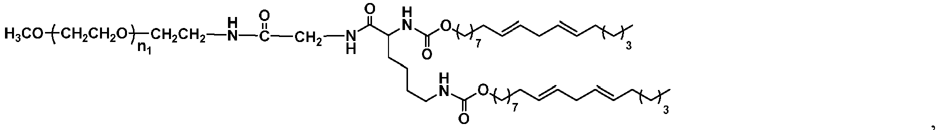

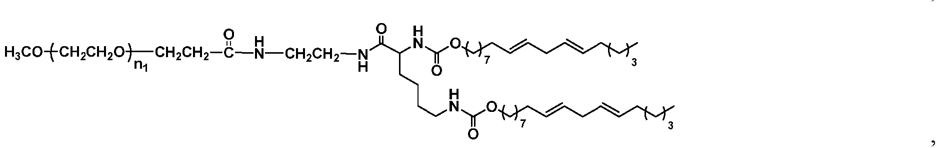

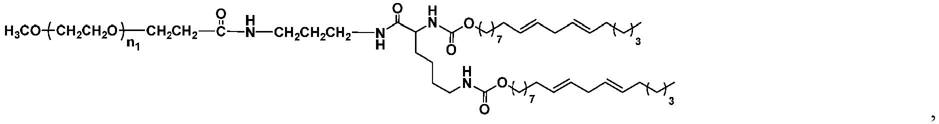

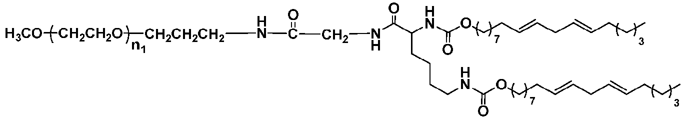

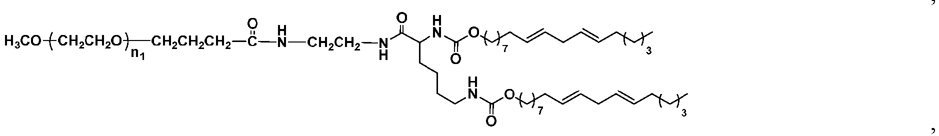

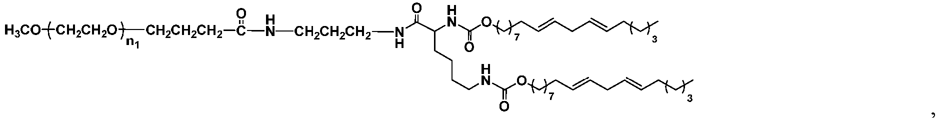

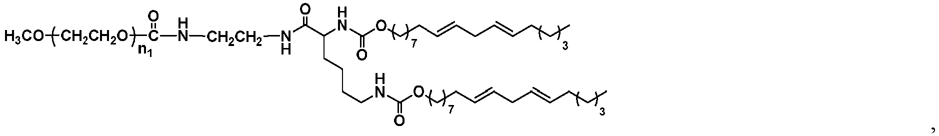

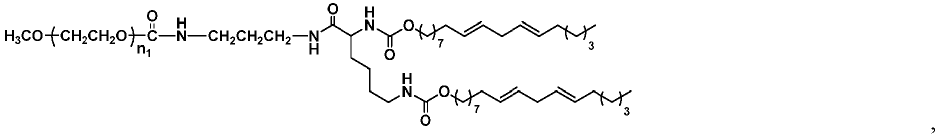

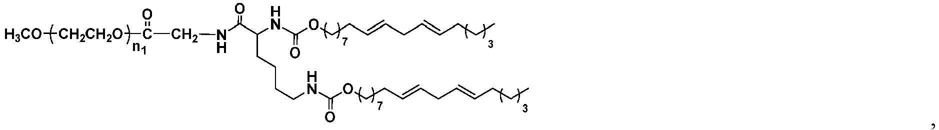

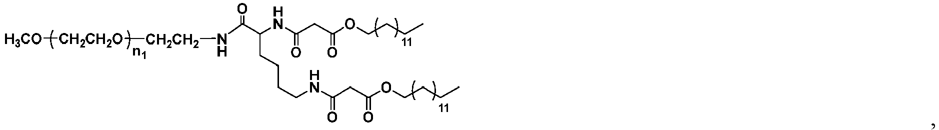

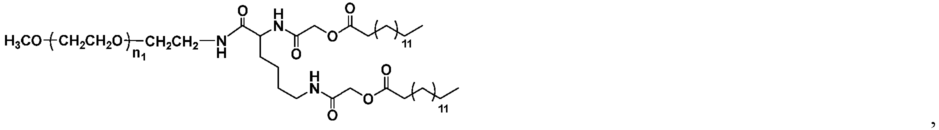

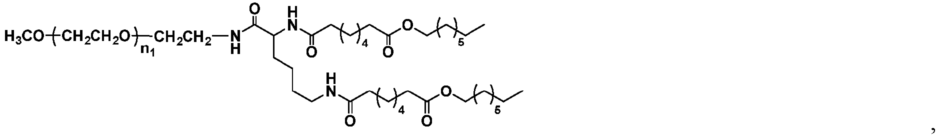

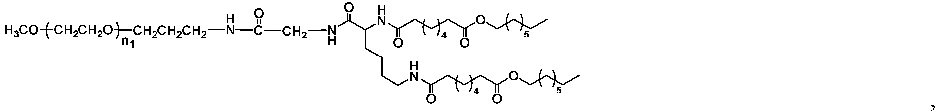

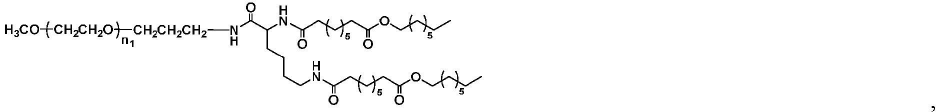

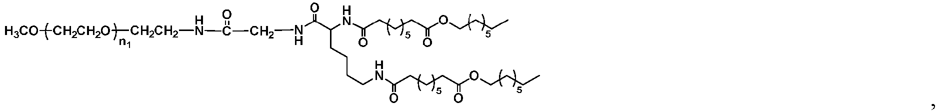

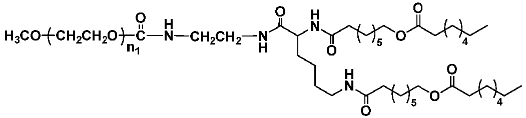

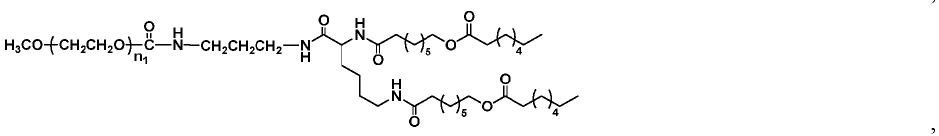

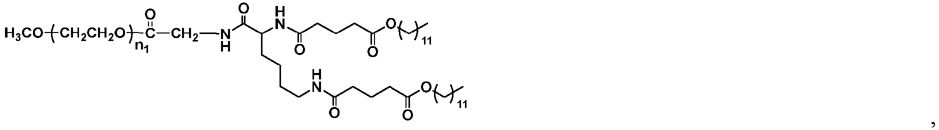

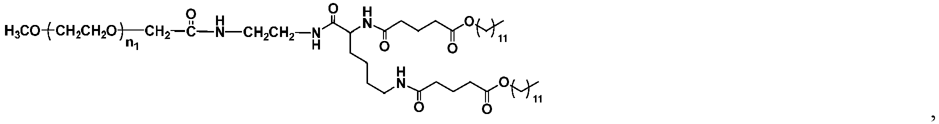

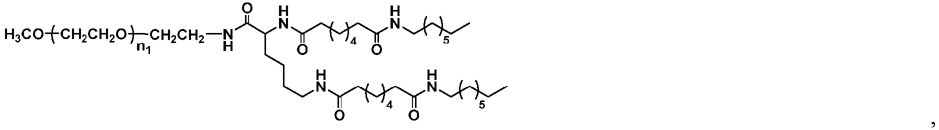

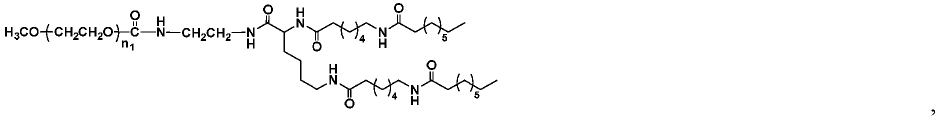

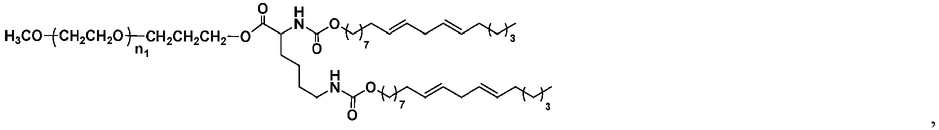

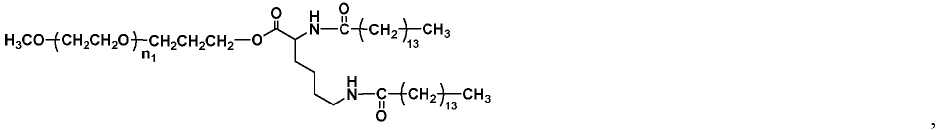

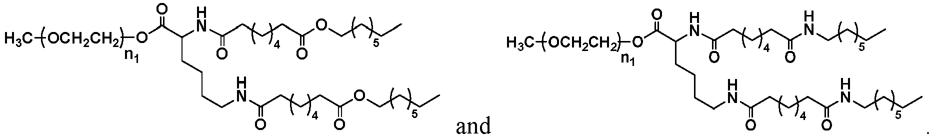

13. A lipid composition containing a PEGylated lipid of any one of claims **1** to **12.**

14. The lipid composition according to claim **13,** wherein the lipid composition contains one or more types of lipids selected from the group consisting of phospholipid, steroid lipid, and cationic lipid; the lipid composition is selected from any of the following cases:
Case (1): also contains a phospholipid;
Case (2): also contains a steroid lipid;
Case (3): also contains a cationic lipid;
Case (4): also contains a phospholipid and steroid lipid;
Case (5): also contains a phospholipid and cationic lipid;
Case (6): also contains a steroid lipid and cationic lipid;
Case (7): also contains a phospholipid, a steroid lipid and a cationic lipid;
more preferably, it also contains a phospholipid, a steroid lipid and a cationic lipid simultaneously.

15. The lipid composition according to claim **14,** wherein the cationic lipid is selected from the group consisting of *N*,*N*-dioleyl-*N*,*N*-dimethylammonium chloride, *N*,*N*-distearyl-*N*,*N*-dimethylammonium bromide, *N-(1-(2,3-dioleoyloxy)propyl)-N,N,N-trimethylammonium* chloride, *N-(1-(2,3-dioleyloxy)propyl)-N,N,N-trimethylammonium* chloride, *N*,*N*-dimethyl-2, 3-dioilyloxypropylamine, 3-(didodecylamino)-*N*1,*N*1,4-tridodecyl-1-piperazineethanamine, *N*-[2(didodecylamino)ethyl]-*N*1,*N*4,*N*4-tridodecyl-1,4-piperazinediethanamine, 14,25-ditridecyl-15,18,21,24-tetraaza-octatriacontane, 1,2-dileutheroxy-*N*, *N*-dimethylaminopropane, 2,2-dilinoleyl-4-dimethylaminomethyl-[1,3]-dioxolane, heptatriaconta-6,9,28,31-tetraen-19-yl-4-(dimethylamino)butanoate, 2,2-dilinoleyl-4-(2dimethylaminoethyl)-[1,3]-dioxolane, ((4-hydroxybutyl)azanediyl)bis(hexane-6,1-diyl)bis(2-hexyldecanoate), heptadecan-9-yl-8-((2-hydroxyethyl)(6-oxo-6-(undecyloxy)hexyl)amino)octanoate) and any combinations thereof;
or the cationic lipid is selected from the group consisting of the following structures and the combinations thereof: 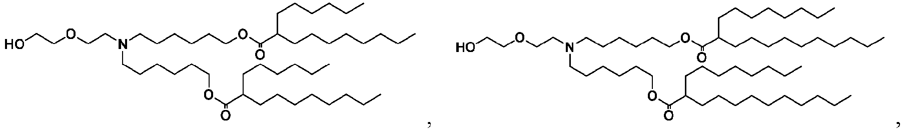 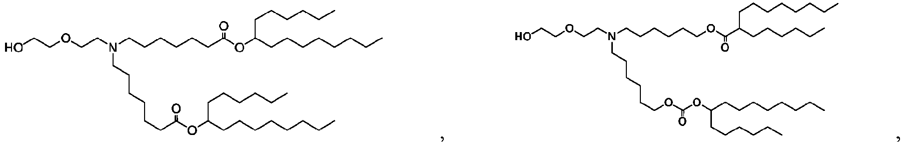 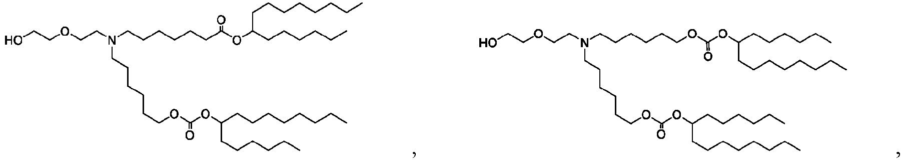 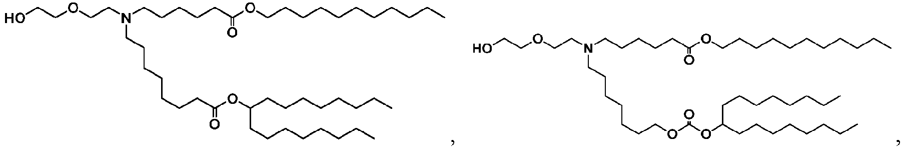 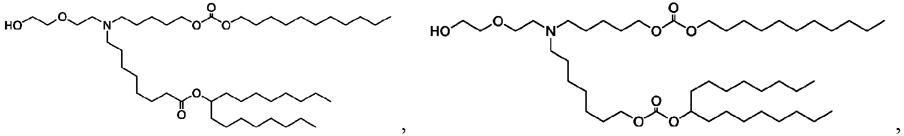 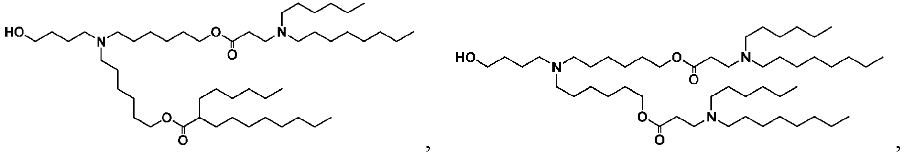 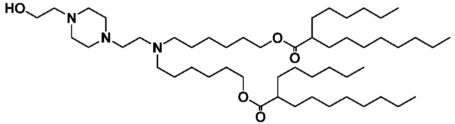 and 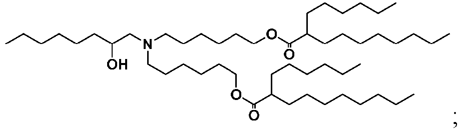
or the phospholipid is selected from the group consisting of 1,2-dilinoleoyl-sn-glycero-3-phosphocholine, 1,2-dimyristoleoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphatidylcholine, 1,2-diundecanoyl-sn-glycero-3-phosphatidylcholine, 1-plamitoyl-2-oleoyl-sn-glycero-3-phosphocholine, 1,2-di-*O*-octadecenyl-sn-glycero-3-phosphatidylcholine, 1-oleoyl-2-cholesterylhemisuccinyl-sn-glycero-3-phosphocholine, 1-*O*-hexadecyl-sn-glycero-3-phosphatidylcholine, 1,2-dilinolenoyl-sn-glycero-3-phosphatidylcholine, 1,2-diarachidonoyl-sn-glycero-3-phosphatidylcholine, 1,2-didecosahexaenoyl-sn-glycero-3-phosphocholine, 1,2-dioleoyl-sn-glycero-3-phosphoethanolamine, 1,2-diphytanyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didecosahexaenoyl-sn-glycero-3-phosphoethanolamine, 1,2-dioleoyl-sn-glycero-3-phospho-rac-(1-glycerol) sodium salt, dioleoyl phosphatidylserine, dipalmitoyl phosphatidylglycerol, palmitoyloleoyl phosphatidylethanolamine, distearoyl phosphatidylethanolamine, dipalmitoyl phosphatidylethanolamine, dimyristoleoyl phosphoethanolamine, 1-stearoyl-2-oleoyl-stearoylethanolamine, 1-stearoyl-2-oleoyl-phosphatidylcholine, sphingomyelin, phosphatidylcholine, phosphatidylethnolamine, phosphatidylserine, phosphatidylinositol, phosphatidic acid, palmitoyloleoyl phosphatidylcholine, lysophosphatidylcholine, lysophosphatidylethanolamine, and combinations thereof; the phospholipid can be synthetic or natural;
or the steroid lipid is selected from the group consisting of cholesterol, coprostanol, sitosterol, ergosterol, campesterol, stigmasterol, brassicasterol tomatidine, ursolic acid, α-tocopherol, and combinations thereof.

16. The lipid composition according to any one of claims **14** to **15,** wherein the lipid composition comprises 20-80% cationic lipids, 5-15% phospholipids, 25-55% steroid lipids and 0.5-10% PEGylated lipids; the percentage is the molar percentage of each lipid in the total lipids in a solution containing solvent.

17. The lipid composition according to claim **16,** wherein the molar percentage of the cationic lipid in the total lipids in a solution containing solvent is 30-65%;
or the molar percentage of the phospholipid in the total lipids in a solution containing solvent is 7.5-13%;
or the molar percentage of the steroid lipid in the total lipids in a solution containing solvent is 35-50%;
or the molar percentage of the PEGylated lipid in the total lipids in a solution containing solvent is 1-3%.

18. The lipid composition according to claim **16,** wherein the molar percentage of the cationic lipid in the total lipids in a solution containing solvent is selected from the group consisting of 35%, 40%, 45%, 46%, 47%, 48%, 49%, 50% and 55%;
or the molar percentage of the phospholipid in the total lipids in a solution containing solvent is selected from the group consisting of 8%, 9%, 10%, 11% and 12%;
or the molar percentage of the steroid lipid in the total lipids in a solution containing solvent is selected from the group consisting of 40%, 41%, 42%, 43%, 44%, 45%, 46%, 47%, 48%, 49% and 50%;
or the molar percentage of the PEGylated lipid in the total lipids in a solution containing solvent is selected from the group consisting of 1.5%, 1.6%, 1.7%, 1.8% and 1.9%.

19. A lipid pharmaceutical composition containing a lipid composition of any one of claims **13** to **18** and a drug, wherein the drug is selected from the group consisting of a nucleic acid drug, a gene vaccine, an anti-tumor drug, a small molecule drug, a peptide drug, and a protein drug.

20. The lipid pharmaceutical composition according to claim **19,** wherein the nucleic acid drug is selected from the group consisting of RNA, DNA, antisense nucleic acid, plasmid, interfering nucleic acid, aptamer, antagomir and ribozyme, and the RNA is selected from any of mRNA, saRNA, circRNA, miRNA and siRNA; preferably, the nucleic acid drug is selected from the group consisting of DNA, mRNA, miRNA and siRNA.

21. A lipid pharmaceutical composition according to claim **19,** wherein the lipid pharmaceutical composition is an LNP-pharmaceutical composition, an LPP-pharmaceutical composition or a PNP-pharmaceutical composition; preferably an LNP-pharmaceutical composition; preferably an LNP-nucleic acid pharmaceutical composition; more preferably an LNP-mRNA pharmaceutical composition.

22. The lipid pharmaceutical composition according to any one of claims **20** to **21,** the pharmaceutical composition is used as a drug, selected from the group consisting of an antineoplastic agent, an antiviral agent, an antifungal agent and a vaccine.

23. A formulation of lipid pharmaceutical composition containing any lipid pharmaceutical composition of claim **22** and a pharmaceutically acceptable diluent or excipient.

24. A formulation of lipid pharmaceutical composition according to claim **23,** wherein the diluent or excipient is preferably selected from the group consisting of deionized water, ultrapure water, phosphate buffer, and physiological saline, more preferably phosphate buffer or physiological saline, and most preferably physiological saline.

25. A liposome or lipid nanoparticle containing a lipid composition of any one of claims **13** to **18.**
